# EUROPEAN PATENT SPECIFICATION

(11) **EP 4 058 148 B1**
(45) Date of publication and mention of the grant of the patent: **13.03.2024**
(21) Application number: 20729951.2
(22) Date of filing: 08.05.2020
(51) Int. Cl.: A61P 35/00, A61K 39/00, A61K 39/395, C07K 16/28

(54) **DOSING REGIMEN FOR ANTI-BCMA AGENTS**
DOSIERSCHEMA FÜR ANTI-BCMA-WIRKSTOFFE
RÉGIME DE DOSAGE POUR AGENTS ANTI-BCMA

(30) Priority: 11.11.2019 EP 19208417
(43) Date of publication of application: 21.09.2022
(73) Proprietor: Amgen Research (Munich) GmbH, 81477 München (DE); Amgen Inc., Thousand Oaks, California 91320-1799 (US)
(72) Inventor: STIEGLMAIER, Julia, 81477 Muenchen (DE); HUBER, Birgit, 81477 Muenchen (DE); MINELLA, Alexander, Thousand Oaks, California 91320 (US); UPRETI, Vijay, Thousand Oaks, California 91320 (US)
(74) Representative: Schiweck Weinzierl Koch Patentanwälte Partnerschaft mbB
(86) International application number: PCT/EP2020/062876
(87) International publication number: WO 2021/094000

(56) References cited:
- WO-A1-2017/134134
- US-A1- 2013 273 055
- CHO S -F: "AMG 701, a half-life extended anti-BCMA BiTE , potently induces T cell-redirected lysis of human multiple myeloma cells and can be combined with IMiDs to overcome the immunosuppressive bone marrow microenvironment", CLINICAL LYMPHOMA, MYELOMA AND LEUKEMIA - 17TH INTERNATIONAL MYELOMA WORKSHOP 20191001 ELSEVIER INC. NLD, vol. 19, no. 10, Supplement, 1 October 2019 (2019-10-01), XP009519992, ISSN: 2152-2650
- YU-TZU TAI ET AL: "B cell maturation antigen (BCMA)-based immunotherapy for multiple myeloma", EXPERT OPINION ON BIOLOGICAL THERAPY, vol. 19, no. 11, 2 November 2019 (2019-11-02), pages 1143-1156, XP055686682, ASHLEY, LONDON; GB ISSN: 1471-2598, DOI: 10.1080/14712598.2019.1641196
- EINSELE H ET AL: "OAB-025: The anti-BCMA Bispecific T-cell Engager (BiTE ) Molecule AMG 420 Induced MRD-Negative Complete Responses in R/R Multiple Myeloma in a FIH study", CLINICAL LYMPHOMA MYELOMA AND LEUKEMIA; 17TH INTERNATIONAL MYELOMA WORKSHOP, ELSEVIER, NL; BOSTON, MA, USA, vol. 19, no. 10, Supplement, 1 October 2019 (2019-10-01), pages e18-e19, XP009519964, ISSN: 2152-2650, DOI: 10.1016/J.CLML.2019.09.026 [retrieved on 2019-10-18]
- TOPP M S ET AL: "8007: Evaluation of AMG 420, an anti-BCMA bispecific T-cell engager (BiTE) immunotherapy, in R/R multiple myeloma (MM) patients: Updated results of a first-in-human (FIH) phase i dose escalation study", JOURNAL OF CLINICAL ONCOLOGY; 2019 ANNUAL MEETING OF THE AMERICAN SOCIETY OF CLINICAL ONCOLOGY, ASCO 2019, LIPPINCOTT WILLIAMS & WILKINS, USA; CHICAGO, IL, USA, vol. 37, no. Supplement 15, 20 May 2019 (2019-05-20), page 8007, XP009519965, ISSN: 1527-7755, DOI: 10.1200/JCO.2019.37.15_SUPPL.8007 [retrieved on 2019-05-26]
- TOPP MAX S ET AL: "Treatment with AMG 420, an Anti-B-CellMaturationAntigen (BCMA) Bispecific T-Cell Engager (BiTE (R)) Antibody Construct, Induces Minimal Residual Disease (MRD) Negative Complete Responses in Relapsed and/or Refractory (R/R) Multiple Myeloma (MM) Patients: Results of a First-in-Human (FIH) Phase I Dos", BLOOD; 60TH ANNUAL MEETING OF THE AMERICAN-SOCIETY-OF-HEMATOLOGY (ASH), THE AMERICAN SOCIETY OF HEMATOLOGY, US; SAN DIEGO, CA, USA, vol. 132, no. Suppl. 1, 29 November 2018 (2018-11-29), page 1010, XP009519963, ISSN: 0006-4971, DOI: 10.1182/BLOOD-2018-99-109769
- TAI YU-TZU ET AL: "Novel anti-B-cell maturation antigen antibody-drug conjugate (GSK2857916) selectively induces killing of multiple myeloma", BLOOD, THE AMERICAN SOCIETY OF HEMATOLOGY, US, vol. 123, no. 20, 15 May 2014 (2014-05-15) , pages 3128-3138, XP009505860, ISSN: 0006-4971, DOI: 10.1182/BLOOD-2013-10-535088

## Description

### Field of the invention

The present invention relates to the dosage and administration of anti-BCMA agents for the treatment of BCMA positive neoplasms. More specifically, the present invention relates to an antibody construct comprising a first domain which binds to BCMA, a second domain which binds to CD3 and a third domain which extends or enhances the half-life of the antibody construct, for use in the treatment or amelioration of a BCMA positive neoplasm, wherein the antibody construct is administered at a specified dose in at least one cycle. Moreover, the disclosure provides a method for the treatment of a BCMA positive neoplasm comprising administering a specified dose of such antibody construct, and the use of such antibody construct for the manufacture of a medicament for the treatment of a BCMA positive neoplasm.

### Background of the invention

Multiple Myeloma (MM) is a malignant tumor of plasma cells which proliferate in bone marrow and release para protein. The resulting clinical laboratory pictures include infection, bone destruction, bone marrow failure, renal failure and hypercalcemia. The age adjusted annual incidence is increasing with approximately 6 new cases per 100,000. The incidence is 2 times higher in the black US population than in Caucasians. The 5-year survival rate for MM has increased from ~ 25% for newly diagnosed patients in 1975 to ~ 45% in 2006. This improvement is mainly due to new drugs such as proteasome inhibitors and immunomodulators. However, MM is not considered curable with current approaches. Patients refractory to proteasome inhibitors and immunomodulators show an unfavorable outcome with a median overall survival of 9 months.

Outcome is particularly poor in high-risk populations such as the subgroup with de117p13 positive MM.

Although many drugs are in clinical development for MM, new treatment options are still needed. Patients who show symptomatic disease are initially treated with primary induction therapy followed by high dose chemotherapy with autologous stem cell support in eligible patients. Patients eligible for intensive treatment are determined by age (65 to 75 years as upper limit), no comorbidities and intact renal function. Although this regimen has improved survival of younger and fit patients, the median duration of response does not exceed 3 years, and few patients remain free of the disease for more than 10 years.

Consolidation and maintenance approaches have been tested in order to increase the depth and duration of remission. Because maintenance treatment is challenging due to no efficacy or tolerability, there is still the option of improving survival outcome in the transplant setting by adding novel treatments to induction, consolidation or maintenance regimens. Patients not eligible for high dose therapy commonly receive induction regimens similar to the ones for the transplant candidates. These regimens include the proteasome inhibitor Bortezomib or a Melphalan based combination with Thalidomide. Median overall survival (OS) of Melphalan-Thalidomide-Prednisone (MPT) in elderly patients is 40 months. Lenalidomide in combination with Dexamethasone is the standard regimen for relapsed/refractory MM, but may move to the first line setting in transplant ineligible patients.

Other established regimens in the relapsed setting are repeat induction regimens or bortezomib or immunomodulator based salvage combinations with alkylating agents. An improvement of outcome (progression-free survival (PFS) and OS) is needed for patients in relapsed disease. Patients which are refractory to established treatments and progressing on treatment have a dismal outcome of 9 months OS on treatment and 3 months without treatment. The unmet need is highest in these patients.

Bispecific molecules such as BiTE^{®} (bispecific T cell engager) antibody constructs are recombinant protein constructs having (at least) one binding domain that is specific for a selected tumor-associated surface antigen on target cells, and a second binding domain that is specific for CD3, a subunit of the T cell receptor complex on T cells. By their particular design, BiTE^{®} antibody constructs are uniquely suited to transiently connect T cells with target cells and, at the same time, potently activate the inherent cytolytic potential of T cells against target cells. The first generation of so-called "canonical" (non-half-life-extended) BiTE^{®} antibody constructs (see WO 99/54440 and WO 2005/040220) was brought into the clinic as AMG 103 (blinatumomab, anti-CD19 x anti-CD3) and AMG 110 (solitomab, anti-EpCAM x anti-CD3). A further development of the first generation of canonical BiTE^{®} antibody constructs was the provision of bispecific antibody constructs binding to a context independent epitope at the N-terminus of the CD3-epsilon chain of human and *Callithrix jacchus, Saguinus oedipus* or *Saimiri sciureus* (WO 2008/119567). The first BiTE^{®} molecule comprising this new CD3-epsilon binding domain that was tested in the clinic was AMG 330.

Antibody constructs as described in WO 2008/119567 are likely to suffer from rapid clearance from the body; thus, whilst they are able to reach most parts of the body rapidly, and are quick to produce and easy to handle, their in vivo applications may be limited by their brief persistence in vivo. Prolonged administration by continuous intravenous infusion was used to achieve therapeutic effects because of the short in vivo half-life of this relatively small molecule. However, such continuous intravenous infusions are classified as inconvenient for the patients and, thus, in case of more convenient alternative treatment approaches, might hamper the election of the compound demonstrated to be more efficient in the treatment of the respective disease. This led to the development of bispecific therapeutics that retain similar therapeutic efficacy and at the same time have favorable pharmacokinetic properties, including a longer half-life. Therefore, an important further development of the so-called canonical BiTE^{®} molecules was the addition of a further domain that extends or enhances the half-life of the antibody construct. The resulting molecules are also called "HLE" (half-life extended) BiTE^{®} molecules, see e.g. WO 2017/134140.

B cell maturation antigen (BCMA, TNFRSF17, CD269) is a transmembrane protein belonging to the TNF receptor super family. BCMA expression is selectively induced during late stage plasma cell differentiation and is absent on naive and memory B cells. Upon BCMA binding to its ligands, B cell activating factor (BAFF) and a proliferation inducing ligand (APRIL), the survival of the bone marrow plasma cells and plasmablasts is promoted. BCMA does not maintain normal B cell homeostasis, but is required for the survival of long lived plasma cells. Studies in BCMA -/- mice showed that the survival of long lived bone marrow plasma cells was impaired, but B cell development and early humoral immune responses were indistinguishable from wild type mice. The mRNA expression of BCMA is highly elevated in malignant plasma cell disorders. By contrast, mRNA expression in normal tissues is very low and restricted to lymphoid tissues where normal long-lived plasma cells are located. BCMA protein expression is reported to be restricted to plasma cells only. Expression of BCMA is confined to plasma blasts and long-lived plasma cells and cannot be detected on other normal human tissues. BCMA is universally expressed on the cell surface of MM cells and at relatively higher levels on malignant plasma cells than the level observed on normal plasma cells. There is no correlation between BCMA expression and MM disease stage, response to last treatment and time from diagnosis. Neither T cells nor myeloid cells or CD34+ hematopoietic stem cells express BCMA. The selective expression of BCMA makes it a very attractive target for antibody-based and chimeric antigen receptor (CAR)-based therapies.

AMG 420 (formerly BI 836909) is a canonical bispecific T cell engager which binds to BCMA on target cells as well as to CD3-epsilon on T cells. It functions as a bridge between BCMA positive target cells such as MM cells and cytotoxic T lymphocytes (CTLs) by directing the cytolytic activity of CTLs to the target cells. AMG 420 consists of two single chain variable fragments (scFv), one being directed to BCMA and the other one to CD3. Each of the scFv fragments consists of a VH and a VL domain connected with a glycine/serine linker. The two scFv fragments are also connected with a glycine/serine linker. Half-life extended anti-BCMA x anti-CD3 antibody constructs such as AMG 701 are described in detail in WO 2017/134134. CHO S -F: ",CLINICAL LYMPHOMA, MYELOMA AND LEUKEMIA - 17TH INTERNATIONAL MYELOMA WORKSHOP ELSEVIER INC. NLD, vol. 19, no. 10, Supplement, 1 October 2019 discloses that AMG 701, a half-life extended anti-BCMA BiTE , potently induces T cell-redirected lysis of human multiple myeloma cells and can be combined with IMiDs to overcome the immunosuppressive bone marrow microenvironment There is an ongoing unmet medical need for the development of therapies for the treatment of BCMA positive neoplasms, in particular with such half-life extended anti-BCMA x anti-CD3 molecules.

### Summary of the invention

Based on the disclosure provided herein, those skilled in the art will recognize, or be able to ascertain using no more than routine experimentation, many equivalents to the specific embodiments of the invention described herein.

Item 1 An antibody construct comprising a first domain which binds to BCMA, a second domain which binds to CD3 and a third domain which extends the half-life of the antibody construct, for use in the treatment or amelioration of a BCMA positive neoplasm, in a human wherein the neoplasm is a B cell neoplasm or a plasma cell neoplasm wherein the antibody construct is administered at a minimum dose of 800 µg/day in at least one cycle, wherein one cycle comprises at least three individual administrations of the antibody construct, wherein one individual administration is the administration on one day.

Item 2. The antibody construct for use according to item 1 which is administered for 2, 3, 4, 5, 6, 7, 8, 9, 10, 11 12, 13, 14, 15, 16, 17, 18, 19, 20, 21, 22, 23, 24, 25 or more cycles.

Item 3. The antibody construct for use according to any one of items 1 or 2, wherein one cycle has about 25 to about 30 days, preferably about 26 or 27 to about 29 days, and more preferably about 28 days.

Item 4. The antibody construct for use according to any one of the preceding items, wherein the antibody construct is administered at a dose of 800 µg/day to 12 mg per day, such as 1000 µg, 1200 µg, 1500 µg, 1600 µg, 2000 µg, 2500 µg, 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg or 12 mg per day.

Item 5. The antibody construct for use according to any one of the preceding items, wherein the antibody construct is administered in one or two dose steps during the first cycle.

Item 6. The antibody construct for use according to item 5, wherein a first dose is between 800 µg/day and 1200 µg/day, an optional second dose is between 2500 µg/day and 5000 µg/day, preferably about 3000 µg/day or about 4500 µg/day, and a last dose (target dose) is between 6500 µg/day and 12 mg/day, such as 6500 µg/day, 7000 µg/day, 7500 µg/day, 8000 µg/day, 8500 µg/day, 9000 µg/day, 9500 µg/day, 10 mg/day, 11 mg/day or 12 mg/day.

Item 7. The antibody construct for use according to any one of the preceding items, wherein the first cycle comprises or consists of three to six individual administrations, preferably four or five individual administrations of the antibody construct.

Item 7a. The antibody construct for use according to item 6 or 7, wherein the first dose of 800 µg/day is administered on day 1.

Item 7b. The antibody construct for use according to item 7a, wherein
a) the target dose is administered on day 8 (+/- one or two days), day 15 (+/- one or two days) and day 22 (+/- one or two days), wherein the target dose is between 3000 µg/day and 12 mg/day, such as 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg or 12 mg per day;
b) the target dose is administered on day 4 or day 5 (+/- one or two days), day 12 (+/- one or two days) and day 19 (+/- one or two days), wherein the target dose is defined as in a);
c) the target dose is administered on day 3 or day 4 (+/- one or two days), day 8 (+/- one or two days), day 15 (+/- one or two days) and day 22 (+/- one or two days), wherein the target dose is defined as in a); or
d) the second dose is administered on day 3 or day 4 (+/- one or two days), and the target dose is administered on day 8 (+/- one or two days), day 15 (+/- one or two days) and day 22 (+/- one or two days), wherein the second dose is between 2500 µg/day and 5000 µg/day, preferably about 3000 µg/day or about 4500 µg/day, and the target dose is higher than the second dose and is defined as in a).

Item 8. The antibody construct for use according to any one of the items 2 to 7, wherein the antibody construct is administered during the second cycle and optionally during any subsequent cycle at constant doses.

Item 8a. The antibody construct for use according to item 8, wherein the constant dose is the highest dose administered during the first cycle (target dose).

Item 8b. The antibody construct for use according to any one of the items 2 to 8a, wherein the antibody construct is administered once by week during the second cycle and optionally during any subsequent cycle.

Item 9. The antibody construct for use according to any one of the preceding items, which is administered parenterally, preferably intravenously, and more preferably via intravenous bolus injection, bolus infusion or short-term intravenous infusion.

Item 10. The antibody construct for use according to any one of the preceding items, wherein the BCMA positive neoplasm is selected from the group consisting of multiple myeloma, relapsed and/or refractory multiple myeloma, heavy chain multiple myeloma, light chain multiple myeloma, extramedullary myeloma, plasmacytoma, plasma cell leukemia, Waldenström's macroglobulinemia, and smoldering myeloma.

Item 10a. The antibody construct for use according to item 10, wherein the BCMA positive neoplasm is multiple myeloma.

Item 10b. The antibody construct for use according to item 10a, wherein the multiple myeloma is selected from the group consisting of relapsed and/or refractory multiple myeloma, heavy chain multiple myeloma, light chain multiple myeloma, extramedullary multiple myeloma, and smoldering multiple myeloma.

Item 11. The antibody construct for use according to any one of the preceding items, wherein
a) the antibody construct is a single chain polypeptide,
b) the first domain is in the format of an scFv,
c) the second domain is in the format of an scFv, and/or
d) the first domain and the second domain are connected via a linker, preferably a peptide linker, more preferably a glycine/serine linker.

Item 12. The antibody construct for use according to any one of the preceding items, which competes for binding to BCMA with or which binds to the same epitope of BCMA as:
a) an antibody or antibody construct comprising a domain which binds to BCMA on the surface of a target cell, wherein said domain comprises a VH region comprising CDR-H1 as depicted in SEQ ID NO: 171, CDR-H2 as depicted in SEQ ID NO: 172, and CDR-H3 as depicted in SEQ ID NO: 173, and a VL region comprising CDR-L1 as depicted in SEQ ID NO: 174, CDR-L2 as depicted in SEQ ID NO: 175, and CDR-L3 as depicted in SEQ ID NO: 176;
b) an antibody or antibody construct comprising a domain which binds to BCMA on the surface of a target cell, wherein said domain comprises a VH region as depicted in SEQ ID NO: 177, and a VL region as depicted in SEQ ID NO: 178;
c) an antibody construct comprising a domain which binds to BCMA on the surface of a target cell, wherein said domain comprises the amino acid sequence as depicted in SEQ ID NO: 179; or
d) an antibody construct having the amino acid sequence as depicted in SEQ ID NO: 661.

Item 13. The antibody construct for use according to any one of the preceding items, which competes for binding to CD3 with or which binds to the same epitope of CD3 as:
a) an antibody or antibody construct comprising a domain which binds to CD3 on the surface of a T cell, wherein said domain comprises a VH region comprising CDR-H1 as depicted in SEQ ID NO: 636, CDR-H2 as depicted in SEQ ID NO: 637, and CDR-H3 as depicted in SEQ ID NO: 638, and a VL region comprising CDR-L1 as depicted in SEQ ID NO: 633, CDR-L2 as depicted in SEQ ID NO: 634, CDR-L3 as depicted in SEQ ID NO: 635;
b) an antibody or antibody construct comprising a domain which binds to CD3 on the surface of a T cell, wherein said domain comprises a VH region as depicted in SEQ ID NO: 639, and a VL region as depicted in SEQ ID NO: 641;
c) an antibody construct comprising a domain which binds to CD3 on the surface of a T cell, wherein said domain comprises the amino acid sequence as depicted in SEQ ID NO: 642; or
d) an antibody construct having the amino acid sequence as depicted in SEQ ID NO: 661.

Item 14. The antibody construct for use according to any one of the preceding items, wherein the first domain which binds to BCMA comprises a VH region comprising CDR-H1, CDR-H2 and CDR-H3 and a VL region comprising CDR-L1, CDR-L2 and CDR-L3 selected from:
(1) CDR-H1 as depicted in SEQ ID NO: 1, CDR-H2 as depicted in SEQ ID NO: 2, CDR-H3 as depicted in SEQ ID NO: 3, CDR-L1 as depicted in SEQ ID NO: 4, CDR-L2 as depicted in SEQ ID NO: 5, and CDR-L3 as depicted in SEQ ID NO: 6;
(2) CDR-H1 as depicted in SEQ ID NO: 11, CDR-H2 as depicted in SEQ ID NO: 12, CDR-H3 as depicted in SEQ ID NO: 13, CDR-L1 as depicted in SEQ ID NO: 14, CDR-L2 as depicted in SEQ ID NO: 15, and CDR-L3 as depicted in SEQ ID NO: 16;
(3) CDR-H1 as depicted in SEQ ID NO: 21, CDR-H2 as depicted in SEQ ID NO: 22, CDR-H3 as depicted in SEQ ID NO: 23, CDR-L1 as depicted in SEQ ID NO: 24, CDR-L2 as depicted in SEQ ID NO: 25, and CDR-L3 as depicted in SEQ ID NO: 26;
(4) CDR-H1 as depicted in SEQ ID NO: 31, CDR-H2 as depicted in SEQ ID NO: 32, CDR-H3 as depicted in SEQ ID NO: 33, CDR-L1 as depicted in SEQ ID NO: 34, CDR-L2 as depicted in SEQ ID NO: 35, and CDR-L3 as depicted in SEQ ID NO: 36;
(5) CDR-H1 as depicted in SEQ ID NO: 41, CDR-H2 as depicted in SEQ ID NO: 42, CDR-H3 as depicted in SEQ ID NO: 43, CDR-L1 as depicted in SEQ ID NO: 44, CDR-L2 as depicted in SEQ ID NO: 45, and CDR-L3 as depicted in SEQ ID NO: 46;
(6) CDR-H1 as depicted in SEQ ID NO: 51, CDR-H2 as depicted in SEQ ID NO: 52, CDR-H3 as depicted in SEQ ID NO: 53, CDR-L1 as depicted in SEQ ID NO: 54, CDR-L2 as depicted in SEQ ID NO: 55, and CDR-L3 as depicted in SEQ ID NO: 56;
(7) CDR-H1 as depicted in SEQ ID NO: 61, CDR-H2 as depicted in SEQ ID NO: 62, CDR-H3 as depicted in SEQ ID NO: 63, CDR-L1 as depicted in SEQ ID NO: 64, CDR-L2 as depicted in SEQ ID NO: 65, and CDR-L3 as depicted in SEQ ID NO: 66;
(8) CDR-H1 as depicted in SEQ ID NO: 71, CDR-H2 as depicted in SEQ ID NO: 72, CDR-H3 as depicted in SEQ ID NO: 73, CDR-L1 as depicted in SEQ ID NO: 74, CDR-L2 as depicted in SEQ ID NO: 75, and CDR-L3 as depicted in SEQ ID NO: 76;
(9) CDR-H1 as depicted in SEQ ID NO: 81, CDR-H2 as depicted in SEQ ID NO: 82, CDR-H3 as depicted in SEQ ID NO: 83, CDR-L1 as depicted in SEQ ID NO: 84, CDR-L2 as depicted in SEQ ID NO: 85, and CDR-L3 as depicted in SEQ ID NO: 86;
(10) CDR-H1 as depicted in SEQ ID NO: 91, CDR-H2 as depicted in SEQ ID NO: 92, CDR-H3 as depicted in SEQ ID NO: 93, CDR-L1 as depicted in SEQ ID NO: 94, CDR-L2 as depicted in SEQ ID NO: 95, and CDR-L3 as depicted in SEQ ID NO: 96;
(11) CDR-H1 as depicted in SEQ ID NO: 101, CDR-H2 as depicted in SEQ ID NO: 102, CDR-H3 as depicted in SEQ ID NO: 103, CDR-L1 as depicted in SEQ ID NO: 104, CDR-L2 as depicted in SEQ ID NO: 105, and CDR-L3 as depicted in SEQ ID NO: 106;
(12) CDR-H1 as depicted in SEQ ID NO: 111, CDR-H2 as depicted in SEQ ID NO: 112, CDR-H3 as depicted in SEQ ID NO: 113, CDR-L1 as depicted in SEQ ID NO: 114, CDR-L2 as depicted in SEQ ID NO: 115, and CDR-L3 as depicted in SEQ ID NO: 116;
(13) CDR-H1 as depicted in SEQ ID NO: 121, CDR-H2 as depicted in SEQ ID NO: 122, CDR-H3 as depicted in SEQ ID NO: 123, CDR-L1 as depicted in SEQ ID NO: 124, CDR-L2 as depicted in SEQ ID NO: 125, and CDR-L3 as depicted in SEQ ID NO: 126;
(14) CDR-H1 as depicted in SEQ ID NO: 131, CDR-H2 as depicted in SEQ ID NO: 132, CDR-H3 as depicted in SEQ ID NO: 133, CDR-L1 as depicted in SEQ ID NO: 134, CDR-L2 as depicted in SEQ ID NO: 135, and CDR-L3 as depicted in SEQ ID NO: 136;
(15) CDR-H1 as depicted in SEQ ID NO: 141, CDR-H2 as depicted in SEQ ID NO: 142, CDR-H3 as depicted in SEQ ID NO: 143, CDR-L1 as depicted in SEQ ID NO: 144, CDR-L2 as depicted in SEQ ID NO: 145, and CDR-L3 as depicted in SEQ ID NO: 146;
(16) CDR-H1 as depicted in SEQ ID NO: 151, CDR-H2 as depicted in SEQ ID NO: 152, CDR-H3 as depicted in SEQ ID NO: 153, CDR-L1 as depicted in SEQ ID NO: 154, CDR-L2 as depicted in SEQ ID NO: 155, and CDR-L3 as depicted in SEQ ID NO: 156;
(17) CDR-H1 as depicted in SEQ ID NO: 161, CDR-H2 as depicted in SEQ ID NO: 162, CDR-H3 as depicted in SEQ ID NO: 163, CDR-L1 as depicted in SEQ ID NO: 164, CDR-L2 as depicted in SEQ ID NO: 165, and CDR-L3 as depicted in SEQ ID NO: 166;
(18) CDR-H1 as depicted in SEQ ID NO: 171, CDR-H2 as depicted in SEQ ID NO: 172, CDR-H3 as depicted in SEQ ID NO: 173, CDR-L1 as depicted in SEQ ID NO: 174, CDR-L2 as depicted in SEQ ID NO: 175, and CDR-L3 as depicted in SEQ ID NO: 176;
(19) CDR-H1 as depicted in SEQ ID NO: 181, CDR-H2 as depicted in SEQ ID NO: 182, CDR-H3 as depicted in SEQ ID NO: 183, CDR-L1 as depicted in SEQ ID NO: 184, CDR-L2 as depicted in SEQ ID NO: 185, and CDR-L3 as depicted in SEQ ID NO: 186;
(20) CDR-H1 as depicted in SEQ ID NO: 191, CDR-H2 as depicted in SEQ ID NO: 192, CDR-H3 as depicted in SEQ ID NO: 193, CDR-L1 as depicted in SEQ ID NO: 194, CDR-L2 as depicted in SEQ ID NO: 195, and CDR-L3 as depicted in SEQ ID NO: 196;
(21) CDR-H1 as depicted in SEQ ID NO: 201, CDR-H2 as depicted in SEQ ID NO: 202, CDR-H3 as depicted in SEQ ID NO: 203, CDR-L1 as depicted in SEQ ID NO: 204, CDR-L2 as depicted in SEQ ID NO: 205, and CDR-L3 as depicted in SEQ ID NO: 206;
(22) CDR-H1 as depicted in SEQ ID NO: 211, CDR-H2 as depicted in SEQ ID NO: 212, CDR-H3 as depicted in SEQ ID NO: 213, CDR-L1 as depicted in SEQ ID NO: 214, CDR-L2 as depicted in SEQ ID NO: 215, and CDR-L3 as depicted in SEQ ID NO: 216;
(23) CDR-H1 as depicted in SEQ ID NO: 221, CDR-H2 as depicted in SEQ ID NO: 222, CDR-H3 as depicted in SEQ ID NO: 223, CDR-L1 as depicted in SEQ ID NO: 224, CDR-L2 as depicted in SEQ ID NO: 225, and CDR-L3 as depicted in SEQ ID NO: 226;
(24) CDR-H1 as depicted in SEQ ID NO: 231, CDR-H2 as depicted in SEQ ID NO: 232, CDR-H3 as depicted in SEQ ID NO: 233, CDR-L1 as depicted in SEQ ID NO: 234, CDR-L2 as depicted in SEQ ID NO: 235, and CDR-L3 as depicted in SEQ ID NO: 236;
(25) CDR-H1 as depicted in SEQ ID NO: 241, CDR-H2 as depicted in SEQ ID NO: 242, CDR-H3 as depicted in SEQ ID NO: 243, CDR-L1 as depicted in SEQ ID NO: 244, CDR-L2 as depicted in SEQ ID NO: 245, and CDR-L3 as depicted in SEQ ID NO: 246;
(26) CDR-H1 as depicted in SEQ ID NO: 251, CDR-H2 as depicted in SEQ ID NO: 252, CDR-H3 as depicted in SEQ ID NO: 253, CDR-L1 as depicted in SEQ ID NO: 254, CDR-L2 as depicted in SEQ ID NO: 255, and CDR-L3 as depicted in SEQ ID NO: 256;
(27) CDR-H1 as depicted in SEQ ID NO: 261, CDR-H2 as depicted in SEQ ID NO: 262, CDR-H3 as depicted in SEQ ID NO: 263, CDR-L1 as depicted in SEQ ID NO: 264, CDR-L2 as depicted in SEQ ID NO: 265, and CDR-L3 as depicted in SEQ ID NO: 266;
(28) CDR-H1 as depicted in SEQ ID NO: 271, CDR-H2 as depicted in SEQ ID NO: 272, CDR-H3 as depicted in SEQ ID NO: 273, CDR-L1 as depicted in SEQ ID NO: 274, CDR-L2 as depicted in SEQ ID NO: 275, and CDR-L3 as depicted in SEQ ID NO: 276;
(29) CDR-H1 as depicted in SEQ ID NO: 281, CDR-H2 as depicted in SEQ ID NO: 282, CDR-H3 as depicted in SEQ ID NO: 283, CDR-L1 as depicted in SEQ ID NO: 284, CDR-L2 as depicted in SEQ ID NO: 285, and CDR-L3 as depicted in SEQ ID NO: 286;
(30) CDR-H1 as depicted in SEQ ID NO: 291, CDR-H2 as depicted in SEQ ID NO: 292, CDR-H3 as depicted in SEQ ID NO: 293, CDR-L1 as depicted in SEQ ID NO: 294, CDR-L2 as depicted in SEQ ID NO: 295, and CDR-L3 as depicted in SEQ ID NO: 296;
(31) CDR-H1 as depicted in SEQ ID NO: 301, CDR-H2 as depicted in SEQ ID NO: 302, CDR-H3 as depicted in SEQ ID NO: 303, CDR-L1 as depicted in SEQ ID NO: 304, CDR-L2 as depicted in SEQ ID NO: 305, and CDR-L3 as depicted in SEQ ID NO: 306;
(32) CDR-H1 as depicted in SEQ ID NO: 311, CDR-H2 as depicted in SEQ ID NO: 312, CDR-H3 as depicted in SEQ ID NO: 313, CDR-L1 as depicted in SEQ ID NO: 314, CDR-L2 as depicted in SEQ ID NO: 315, and CDR-L3 as depicted in SEQ ID NO: 316;
(33) CDR-H1 as depicted in SEQ ID NO: 321, CDR-H2 as depicted in SEQ ID NO: 322, CDR-H3 as depicted in SEQ ID NO: 323, CDR-L1 as depicted in SEQ ID NO: 324, CDR-L2 as depicted in SEQ ID NO: 325, and CDR-L3 as depicted in SEQ ID NO: 326;
(34) CDR-H1 as depicted in SEQ ID NO: 331, CDR-H2 as depicted in SEQ ID NO: 332, CDR-H3 as depicted in SEQ ID NO: 333, CDR-L1 as depicted in SEQ ID NO: 334, CDR-L2 as depicted in SEQ ID NO: 335, and CDR-L3 as depicted in SEQ ID NO: 336;
(35) CDR-H1 as depicted in SEQ ID NO: 341, CDR-H2 as depicted in SEQ ID NO: 342, CDR-H3 as depicted in SEQ ID NO: 343, CDR-L1 as depicted in SEQ ID NO: 344, CDR-L2 as depicted in SEQ ID NO: 345, and CDR-L3 as depicted in SEQ ID NO: 346;
(36) CDR-H1 as depicted in SEQ ID NO: 351, CDR-H2 as depicted in SEQ ID NO: 352, CDR-H3 as depicted in SEQ ID NO: 353, CDR-L1 as depicted in SEQ ID NO: 354, CDR-L2 as depicted in SEQ ID NO: 355, and CDR-L3 as depicted in SEQ ID NO: 356;
(37) CDR-H1 as depicted in SEQ ID NO: 361, CDR-H2 as depicted in SEQ ID NO: 362, CDR-H3 as depicted in SEQ ID NO: 363, CDR-L1 as depicted in SEQ ID NO: 364, CDR-L2 as depicted in SEQ ID NO: 365, and CDR-L3 as depicted in SEQ ID NO: 366;
(38) CDR-H1 as depicted in SEQ ID NO: 371, CDR-H2 as depicted in SEQ ID NO: 372, CDR-H3 as depicted in SEQ ID NO: 373, CDR-L1 as depicted in SEQ ID NO: 374, CDR-L2 as depicted in SEQ ID NO: 375, and CDR-L3 as depicted in SEQ ID NO: 376;
(39) CDR-H1 as depicted in SEQ ID NO: 381, CDR-H2 as depicted in SEQ ID NO: 382, CDR-H3 as depicted in SEQ ID NO: 383, CDR-L1 as depicted in SEQ ID NO: 384, CDR-L2 as depicted in SEQ ID NO: 385, and CDR-L3 as depicted in SEQ ID NO: 386;
(40) CDR-H1 as depicted in SEQ ID NO: 391, CDR-H2 as depicted in SEQ ID NO: 392, CDR-H3 as depicted in SEQ ID NO: 393, CDR-L1 as depicted in SEQ ID NO: 394, CDR-L2 as depicted in SEQ ID NO: 395, and CDR-L3 as depicted in SEQ ID NO: 396;
(41) CDR-H1 as depicted in SEQ ID NO: 401, CDR-H2 as depicted in SEQ ID NO: 402, CDR-H3 as depicted in SEQ ID NO: 403, CDR-L1 as depicted in SEQ ID NO: 404, CDR-L2 as depicted in SEQ ID NO: 405, and CDR-L3 as depicted in SEQ ID NO: 406;
(42) CDR-H1 as depicted in SEQ ID NO: 411, CDR-H2 as depicted in SEQ ID NO: 412, CDR-H3 as depicted in SEQ ID NO: 413, CDR-L1 as depicted in SEQ ID NO: 414, CDR-L2 as depicted in SEQ ID NO: 415, and CDR-L3 as depicted in SEQ ID NO: 416;
(43) CDR-H1 as depicted in SEQ ID NO: 421, CDR-H2 as depicted in SEQ ID NO: 422, CDR-H3 as depicted in SEQ ID NO: 423, CDR-L1 as depicted in SEQ ID NO: 424, CDR-L2 as depicted in SEQ ID NO: 425, and CDR-L3 as depicted in SEQ ID NO: 426;
(44) CDR-H1 as depicted in SEQ ID NO: 431, CDR-H2 as depicted in SEQ ID NO: 432, CDR-H3 as depicted in SEQ ID NO: 433, CDR-L1 as depicted in SEQ ID NO: 434, CDR-L2 as depicted in SEQ ID NO: 435, and CDR-L3 as depicted in SEQ ID NO: 436;
(45) CDR-H1 as depicted in SEQ ID NO: 441, CDR-H2 as depicted in SEQ ID NO: 442, CDR-H3 as depicted in SEQ ID NO: 443, CDR-L1 as depicted in SEQ ID NO: 444, CDR-L2 as depicted in SEQ ID NO: 445, and CDR-L3 as depicted in SEQ ID NO: 446;
(46) CDR-H1 as depicted in SEQ ID NO: 451, CDR-H2 as depicted in SEQ ID NO: 452, CDR-H3 as depicted in SEQ ID NO: 453, CDR-L1 as depicted in SEQ ID NO: 454, CDR-L2 as depicted in SEQ ID NO: 455, and CDR-L3 as depicted in SEQ ID NO: 456;
(47) CDR-H1 as depicted in SEQ ID NO: 461, CDR-H2 as depicted in SEQ ID NO: 462, CDR-H3 as depicted in SEQ ID NO: 463, CDR-L1 as depicted in SEQ ID NO: 464, CDR-L2 as depicted in SEQ ID NO: 465, and CDR-L3 as depicted in SEQ ID NO: 466;
(48) CDR-H1 as depicted in SEQ ID NO: 471, CDR-H2 as depicted in SEQ ID NO: 472, CDR-H3 as depicted in SEQ ID NO: 473, CDR-L1 as depicted in SEQ ID NO: 474, CDR-L2 as depicted in SEQ ID NO: 475, and CDR-L3 as depicted in SEQ ID NO: 476;
(49) CDR-H1 as depicted in SEQ ID NO: 481, CDR-H2 as depicted in SEQ ID NO: 482, CDR-H3 as depicted in SEQ ID NO: 483, CDR-L1 as depicted in SEQ ID NO: 484, CDR-L2 as depicted in SEQ ID NO: 485, and CDR-L3 as depicted in SEQ ID NO: 486;
(50) CDR-H1 as depicted in SEQ ID NO: 491, CDR-H2 as depicted in SEQ ID NO: 492, CDR-H3 as depicted in SEQ ID NO: 493, CDR-L1 as depicted in SEQ ID NO: 494, CDR-L2 as depicted in SEQ ID NO: 495, and CDR-L3 as depicted in SEQ ID NO: 496;
(51) CDR-H1 as depicted in SEQ ID NO: 501, CDR-H2 as depicted in SEQ ID NO: 502, CDR-H3 as depicted in SEQ ID NO: 503, CDR-L1 as depicted in SEQ ID NO: 504, CDR-L2 as depicted in SEQ ID NO: 505, and CDR-L3 as depicted in SEQ ID NO: 506;
(52) CDR-H1 as depicted in SEQ ID NO: 511, CDR-H2 as depicted in SEQ ID NO: 512, CDR-H3 as depicted in SEQ ID NO: 513, CDR-L1 as depicted in SEQ ID NO: 514, CDR-L2 as depicted in SEQ ID NO: 515, and CDR-L3 as depicted in SEQ ID NO: 516; and
(53) CDR-H1 as depicted in SEQ ID NO: 521, CDR-H2 as depicted in SEQ ID NO: 522, CDR-H3 as depicted in SEQ ID NO: 523, CDR-L1 as depicted in SEQ ID NO: 524, CDR-L2 as depicted in SEQ ID NO: 525, and CDR-L3 as depicted in SEQ ID NO: 526.

Item 15. The antibody construct for use according to any one of the preceding items, wherein the first domain which binds to BCMA comprises a VH region having an amino acid sequence selected from the group consisting of those depicted in SEQ ID NOs: 7, 17, 27, 37, 47, 57, 67, 77, 87, 97, 107, 117, 127, 137, 147, 157, 167, 177, 187, 197, 207, 217, 227, 237, 247, 257, 267, 277, 287, 307, 317, 327, 337, 347, 357, 367, 377, 387, 397, 407, 417, 427, 437, 447, 457, 467, 477, 487, 497, 507, 517, and 527, preferably SEQ ID NO: 177.

Item 16. The antibody construct for use according to any one of the preceding items, wherein the first domain which binds to BCMA comprises a VL region having an amino acid sequence selected from the group consisting of those depicted in SEQ ID NOs: 8, 18, 28, 38, 48, 58, 68, 78, 88, 98, 108, 118, 128, 138, 148, 158, 168, 178, 188, 198, 208, 218, 228, 238, 248, 258, 268, 278, 288, 298, 308, 318, 328, 338, 348, 358, 368, 378, 388, 398, 408, 418, 428, 438, 448, 458, 468, 478, 488, 498, 508, 518, and 528, preferably SEQ ID NO: 178.

Item 17. The antibody construct for use according to any one of the preceding items, wherein the first domain which binds to BCMA comprises a VH region and a VL region selected from the group consisting of:
(1) a VH region as depicted in SEQ ID NO: 7 and a VL region as depicted in SEQ ID NO: 8;
(2) a VH region as depicted in SEQ ID NO: 17 and a VL region as depicted in SEQ ID NO: 18;
(3) a VH region as depicted in SEQ ID NO: 27 and a VL region as depicted in SEQ ID NO: 28;
(4) a VH region as depicted in SEQ ID NO: 37 and a VL region as depicted in SEQ ID NO: 38;
(5) a VH region as depicted in SEQ ID NO: 47 and a VL region as depicted in SEQ ID NO: 48;
(6) a VH region as depicted in SEQ ID NO: 57 and a VL region as depicted in SEQ ID NO: 58;
(7) a VH region as depicted in SEQ ID NO: 67 and a VL region as depicted in SEQ ID NO: 68;
(8) a VH region as depicted in SEQ ID NO: 77 and a VL region as depicted in SEQ ID NO: 78;
(9) a VH region as depicted in SEQ ID NO: 87 and a VL region as depicted in SEQ ID NO: 88;
(10) a VH region as depicted in SEQ ID NO: 97 and a VL region as depicted in SEQ ID NO: 98;
(11) a VH region as depicted in SEQ ID NO: 107 and a VL region as depicted in SEQ ID NO: 108;
(12) a VH region as depicted in SEQ ID NO: 117 and a VL region as depicted in SEQ ID NO: 118;
(13) a VH region as depicted in SEQ ID NO: 127 and a VL region as depicted in SEQ ID NO: 128;
(14) a VH region as depicted in SEQ ID NO: 137 and a VL region as depicted in SEQ ID NO: 138;
(15) a VH region as depicted in SEQ ID NO: 147 and a VL region as depicted in SEQ ID NO: 148;
(16) a VH region as depicted in SEQ ID NO: 157 and a VL region as depicted in SEQ ID NO: 158;
(17) a VH region as depicted in SEQ ID NO: 167 and a VL region as depicted in SEQ ID NO: 168;
(18) a VH region as depicted in SEQ ID NO: 177 and a VL region as depicted in SEQ ID NO: 178;
(19) a VH region as depicted in SEQ ID NO: 187 and a VL region as depicted in SEQ ID NO: 188;
(20) a VH region as depicted in SEQ ID NO: 197 and a VL region as depicted in SEQ ID NO: 198;
(21) a VH region as depicted in SEQ ID NO: 207 and a VL region as depicted in SEQ ID NO: 208;
(22) a VH region as depicted in SEQ ID NO: 217 and a VL region as depicted in SEQ ID NO: 218;
(23) a VH region as depicted in SEQ ID NO: 227 and a VL region as depicted in SEQ ID NO: 228;
(24) a VH region as depicted in SEQ ID NO: 237 and a VL region as depicted in SEQ ID NO: 238;
(25) a VH region as depicted in SEQ ID NO: 247 and a VL region as depicted in SEQ ID NO: 248;
(26) a VH region as depicted in SEQ ID NO: 257 and a VL region as depicted in SEQ ID NO: 258;
(27) a VH region as depicted in SEQ ID NO: 267 and a VL region as depicted in SEQ ID NO: 268;
(28) a VH region as depicted in SEQ ID NO: 277 and a VL region as depicted in SEQ ID NO: 278;
(29) a VH region as depicted in SEQ ID NO: 287 and a VL region as depicted in SEQ ID NO: 288;
(30) a VH region as depicted in SEQ ID NO: 297 and a VL region as depicted in SEQ ID NO: 298;
(31) a VH region as depicted in SEQ ID NO: 307 and a VL region as depicted in SEQ ID NO: 308;
(32) a VH region as depicted in SEQ ID NO: 317 and a VL region as depicted in SEQ ID NO: 318;
(33) a VH region as depicted in SEQ ID NO: 327 and a VL region as depicted in SEQ ID NO: 328;
(34) a VH region as depicted in SEQ ID NO: 337 and a VL region as depicted in SEQ ID NO: 338;
(35) a VH region as depicted in SEQ ID NO: 347 and a VL region as depicted in SEQ ID NO: 348;
(36) a VH region as depicted in SEQ ID NO: 357 and a VL region as depicted in SEQ ID NO: 358;
(37) a VH region as depicted in SEQ ID NO: 367 and a VL region as depicted in SEQ ID NO: 368;
(38) a VH region as depicted in SEQ ID NO: 377 and a VL region as depicted in SEQ ID NO: 378;
(39) a VH region as depicted in SEQ ID NO: 387 and a VL region as depicted in SEQ ID NO: 388;
(40) a VH region as depicted in SEQ ID NO: 397 and a VL region as depicted in SEQ ID NO: 398;
(41) a VH region as depicted in SEQ ID NO: 407 and a VL region as depicted in SEQ ID NO: 408;
(42) a VH region as depicted in SEQ ID NO: 417 and a VL region as depicted in SEQ ID NO: 418;
(43) a VH region as depicted in SEQ ID NO: 427 and a VL region as depicted in SEQ ID NO: 428;
(44) a VH region as depicted in SEQ ID NO: 437 and a VL region as depicted in SEQ ID NO: 438;
(45) a VH region as depicted in SEQ ID NO: 447 and a VL region as depicted in SEQ ID NO: 448;
(46) a VH region as depicted in SEQ ID NO: 457 and a VL region as depicted in SEQ ID NO: 458;
(47) a VH region as depicted in SEQ ID NO: 467 and a VL region as depicted in SEQ ID NO: 468;
(48) a VH region as depicted in SEQ ID NO: 477 and a VL region as depicted in SEQ ID NO: 478;
(49) a VH region as depicted in SEQ ID NO: 487 and a VL region as depicted in SEQ ID NO: 488;
(50) a VH region as depicted in SEQ ID NO: 497 and a VL region as depicted in SEQ ID NO: 498;
(51) a VH region as depicted in SEQ ID NO: 507 and a VL region as depicted in SEQ ID NO: 508;
(52) a VH region as depicted in SEQ ID NO: 517 and a VL region as depicted in SEQ ID NO: 518; and
(53) a VH region as depicted in SEQ ID NO: 527 and a VL region as depicted in SEQ ID NO: 528.

Item 18. The antibody construct for use according to any one of the preceding items, wherein the first domain which binds to BCMA comprises or consists of a polypeptide having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529, preferably SEQ ID NO: 179.

Item 19. The antibody construct for use according to any one of the preceding items, wherein the second domain which binds to CD3 comprises a VL region comprising CDR-L1, CDR-L2 and CDR-L3 selected from the group consisting of:
(a) CDR-L1 as depicted in SEQ ID NO: 542, CDR-L2 as depicted in SEQ ID NO: 543, and CDR-L3 as depicted in SEQ ID NO: 544;
(b) CDR-L1 as depicted in SEQ ID NO: 599, CDR-L2 as depicted in SEQ ID NO: 600, and CDR-L3 as depicted in SEQ ID NO: 601; and
(c) CDR-L1 as depicted in SEQ ID NO: 621, CDR-L2 as depicted in SEQ ID NO: 622, and CDR-L3 as depicted in SEQ ID NO: 623.

Item 20. The antibody construct for use according to any one of the preceding items, wherein the second domain which binds to CD3 comprises a VH region comprising CDR-H1, CDR-H2 and CDR-H3 selected from the group consisting of:
(a) CDR-H1 as depicted in SEQ ID NO: 534, CDR-H2 as depicted in SEQ ID NO: 535, and CDR-H3 as depicted in SEQ ID NO: 536;
(b) CDR-H1 as depicted in SEQ ID NO: 545, CDR-H2 as depicted in SEQ ID NO: 546, and CDR-H3 as depicted in SEQ ID NO: 547;
(c) CDR-H1 as depicted in SEQ ID NO: 557, CDR-H2 as depicted in SEQ ID NO: 558, and CDR-H3 as depicted in SEQ ID NO: 559;
(d) CDR-H1 as depicted in SEQ ID NO: 568, CDR-H2 as depicted in SEQ ID NO: 569, and CDR-H3 as depicted in SEQ ID NO: 570;
(e) CDR-H1 as depicted in SEQ ID NO: 579, CDR-H2 as depicted in SEQ ID NO: 580, and CDR-H3 as depicted in SEQ ID NO: 581;
(f) CDR-H1 as depicted in SEQ ID NO: 591, CDR-H2 as depicted in SEQ ID NO: 592, and CDR-H3 as depicted in SEQ ID NO: 593;
(g) CDR-H1 as depicted in SEQ ID NO: 602, CDR-H2 as depicted in SEQ ID NO: 603, and CDR-H3 as depicted in SEQ ID NO: 604;
(h) CDR-H1 as depicted in SEQ ID NO: 613, CDR-H2 as depicted in SEQ ID NO: 614, and CDR-H3 as depicted in SEQ ID NO: 615;
(i) CDR-H1 as depicted in SEQ ID NO: 624, CDR-H2 as depicted in SEQ ID NO: 625, and CDR-H3 as depicted in SEQ ID NO: 626; and
(j) CDR-H1 as depicted in SEQ ID NO: 636, CDR-H2 as depicted in SEQ ID NO: 637, and CDR-H3 as depicted in SEQ ID NO: 638.

Item 21. The antibody construct for use according to any one of the preceding items, wherein the second domain which binds to CD3 comprises a VL region comprising CDR-L1, CDR-L2 and CDR-L3 and a VH region comprising CDR-H1, CDR-H2 and CDR-H3 selected from the group consisting of:
(a) CDR-L1 as depicted in SEQ ID NO: 531, CDR-L2 as depicted in SEQ ID NO: 532, CDR-L3 as depicted in SEQ ID NO: 533, CDR-H1 as depicted in SEQ ID NO: 534, CDR-H2 as depicted in SEQ ID NO: 535, and CDR-H3 as depicted in SEQ ID NO: 536;
(b) CDR-L1 as depicted in SEQ ID NO: 542, CDR-L2 as depicted in SEQ ID NO: 543, CDR-L3 as depicted in SEQ ID NO: 544, CDR-H1 as depicted in SEQ ID NO: 545, CDR-H2 as depicted in SEQ ID NO: 546, and CDR-H3 as depicted in SEQ ID NO: 547;
(c) CDR-L1 as depicted in SEQ ID NO: 554, CDR-L2 as depicted in SEQ ID NO: 555, CDR-L3 as depicted in SEQ ID NO: 556, CDR-H1 as depicted in SEQ ID NO: 557, CDR-H2 as depicted in SEQ ID NO: 558, and CDR-H3 as depicted in SEQ ID NO: 559;
(d) CDR-L1 as depicted in SEQ ID NO: 565, CDR-L2 as depicted in SEQ ID NO: 566, CDR-L3 as depicted in SEQ ID NO: 567, CDR-H1 as depicted in SEQ ID NO: 568, CDR-H2 as depicted in SEQ ID NO: 569, and CDR-H3 as depicted in SEQ ID NO: 570;
(e) CDR-L1 as depicted in SEQ ID NO: 576, CDR-L2 as depicted in SEQ ID NO: 577, CDR-L3 as depicted in SEQ ID NO: 578, CDR-H1 as depicted in SEQ ID NO: 579, CDR-H2 as depicted in SEQ ID NO: 580, and CDR-H3 as depicted in SEQ ID NO: 581;
(f) CDR-L1 as depicted in SEQ ID NO: 588, CDR-L2 as depicted in SEQ ID NO: 589, CDR-L3 as depicted in SEQ ID NO: 590, CDR-H1 as depicted in SEQ ID NO: 591, CDR-H2 as depicted in SEQ ID NO: 592, and CDR-H3 as depicted in SEQ ID NO: 593;
(g) CDR-L1 as depicted in SEQ ID NO: 599, CDR-L2 as depicted in SEQ ID NO: 600, CDR-L3 as depicted in SEQ ID NO: 601, CDR-H1 as depicted in SEQ ID NO: 602, CDR-H2 as depicted in SEQ ID NO: 603, and CDR-H3 as depicted in SEQ ID NO: 604;
(h) CDR-L1 as depicted in SEQ ID NO: 610, CDR-L2 as depicted in SEQ ID NO: 611, CDR-L3 as depicted in SEQ ID NO: 612, CDR-H1 as depicted in SEQ ID NO: 613, CDR-H2 as depicted in SEQ ID NO: 614, and CDR-H3 as depicted in SEQ ID NO: 615;
(i) CDR-L1 as depicted in SEQ ID NO: 621, CDR-L2 as depicted in SEQ ID NO: 622, CDR-L3 as depicted in SEQ ID NO: 623, CDR-H1 as depicted in SEQ ID NO: 624, CDR-H2 as depicted in SEQ ID NO: 625, and CDR-H3 as depicted in SEQ ID NO: 626; and
(j) CDR-L1 as depicted in SEQ ID NO: 633, CDR-L2 as depicted in SEQ ID NO: 634, CDR-L3 as depicted in SEQ ID NO: 635, CDR-H1 as depicted in SEQ ID NO: 636, CDR-H2 as depicted in SEQ ID NO: 637, and CDR-H3 as depicted in SEQ ID NO: 638.

Item 22. The antibody construct for use according to any one of the preceding items, wherein the second domain which binds to CD3 comprises a VL region having an amino acid sequence selected from the group consisting of those depicted in SEQ ID NO: 550, SEQ ID NO: 551, SEQ ID NO: 584, SEQ ID NO: 585, SEQ ID NO: 629 and SEQ ID NO: 630,preferably SEQ ID NO: 629.

Item 23. The antibody construct for use according to any one of the preceding items, wherein the second domain which binds to CD3 comprises a VH region having an amino acid sequence selected from the group consisting of those depicted in SEQ ID NO: 537, SEQ ID NO: 538, SEQ ID NO: 548, SEQ ID NO: 549, SEQ ID NO: 560, SEQ ID NO: 561, SEQ ID NO: 571, SEQ ID NO: 572, SEQ ID NO: 582, SEQ ID NO: 583, SEQ ID NO: 594, SEQ ID NO: 595, SEQ ID NO: 605, SEQ ID NO: 606, SEQ ID NO: 616, SEQ ID NO: 617, SEQ ID NO: 627, SEQ ID NO: 628, SEQ ID NO: 639, SEQ ID NO: 640, and SEQ ID NO: 644, preferably SEQ ID NO: 639.

Item 24. The antibody construct for use according to any one of the preceding items, wherein the second domain which binds to CD3 comprises a VL region and a VH region selected from the group consisting of:
(a) a VL region as depicted in SEQ ID NO: 539 or 521 and a VH region as depicted in SEQ ID NO: 537 or 538;
(b) a VL region as depicted in SEQ ID NO: 550 or 521 and a VH region as depicted in SEQ ID NO: 548 or 549;
(c) a VL region as depicted in SEQ ID NO: 562 or 521 and a VH region as depicted in SEQ ID NO: 560 or 561;
(d) a VL region as depicted in SEQ ID NO: 573 or 521 and a VH region as depicted in SEQ ID NO: 571 or 572;
(e) a VL region as depicted in SEQ ID NO: 584 or 585 and a VH region as depicted in SEQ ID NO: 582 or 583;
(f) a VL region as depicted in SEQ ID NO: 596 or 521 and a VH region as depicted in SEQ ID NO: 594 or 595;
(g) a VL region as depicted in SEQ ID NO: 607 or 585 and a VH region as depicted in SEQ ID NO: 605 or 606;
(h) a VL region as depicted in SEQ ID NO: 618 or 521 and a VH region as depicted in SEQ ID NO: 616 or 617;
(i) a VL region as depicted in SEQ ID NO: 629 or 630 and a VH region as depicted in SEQ ID NO: 627 or 628;
(j) a VL region as depicted in SEQ ID NO: 641 or 630 and a VH region as depicted in SEQ ID NO: 639 or 640; and
(k) a VL region as depicted in SEQ ID NO: 645 and a VH region as depicted in SEQ ID NO: 644.

Item 25. The antibody construct for use according to any one of the preceding items, wherein the second domain which binds to CD3 comprises or consists of a polypeptide having an amino acid sequence selected from the group consisting of those depicted in SEQ ID NOs: 540, 541, 552, 553, 563, 564, 574, 575, 586, 587, 597, 598, 608, 609, 619, 620, 631, 632, 642, 643, and 646, preferably SEQ ID NO: 642.

Item 26. The antibody construct for use according to any one of the preceding items, which comprises a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530.

Item 27. The antibody construct for use according to any one of the preceding items, comprising in an N-to C-terminal order:
(a) the first domain;
(b) a peptide linker having an amino acid sequence selected from the group consisting of SEQ ID NOs: 687, 693 and 694;
(c) the second domain;
(d) a peptide linker having an amino acid sequence selected from the group consisting of SEQ ID NO: 686, 687, 688, 689, 690, 691, 692, 693 or 694;
(e) a first polypeptide monomer (comprising a hinge, a CH2 domain and a CH3 domain);
(f) a peptide linker having an amino acid sequence selected from the group consisting of SEQ ID NOs: 695, 696, 697, 698 or 699; and
(g) a second polypeptide monomer (comprising a hinge, a CH2 domain and a CH3 domain).

Item 28. The antibody construct for use according to any one of the preceding items, comprising in an N-to C-terminal order:
(a) the first domain having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529; wherein the peptide linker comprised within those sequences and having SEQ ID NO: 694 can be replaced by any one of SEQ ID NOs: 686-693 and 695-699;
(b) a peptide linker having an amino acid sequence selected from the group consisting of SEQ ID NOs: 687, 693 and 694;
(c) the second domain having an amino acid sequence selected from the group consisting of SEQ ID NOs: 540, 541, 552, 553, 563, 564, 574, 575, 586, 587, 597, 598, 608, 609, 619, 620, 631, 632, 642, 643, and 646; wherein the peptide linker comprised within those sequences and having SEQ ID NO: 694 can be replaced by any one of SEQ ID NOs: 686-693 and 695-699;
(d) a peptide linker having an amino acid sequence selected from the group consisting of SEQ ID NOs: 686, 687, 688, 689, 690, 691, 692, 693, and 694; and
(e) the third domain having an amino acid sequence selected from the group consisting of SEQ ID NOs: 700-707.

Item 29. The antibody construct for use according to any one of the preceding items, comprising in an N-to C-terminal order:
(a) a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530;
(b) a linker having an amino acid sequence as depicted in SEQ ID NO: 686; and
(c) a polypeptide having an amino acid sequence as depicted in SEQ ID NO: 700.

Item 30. The antibody construct for use according to any one of the preceding items, which has a molecular weight of about 75 to about 200 kDa, about 80 to about 175 kDa, about 85 to about 150 kDa, about 90 to about 130 kDa, about 95 to about 120 kDa, and preferably about 100 to about 115 kDa or about 105 to about 110 kDa.

Item 31. The antibody construct for use according to any one of the preceding items, which has an elimination half-life (T_{1/2}) of about 3 to about 14 days, about 4 to about 12 days, about 3 or 4 to about 10 days, about 3 or 4 to about 8 days, or about 5 to about 7 days.

### Brief description of the drawings

Figure 1 shows an exemplary dose exploration of AMG 701
Figure 2 shows a week 1 step dosing optimization protocol

### Detailed description of the invention

It is hence an object of the present invention to provide an administration scheme for a half-life extended anti-BCMA x anti-CD3 antibody construct which provides for a favorable safety and tolerability profile, while resulting in a positive efficacy signal in BCMA positive neoplasms.

The present invention therefore relates to an antibody construct comprising a first domain which binds to BCMA, a second domain which binds to CD3 and a third domain which extends the half-life of the antibody construct, for use in the treatment or amelioration of a BCMA positive neoplasm, wherein the antibody construct is administered at a minimum dose of 800 µg/day in at least one cycle, wherein one cycle comprises at least three individual administrations of the antibody construct.

Moreover, the disclosure provides a method for the treatment or amelioration of a BCMA positive neoplasm, comprising administering an antibody construct comprising a first domain which binds to BCMA, a second domain which binds to CD3 and a third domain which extends the half-life of the antibody construct to a subject in need thereof, wherein the antibody construct is administered at a minimum dose of 800 µg/day in at least one cycle, wherein one cycle comprises at least three individual administrations of the antibody construct.

The invention further relates to the use of an antibody construct comprising a first domain which binds to BCMA, a second domain which binds to CD3 and a third domain which extends the half-life of the antibody construct, for the manufacture of a medicament for the treatment of a BCMA positive neoplasm, wherein the antibody construct is administered at a minimum dose of 800 µg/day in at least one cycle, wherein one cycle comprises at least three individual administrations of the antibody construct.

The invention also relates to the use of an antibody construct comprising a first domain which binds to BCMA, a second domain which binds to CD3 and a third domain which extends the half-life of the antibody construct, for the treatment or amelioration of a BCMA positive neoplasm, wherein the antibody construct is administered at a minimum dose of 800 µg/day in at least one cycle, wherein one cycle comprises at least three individual administrations of the antibody construct.

A "neoplasm" is an abnormal growth of tissue, usually but not always forming a mass. When also forming a mass, it is commonly referred to as a "tumor". In brain tumors, the uncontrolled division of cells means that the mass of a neoplasm increases in size, and in a confined space such as the intracranial cavity this quickly becomes problematic because the mass invades the space of the brain pushing it aside, leading to compression of the brain tissue and increased intracranial pressure and destruction of parenchyma. According to the invention, the term "neoplasm" or "tumor" also refers to a condition that would benefit from treatment with the antibody construct as described herein. This includes chronic and acute disorders or diseases, including those pathological conditions that predispose a mammal to the condition (neoplasm or tumor) in question.

Neoplasms or tumors can be benign, potentially malignant (pre-cancerous), or malignant (cancerous). Malignant neoplasms / tumors are commonly called cancer. They usually invade and destroy the surrounding tissue and may form metastases, i.e., they spread to other parts, tissues or organs of the body. A "primary tumor" is a tumor growing at the anatomical site where tumor progression began and proceeded to yield a cancerous mass. For example, a brain tumor occurs when abnormal cells form within the brain. Most cancers develop at their primary site but then go on to form metastases or spread to other parts (e.g. tissues and organs) of the body. These further tumors are secondary tumors. Most cancers continue to be called after their primary site, even after they have spread to other parts of the body.

Lymphomas and leukemias are hematopoietic or lymphoid neoplasms. Multiple Myeloma (MM), also known as plasma cell myeloma, is a cancer of plasma cells, a type of white blood cells that normally produce antibodies. For the purposes of the present invention, lymphomas and leukemias as well as MM are also encompassed by the terms "tumor", "cancer" or "neoplasm". Lymphoma is a group of blood cancers that develop from lymphocytes (a type of white blood cell). Leukemia is a group of cancers that usually begin in the bone marrow and result in high numbers of abnormal white blood cells. These white blood cells are not fully developed and are called blasts or leukemia cells. Lymphomas and leukemias are a part of the broader group of tumors of the hematopoietic and lymphoid tissues.

For the purposes of the present invention, the terms "neoplasm", "tumor" and "cancer" may be used interchangeably, and they comprise both primary tumors / cancers and secondary tumors / cancers (or "metastases"), as well as mass-forming neoplasms (tumors) and lymphoid neoplasms (such as lymphomas and leukemias), and also MRD.

The term "minimal residual disease" (MRD) refers to the evidence for the presence of small numbers of residual cancer cells that remain in the patient after cancer treatment, e.g. when the patient is in remission (the patient has no symptoms or signs of disease). A very small number of remaining cancer cells usually cannot be detected by routine means because the standard tests used to assess or detect cancer are not sensitive enough to detect MRD. Nowadays, very sensitive molecular biology tests for MRD are available, such as flow cytometry, PCR and next-generation sequencing. These tests can measure minimal levels of cancer cells in tissue samples, sometimes as low as one cancer cell in a million normal cells. In the context of the present invention, the terms "prevention", "treatment" or "amelioration" of a neoplasm are envisaged to also encompass "prevention, treatment or amelioration of MRD", whether the MRD was detected or not.

The BCMA positive neoplasm is a B cell neoplasm or a plasma cell neoplasm. B cells, also known as B lymphocytes, are a type of white blood cell of the lymphocyte subtype. They function in the humoral immunity component of the adaptive immune system by secreting antibodies. Additionally, B cells present antigen (they are also classified as professional antigen-presenting cells) and secrete cytokines. In mammals, B cells mature in the bone marrow, which is at the core of most bones. B cells, unlike the other two classes of lymphocytes - T cells and natural killer (NK) cells - express B cell receptors (BCRs) on their cell membrane. BCRs allow the B cell to bind to a specific antigen, against which it will initiate an antibody response. Plasma cells, also called plasma B cells, plasmocytes, or effector B cells, are white blood cells that secrete large volumes of antibodies. They are usually transported by the blood plasma and the lymphatic system. Plasma cells originate in the bone marrow. B cells differentiate into plasma cells that produce antibody molecules closely modelled after the receptors of the precursor B cell. Once released into the blood and lymph, these antibody molecules bind to the target antigen and initiate its neutralization or destruction.

The level of expression of BCMA on the cell surface can be determined e.g. by flow cytometry analysis.

The subpopulation of cells (e.g. B cells, plasma cells, MM cells, CD138+ cells) that is selected for analysis of BCMA expression can e.g. be stained with an anti-BCMA antibody, followed by a secondary antibody, and then analyzed in a FACS assay. A BCMA negative cell line (such as K562, A549, TC71, CCRF-CEM) can be used as control. A shift in the FACS assay (with the BCMA negative cell line defining 0% BCMA expression) indicates that the cells to be analyzed are BCMA positive. Different levels of BCMA expression can exist on the surface cells, such as low, medium or high expression. See also Quinn et al., Blood (2011) 117:890-901 and Sanchez et al, Br J Heamatol 2012 Jul 18.

The "BCMA positive neoplasm" or the "(BCMA positive) B cell neoplasm or plasma cell neoplasm" can be selected from the group including, but not limited to, multiple myeloma, relapsed and/or refractory multiple myeloma, heavy chain multiple myeloma, light chain multiple myeloma, extramedullary myeloma (extramedullary plasmacytoma, extramedullary multiple myeloma), plasmacytoma, plasma cell leukemia, Waldenström's macroglobulinemia (lymphoplasmacytic lymphoma), and smoldering myeloma (smoldering multiple myeloma). The present disclosure hence also relates to an antibody construct for use in the treatment or amelioration of multiple myeloma (MM), plasmacytoma, plasma cell leukemia and Waldenström's macroglobulinemia, as described herein. The MM can be selected from the group consisting of or comprising relapsed and/or refractory multiple myeloma, heavy chain multiple myeloma, light chain multiple myeloma, extramedullary multiple myeloma, and smoldering multiple myeloma.

The antibody construct of the invention (and the pharmaceutical composition comprising such antibody construct) is/are useful in the treatment, amelioration and/or prevention of the BCMA positive neoplasm as described herein in a subject in need thereof. The term "treatment" refers to both therapeutic treatment and prophylactic or preventative measures. Treatment includes the administration of the antibody construct (or the pharmaceutical composition comprising such antibody construct) to the patient's body, to an isolated tissue, or to a cell from a patient or a subject in need who has a BCMA positive neoplasm as described herein, a symptom of such neoplasm, or a predisposition toward such neoplasm, with the purpose to cure, heal, alleviate, relieve, alter, remedy, ameliorate, improve, or affect the BCMA positive neoplasm, one or more symptoms of the BCMA positive neoplasm, or the predisposition toward the disease.

The terms "subject in need", "patient" or those "in need of treatment" include those already with the BCMA positive neoplasm, as well as those in an MRD setting and those in which the neoplasm is to be prevented. The terms include human subjects that receive either prophylactic or therapeutic treatment.

The term "amelioration" as used herein refers to any improvement of the disease state (the disease being a BCMA positive neoplasm) of a patient, by the administration of an antibody construct according to the invention to such patient or subject in need thereof. Such an improvement may be seen as a slowing down the progression or stopping the progression of the disease of the patient, and/or as a decrease in severity of disease symptoms, an increase in frequency or duration of disease symptom-free periods or a prevention of impairment or disability due to the disease.

The term "prevention" as used herein means the avoidance of the occurrence or of the re-occurrence of a disease as specified herein, by the administration of an antibody construct according to the invention to a subject in need thereof.

In the case of Multiple Myeloma, the symptoms and signs vary greatly because many organs can be affected by the disease. The common symptoms of multiple myeloma include elevated calcium levels, renal failure, anemia, and bone lesions (together, "CRAB" features). In advanced MM, bone pain, bleeding, and frequent infections may occur. Complications may also include amyloidosis. The International Myeloma Working Group (IMWG) has established criteria for the diagnosis of MM which teach, in addition to the classic CRAB features, three "myeloma defining events" (MDEs):
- 60% or greater clonal plasma cells on bone marrow examination
- Serum involved / uninvolved free light chain ratio of 100 or greater, provided the absolute level of the involved light chain is at least 100 mg/L (a patient's "involved" free light chain-either kappa or lambda-is the one that is above the normal reference range; the "uninvolved" free light chain is the one that is typically in, or below, the normal range)
- More than one focal lesion on MRI (magnetic resonance imaging) that is at least 5 mm or greater in size

The presence of at least one of these markers is considered sufficient for a diagnosis of multiple myeloma, regardless of the presence or absence of symptoms or CRAB features. See also Palumbo A. J Clin Oncol. 2014 Feb 20; 32(6): 587-600.

Bone pain affects almost 70% of patients and is the most common symptom. It usually involves the spine and ribs. Involvement of the vertebrae may lead to spinal cord compression or kyphosis. Myeloma bone disease is due to the overexpression of receptor activator for nuclear factor κB ligand (RANKL) by bone marrow stroma. RANKL activates osteoclasts, which resorb bone. The resultant bone lesions are lytic (i.e. they cause breakdown) in nature, and are best seen in plain radiographs. The breakdown of bone also leads to the release of calcium into the blood, leading to hypercalcemia and its associated symptoms.

The anemia found in myeloma is usually normocytic and normochromic. It results from the replacement of normal bone marrow by infiltrating tumor cells and inhibition of normal red blood cell production (hematopoiesis) by cytokines.

A bone marrow biopsy can be performed to estimate the percentage of bone marrow occupied by plasma cells. This percentage is used in the diagnostic criteria for MM. Usually, MM patients have ≥ 10% clonal bone marrow plasma cells. Immunohistochemistry (staining particular cell types using antibodies against surface proteins) can detect plasma cells which express immunoglobulin in the cytoplasm and occasionally on the cell surface; for example, myeloma cells are typically positive for the markers CD56, CD38, CD138, CD319, but other markers may be included as well to define or identify MM.

The so-called "paraprotein" (also called myeloma protein, monoclonal protein or M protein) is an abnormal immunoglobulin fragment that is produced in excess by an abnormal monoclonal proliferation of plasma cells, typically in MM. In theory, MM patients can produce all classes of immunoglobulin, but IgG paraproteins are most common, followed by IgA and IgM, while IgD and IgE myeloma are very rare. In addition, antibody light chains and/or heavy chains may be secreted in isolation: kappa or lambda light chains or any of the five types of heavy chains (alpha, gamma, delta, epsilon or my (µ)-heavy chains). This proliferation of the paraprotein has several deleterious effects on the body, including impaired immune function, abnormally high blood viscosity, and kidney damage. Patients without evidence of paraprotein may have "nonsecretory" myeloma (not producing immunoglobulins); they represent approximately 3% of all MM patients. The presence of serum and/or urinary paraprotein is an indicator for MM, except in patients with true nonsecretory MM. Quantitative measurements of the paraprotein in urine and/or serum of a patient can be used to establish a diagnosis and/or to monitor the disease.

Kidney failure may develop both acutely and chronically. The most common cause of kidney failure in MM is due to proteins secreted by the malignant cells. Myeloma cells produce monoclonal proteins of varying types, most commonly immunoglobulins (antibodies) and free light chains, resulting in abnormally high levels of these proteins (paraproteins) in the blood. Depending on the size of these proteins, they may be excreted through the kidneys, but kidneys can also be damaged by their effects. Furthermore, increased bone resorption leads to hypercalcemia and causes nephrocalcinosis, thereby contributing to the kidney failure.

The most common infections occurring in MM are pneumonias and pyelonephritis. The increased risk of infection is due to immune deficiency. Although the total immunoglobulin level is typically elevated in MM, the majority of the antibodies are ineffective monoclonal antibodies from the clonal plasma cell.

It is envisaged that the administration of the antibody construct in the treatment or amelioration of a BCMA positive neoplasm according to the invention
- reduces the level of paraprotein or free light chain in the urine and/or serum by at least about 20%, at least about 30%, at least about 40%, at least about 50%, at least about 60%, at least about 70%, at least about 80%, or at least about 90% relative to the paraprotein or free light chain level in the urine and/or serum, respectively, prior to the start of the treatment, i.e. prior to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before);
- reduces the percentage of plasma cells in the bone marrow by at least about 20%, at least about 30%, at least about 40%, at least about 50%, at least about 60%, at least about 70%, at least about 80%, or at least about 90% relative to the percentage of plasma cells in the bone marrow prior to the start of the treatment, i.e. prior to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before);
- induces a reduction of any of the symptoms described above by at least about 20%, at least about 30%, at least about 40%, at least about 50%, at least about 60%, at least about 70%, at least about 80%, or at least about 90% relative to the symptoms prior to the start of the treatment, i.e. to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before, depending on the symptom);
- inhibits tumor growth or tumor cell proliferation by at least about 20%, at least about 30%, at least about 40%, at least about 50%, at least about 60%, at least about 70%, at least about 80%, or at least about 90% relative to untreated patients or relative to untreated cells; and/or
- induces lysis of the cells of the BCMA positive neoplasm of at least about 20%, at least about 30%, at least about 40%, at least about 50%, at least about 60%, at least about 70%, at least about 80%, or at least about 90% relative to untreated patients or relative to untreated cells.

The ability of an antibody construct of the invention to inhibit tumor growth / tumor cell proliferation or to induce cell lysis may be evaluated in an animal model predictive of efficacy in human tumors, or in an *in vitro* or *ex vivo* study (such as depletion of BCMA positive cells by autologous T cells from a multiple myeloma patient's BM aspirate induced by the antibody construct). Efficacy assessments of the antibody construct may furthermore be performed as follows: Tumor assessment can be done by analysis of percent myeloma involvement, by FISH (fluorescent in situ hybridization) as well as by karyotyping in the bone marrow (BM). Data for BM karyotyping and FISH may be obtained from a BM sample. Serum protein electrophoresis (SPEP) and urine protein electrophoresis (UPEP) allow for measurement of serum / urine M protein. Immunofixation is another means to detect serum and/or urine M protein. It is also envisaged that serum free light chain assay and ratio analysis can be performed. In case of free light chain (FLC) multiple myeloma, FLC can be analyzed in serum and urine (sFLC and uFLC). Levels of involved/uninvolved FLC, ratio of monoclonal lambda-FLC/kappa-FLC, and ratio of monoclonal kappa-FLC/lambda-FLC can be determined. Furthermore, quantitative and qualitative immunoglobulin (Ig) can also be analyzed, and beta-2 microglobulin in serum can be assessed. It is also envisaged that skeletal survey and plasmacytoma assessments can be performed. Screening imaging to evaluate for extramedullary relapse using whole-body MRI or PET/CT can be performed. Imaging appropriate for assessment of bone lesions includes, but is not limited to, CT scan, MRI, PET, PET-CT, or other standard-of-care method. It is also envisaged that minimal residual disease is measured by a next generation sequencing (NGS) based assay. For this purpose, bone marrow aspirates can be collected from subjects suspected to be complete responders. Plasma samples can additionally be collected from subjects at the same time points as BM MRD samples are collected, to assess the feasibility of MRD detection on ctDNA (circulating tumor DNA). MRD response may be defined as <1 tumor cell / 10⁴ normal cells in the bone marrow per FACS using antibodies to cytIgλ, cytIgx, CD19, CD56 or CD138, CD38, and CD45, as needed. In one embodiment of the invention, these markers are a sufficient condition to define a tumor cell in the context of the present invention.

It is envisaged that a patient's or subject's response to the administration of the antibody construct according to the invention is measured in one of the following ways:
- quantitative measurement of the paraprotein (M protein) or free light chain in the urine and/or serum;
- determination of the percentage of plasma cells in the bone marrow; and/or
- imaging (CT, MRI) of extramedullary manifestations.

The invention provides an antibody construct comprising a first domain which binds to BCMA, a second domain which binds to CD3 and a third domain which extends the half-life of the antibody construct, for use in the treatment or amelioration of a BCMA positive neoplasm, wherein the antibody construct is administered at a minimum dose of 800 µg/day in at least one cycle, wherein one cycle comprises at least three individual administrations of the antibody construct.

The antibody construct of the invention is administered in at least one cycle, but more cycles of administration such as 2, 3, 4, 5, 6, 7, 8, 9, 10, 11, 12, 13, 14, 15, 16, 17, 18, 19, 20, 21, 22, 23, 24, 25 or more are also envisaged. The term "at least one cycle" means "one or more cycles". According to the invention, "one cycle" corresponds to a period of about 25 to about 30 or 31 days, of about 26 or 27 to about 29 days, preferably of about 28 days or four weeks. A cycle begins with the first administration of the antibody construct of the invention on "day 1". One cycle may immediately be followed by a subsequent cycle, but an administration-free interval between two cycles is also envisaged. The different administration cycles do not have to be fully identical. In particular, it is envisaged that the first cycle may differ from the following cycles, in particular in terms of the dose of the antibody construct.

The term "a minimum dose of... " means "a dose of at least ... ". The dose of the antibody construct is between 800 µg per day and 12 mg per day. It may be 1000 µg, 1200 µg, 1500 µg, 1600 µg, 2000 µg, 2500 µg, 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg or 12 mg per day.

The dose (the daily dose) of the antibody construct to be administered in the first cycle may be stable throughout the cycle, or it may increase in one or two steps. In this context, "stable" or "stable throughout the cycle" means that the dose to be administered daily (such as 800 µg) is identical / constant in each administration within the cycle. In contrast, a "step" or "dose step" means that there is an increase in the dose (daily dose) to be administered from one administration to the subsequent administration. One dose step within a cycle means that a first dose to be administered on one day is followed by a second (higher) dose to be administered on a subsequent day. Accordingly, two dose steps within a cycle mean that a first dose is followed by a second dose which is higher than the first dose, and the second dose is followed by a third dose which is higher than the second dose. In case there are one or two steps, (at least) the last dose to be administered in the first cycle is the so-called "target dose". The target dose is envisaged to be between 6500 µg per day and 12 mg per day. The target dose may be e.g. 6500 µg, 7000 µg, 7500 µg, 8000 µg, 8500 µg, 9000 µg, 9500 µg, 10 mg, 11 mg or 12 mg per day. It is envisaged that the target dose is reached on day 3, on day 4, on day 5, on day 6, on day 7, on day 8, on day 9 or on day 10 of the first cycle. In case there are one or two steps in the first cycle, it is envisaged the first dose to be administered on day 1 is between 800 µg and 1200 µg per day. In case there are two steps in the first cycle, the second dose to be administered is envisaged to be between 2500 µg and 5000 µg per day, preferably about 3000 µg or about 4500 µg per day. In case there are two steps in the first cycle, the second dose is envisaged to be administered on day 3, day 4 or day 5.

One cycle comprises at least three individual administrations of the antibody construct, preferably four individual administrations. This means that the subject in need receives the antibody construct of the invention at least three times during a cycle, preferably four times. For example, if a cycle consists of four weeks, the subject in need may be administered with the antibody construct once per week. An "individual administration" hence means the administration of a specified dose as defined above on one day. Within one cycle, such individual administration is followed by a period (of usually at least one day) in which the antibody construct is not administered. It is also envisaged that the first cycle comprises or consists of three to six individual administrations, preferably four or five individual administrations of the antibody construct. It is envisaged that the last two or three administrations within the first cycle are at the target dose.

In the first cycle, it is envisaged that the antibody construct is administered on day 1 (d1). Further or subsequent days of administration in the first cycle are envisaged to be d3 or d4 or d5. Further days of administration are envisaged to be d7 or d8 or d9. Further days of administration are envisaged to be d11 or d12 or d13. Further days of administration are envisaged to be d14 or d15 or d16. Further days of administration are envisaged to be d18 or d19 or d20. Further days of administration are envisaged to be d21 or d22 or d23.

According to one embodiment, the first cycle begins with the administration of 800 µg of the antibody construct on day 1. According to a further embodiment, the administration of the antibody construct in the first cycle continues as follows:
e) administration of the target dose on day 8 (+/- one or two days), day 15 (+/- one or two days) and day 22 (+/- one or two days), wherein the target dose may be e.g. 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg or 12 mg per day;
f) administration of the target dose on day 4 or day 5 (+/- one or two days), day 12 (+/- one or two days) and day 19 (+/- one or two days), wherein the target dose may be as defined in a);
g) administration of the target dose on day 3 or day 4 (+/- one or two days), day 8 (+/- one or two days), day 15 (+/- one or two days) and day 22 (+/- one or two days), wherein the target dose may be as defined in a); or
h) administration of a second dose (higher than 800 µg per day) on day 3 or day 4 (+/- one or two days), and of the target dose on day 8 (+/- one or two days), day 15 (+/- one or two days) and day 22 (+/- one or two days). The second dose is envisaged to be between 2500 µg and 5000 µg per day, preferably about 3000 µg or about 4500 µg per day, and the target dose may be as defined in a) and must be higher than the second dose.

It is envisaged that the antibody construct is administered during the second cycle and optionally during any subsequent cycle at constant doses (constant daily doses). No dose steps are envisaged for the second cycle and optionally for any subsequent cycle. Beginning with the second cycle, it is envisaged that the antibody construct is administered at the target dose. The target dose is envisaged to be between 6500 µg and 12 mg per day. The target dose may be e.g. 6500 µg, 7000 µg, 7500 µg, 8000 µg, 8500 µg, 9000 µg, 9500 µg, 10 mg, 11 mg or 12 mg per day. It is envisaged that beginning with the second cycle, the antibody construct is administered in weekly intervals. For example, the administration in the second and any subsequent cycle is on d1, d8 (+/- one or two days), d15 (+/- one or two days) and d22 (+/- one or two days).

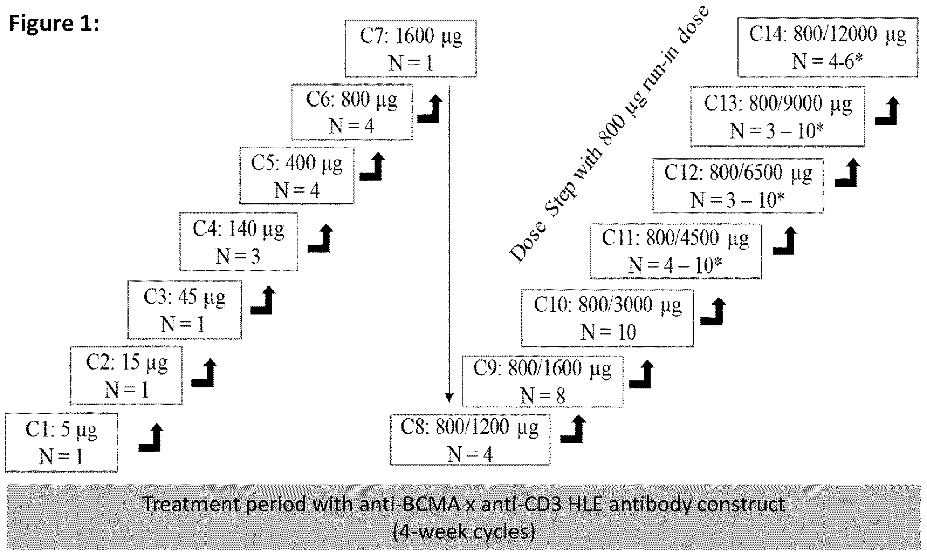

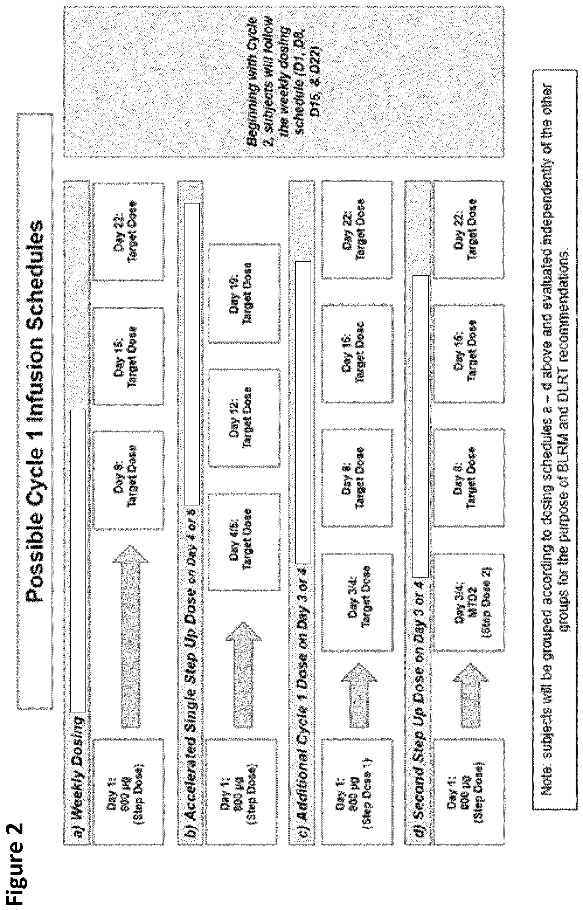

Preferred administration schemes are depicted in Figures 1 and 2.

It is envisaged that the antibody construct of the invention has a molecular weight of about 75 to about 200 kDa, about 80 to about 175 kDa, about 85 to about 150 kDa, about 90 to about 130 kDa, about 95 to about 120 kDa, and preferably about 100 to about 115 kDa or about 105 to about 110 kDa.

The antibody construct of the present invention comprises a domain (the "third domain") which extends or enhances the half-life or the elimination half-life of the construct. The term "extends" or "enhances" may be defined with respect to or in comparison to an antibody construct not comprising such a domain (an "HLE domain"). It is envisaged that the half-life or the elimination half-life of the antibody construct of the invention is at least twice longer than the half-life or the elimination half-life of the antibody construct not comprising the third domain, or at least five times longer, or at least ten times longer, or at least 20 times longer.

The third domain may alternatively be defined as a domain which provides the antibody construct of the invention with a "half-life" or "terminal half-life" or "elimination half-life" (T_{1/2}) of about 3 to about 14 days, about 4 to about 12 days, about 3 or 4 to about 10 days, about 3 or 4 to about 8 days, or about 5 to about 7 days. "Half-life" is the time required for a quantity to reduce to half its initial value. The medical sciences refer to the half-life of substances or drugs (here: the antibody construct) in the human body, e.g. in the serum or in the plasma. Therefore, the "half-life" is sometimes also referred to as "serum half-life" or "plasma half-life". It can be determined by measuring the concentration of the administered antibody construct in the serum / plasma of a subject. Typically, the elimination or removal of an administered substance / drug refers to the body's cleansing through biological processes such as metabolism, excretion, also involving the function of kidneys and liver. "Elimination half-life" may be defined as the time required for the concentration of the drug (here: the antibody construct of the invention) to reach half of its original value.

The antibody construct of the invention will generally be designed for specific routes and methods of administration. A route of administration in pharmacology is the path by which a substance is taken into the body. Routes of administration are generally classified by the location at which the substance is applied, these can be topical (local), enteral (system-wide effect of the substance, but delivered through the gastrointestinal tract), or parenteral (systemic action of the substance, but delivered by routes other than the gastrointestinal tract). In the context of the present invention, the routes of administration include topical, enteral and parenteral routes. The reason for the choice of routes of drug administration are governed by various factors such as:
- Physical and chemical properties of the drug. The physical properties are solid, liquid and gas. The chemical properties are solubility, stability, pH, irritancy etc.
- Site of desired action: The action may be localized and approachable or generalized and not approachable.
- Rate of extent of absorption of the drug from different routes.
- Condition of the patient.

The antibody construct of this invention (and a pharmaceutical composition comprising this antibody construct) is particularly useful for parenteral administration. Parenteral administration generally acts more rapidly than topical or enteral administration, and often comes along with a very high bioavailability of up to 100% (in particular, in the case of IV administration). In general, parenteral administration includes, but is not limited to, intravenous (IV), intracerebral, intraarterial, intraperitoneal, intraosseous, and intravesical delivery.

The administration according to the present invention is preferably intravenous. Parenteral or intravenous administration can be performed by injection (e.g. using a needle and a syringe) or by infusion (e.g. via a catheter and a pump system). It is hence envisaged that the administration according to the present invention is via intravenous injection or via intravenous infusion. Usually, an IV infusion is administered via a line, a port or a catheter (small, flexible tube), such as a central venous access or a central venous catheter (CVC) which is a catheter placed into a large vein, or a peripheral venous catheter (PVC), which is a catheter placed into a peripheral vein. In general, catheters or lines can be placed in veins in the neck (internal jugular vein), chest (subclavian vein or axillary vein), groin (femoral vein), or through veins in the arms (also known as a PICC line, or peripherally inserted central catheters). Central IV lines have their catheters that are advanced through a vein and empty into a large central vein, usually the superior vena cava, inferior vena cava or even the right atrium of the heart. A peripheral intravenous (PIV) line is used on peripheral veins (the veins in the arms, hands, legs and feet). A port is a central venous line that does not have an external connector; instead, it has a small reservoir that is covered with silicone rubber and is implanted under the skin. Medication is administered intermittently by placing a small needle through the skin, piercing the silicone, into the reservoir. When the needle is withdrawn, the reservoir cover reseals itself. The cover can accept hundreds of needle sticks during its lifetime.

The present invention provides for a bolus administration of the antibody construct of the invention. A bolus is the administration of a discrete amount of a medication, drug, or other compound within a specific negligible time, generally within 1-30 minutes. In most cases, the bolus administration is given intravenously. A bolus is usually administered via injection (e.g. an intravenous bolus injection), but a bolus infusion (e.g. an intravenous bolus infusion) is also possible. A short-term infusion is an infusion (usually an IV infusion), of a relatively small volume (such as 50 mL to 500 mL, or 100 mL to 250 mL), which is administered over a period of, at most, three hours, usually of 30 to 60 minutes or about 60 minutes. A short-term (or short-term IV) infusion of the antibody construct is envisaged by the present invention.

Intravenous "intermittent infusion" is an infusion of a volume of medication over a set period of time, such as 20-120 minutes or 30-60 minutes, at prescribed intervals, such as every 4, 6, 8, 10, 12, 14, 16, 18, 20, 22 or 24 hours. The purpose is to administer small amounts of medication at regular intervals. An intermittent medication - like any other form of infusion - may be administered by gravity or via an electronic infusion device (EID), also known as an infusion pump.

In the case of an infusion, an infusion pump may be used to infuse the medication (antibody construct) into a patient's circulatory system. The pump is generally used intravenously, although arterial and epidural infusions with pumps are also possible. The solution for infusion may be prepared in bags for IV infusion and delivered through infusion lines. Infusion pumps can administer fluids in ways that would be unreliable if performed manually. For example, they can administer as little as 0.1 mL per hour injections, injections every minute, injections with repeated boluses, up to a maximum number per hour, or fluids whose volumes vary by the time of day. It is also possible that infusions are administered using only the pressure supplied by gravity. Different types of infusions according to the present invention include, but are not limited to, bolus infusion, short-term infusion, and intermittent infusion. The antibody construct of the present invention may hence be administered e.g. as a bolus administration (bolus injection or bolus infusion), as an injection, or as a short-term infusion, for example over a period of about 30 to about 90 minutes or about 60 minutes.

Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

In the context of the present invention, it is envisaged that a premedication is administered prior to each administration (or "individual administration") of the antibody construct, in particular during the first cycle. It is envisaged that "prior to", in this specific context, means within 24 hours, 18 hours, twelve hours, six hours, five hours, four hours, or three hours, and preferably within 120, 90, 60 or 30 minutes before the administration of the antibody construct. The premedication may e.g. be administered 30-120 or 30-60 minutes prior to the administration of the antibody construct. It is also envisaged that a comedication is administered concurrent with or after the start of administration of the antibody construct in the first cycle, and optionally also concurrent with or after the start of administration of the antibody construct in one or more of the following cycles, as needed by the patient. It is envisaged that "after", in this specific context, means within 24 hours, 18 hours, twelve hours, six hours, five hours, four hours, or three hours, and preferably within 120, 90, 60, 30, 20, 15 or 10 minutes after the start of administration of the antibody construct. The comedication may e.g. be administered 10-120, 10-60, 10-30 or 15-20 minutes after start of administration of the antibody construct. The purpose of the premedication or comedication may be e.g. to prevent or reduce severity of infusion-related reactions. Premedication is preferred over comedication.

The premedication or comedication may include any one or a combination of the following:
- Paracetamol (acetaminophen, APAP) or an equivalent; to be preferably administered orally (p.o.) or intravenously; and to be preferably administered at a dose of 100-4000 mg, preferably 200-3000 mg or 300-2500 mg or 400-2000 mg or 500-1500 mg, preferably 600-1400 mg, 700-1300 mg, 800-1200 mg, 900-1100 mg or about 1000 mg p.o. paracetamol (or an equivalent dose for an equivalent medication and/or another route of administration). The skilled person knows how to identify paracetamol equivalents. They include, but are not limited to, ibuprofen (to be administered e.g. at a dose of 100-3200 mg, preferably 200-3000 mg or 300-2500 mg or 400-2000 mg, preferably 500-1500 mg, 600-1200 mg, 700-1000 mg, 750-900 mg or about 800 mg) and metamizole (to be administered e.g. at a dose of 100-4000 mg, preferably 200-3000 mg or 300-2500 mg or 400-2000 mg, or about 500-1000 mg)
- One or more analgesics selected from meperidine, dipyrone, hydromorphone, fentanyl, and tramadol
- Antihistamine, to be preferably administered orally or intravenously, and to be preferably administered at a dose equivalent to diphenhydramine 50 mg i.v. The skilled person knows how to identify antihistamines. They include, but are not limited to, antihistamines of oral, parenteral or rectal route such as: azatadine (maximum dose e.g. 4 mg/day), brompheniramine (maximum dose e.g. 30 mg/day), cetirizine (maximum dose e.g. 15 mg/day), chlorpheniramine (maximum dose e.g. 30 mg/day), clemastine (maximum dose e.g. 10 mg/day), cyproheptadine (maximum dose e.g. 15 mg/day), desloratadine (maximum dose e.g. 7 mg/day), dexchlorpheniramine (maximum dose e.g. 15 mg/day), diphenhydramine (maximum dose e.g. 350/per day), doxylamine (maximum dose e.g. 180 mg/day), fexofenadine (maximum dose e.g. 200 mg/day), loratadine (maximum dose e.g.15 mg/day), phenindamine (maximum dose e.g. 180 mg/day)
- Glucocorticoid, to be preferably administered orally or intravenously, and to be preferably administered at a dose equivalent to 2-20 mg or 4-16 mg or 6-12 mg or 8 mg dexamethasone i.v. (the equivalence referring to the glucocorticoid potency)

All four substances or substance groups listed above may be administered as premedication or comedication, or a combination of only two or a combination of three substances, or only one of the four substances. It is envisaged that the glucocorticoid (GC) dose administered before the start of the second cycle may be identical to the GC dose administered before start of the first cycle, or may be reduced to about 50% of the dose administered before start of the first cycle, or may be omitted for the second (and/or potentially any subsequent) cycle. A reduction of the GC dosage may apply e.g. if the antibody construct according to the invention is well tolerated without significant signs of infusion-related reactions during the first cycle. It is furthermore envisaged that the dose may further be reduced before start of the third and any subsequent cycle. Alternatively, while GC is administered as premedication (and potentially comedication) before the start of the first cycle, no GC premedication or comedication is administered in the second, third, fourth and/or fifth cycle. In general, the dose of the premedication or comedication that is to be used in accordance with the embodiments of the present invention will depend on the circumstances of the individual patient.

Glucocorticoids are a class of corticosteroids, which are a class of steroid hormones. Glucocorticoids are corticosteroids that bind to the glucocorticoid receptor. A less common synonym is glucocorticosteroid. Cortisol (known as hydrocortisone when used as a medication) is the most important human glucocorticoid. A variety of synthetic glucocorticoids, some far more potent than cortisol, have been created for therapeutic use. They differ in both pharmacokinetics (e.g. absorption factor, half-life, volume of distribution, clearance) and pharmacodynamics (e.g. glucocorticoid potency or mineralocorticoid potency). Cortisol is the standard of comparison for glucocorticoid potency. One example for commonly prescribed replacement steroid equivalents may be prednisone (5 mg) = cortisone (25 mg) = dexamethasone (0.75 mg) = hydrocortisone (20 mg) = methylprednisolone (4 mg). These doses indicate the equivalent pharmacologic dose of systemic glucocorticoids. Another corticosteroid comparison chart indicating the half-lives of the different substances can be found e.g. in www.nadf.us/downloads/adrenalhormone.pdf. Corticosteroid conversion calculators or equivalency tables are available in the internet.

Examples of GCs to be used as premedication or comedication in the present embodiment include, but are not limited to, cortisone, hydrocortisone, prednisone, prednisolone, methylprednisolone, dexamethasone, betamethasone, beclomethasone, budesonide, triamcinolone, cloprednol, deflazacort, fluocortolone, cortivazol, paramethasone, fluticasone, fluticasone propionate, triamcinolone acetonide, as well as combinations and/or pharmaceutically acceptable derivatives thereof. In the context of the present invention, the different GCs may be used alone or in combination. Dexamethasone, prednisone and prednisolone are preferred embodiments of GCs.

It is envisaged that all substances which already are or will be classified as a "glucocorticoid" may be employed in the context of the present invention as well. Such future glucocorticoids include compounds which specifically bind to and activate the glucocorticoid receptor. The term "specifically binds to the GC receptor" means in accordance with the present invention that the GC (or a compound which is assumed to act like a GC) associates with (e.g., interacts with) the GC receptor (also known as NR3C1) to a statistically significant degree as compared to association with proteins/receptors generally (i.e., nonspecific binding). When the GC receptor binds to glucocorticoids, its primary mechanism of action is the regulation of gene transcription. In the absence of GC, the glucocorticoid receptor (GR) resides in the cytosol complexed with a variety of proteins including heat shock protein 90 (hsp90), heat shock protein 70 (hsp70) and the protein FKBP52 (FK506-binding protein 52). Binding of the GC to the glucocorticoid receptor results in release of the heat shock proteins. It is thus envisaged that a future GC, or a pharmaceutically acceptable derivative or salt of a GC, is able to bind to the GC receptor and to release the above mentioned heat shock proteins. The activated GR complex then up-regulates the expression of anti-inflammatory proteins in the nucleus or represses the expression of pro-inflammatory proteins in the cytosol by preventing the translocation of other transcription factors from the cytosol into the nucleus.

The antibody construct of the present invention comprises a first domain which binds to BCMA, a second domain which binds to CD3 and a third domain which extends (or enhances) the half-life of the antibody construct.

The term "antibody construct" refers to a molecule in which the structure and/or function is/are based on the structure and/or function of an antibody, e.g., of a full-length immunoglobulin molecule. An antibody construct hence immunospecifically binds to its target or antigen, and/or it comprises domains which are derived from or which are the heavy chain variable region (VH) and/or the light chain variable region (VL) of an antibody. Furthermore, an antibody construct according to the invention comprises the minimum structural requirements of an antibody which allow for immunospecific target binding. This minimum requirement may e.g. be defined by the presence of at least three light chain CDRs (i.e. CDR1, CDR2 and CDR3 of the VL region) and/or three heavy chain CDRs (i.e. CDR1, CDR2 and CDR3 of the VH region), preferably of all six CDRs.

Within the definition of "antibody" according to the invention are full-length antibodies, also including camelid antibodies and other immunoglobulins generated by biotechnological or protein engineering methods or processes. These full-length antibodies may be for example monoclonal, recombinant, chimeric, deimmunized, humanized and human antibodies, as well as antibodies from other species such as mouse, hamster, rabbit, rat, goat, or non-human primates.

"Antibody constructs" of the present invention may have the general structure of a full-length immunoglobulin as it occurs naturally. For example, they may comprise (at least) two full-length antibody heavy chains and two full-length antibody light chains. However, given that the antibody constructs according to the invention comprise one domain binding to BCMA and another domain binding to CD3, they do not occur naturally, and they are markedly different in their function from naturally occurring products. An antibody construct of the invention is hence an artificial "hybrid" molecule comprising at least two distinct binding domains with different specificities.

"Antibody constructs" of the present invention may also comprise fragments of full-length antibodies, such as VH, VHH, VL, (s)dAb, Fv, light chain (VL-CL), Fd (VH-CH1), heavy chain, Fab, Fab', F(ab')₂ or "r IgG" ("half antibody" consisting of a heavy chain and a light chain). Antibody constructs according to the invention may also comprise modified fragments of antibodies, also called antibody variants or antibody derivatives. Examples include, but are not limited to, scFv, di-scFv or bi(s)-scFv, scFv-Fc, scFv-zipper, scFab, Fab₂, Fab₃, diabodies, single chain diabodies, tandem diabodies (Tandab's), tandem di-scFv, tandem tri-scFv, "minibodies" exemplified by a structure which is as follows: (VH-VL-CH3)₂, (scFv-CH3)₂ , ((scFv)₂-CH3 + CH3), ((scFv)₂-CH3) or (scFv-CH3-scFv)₂, multibodies such as triabodies or tetrabodies, and single domain antibodies such as nanobodies or single variable domain antibodies comprising merely one variable region, which might be VHH, VH or VL, that specifically binds to an antigen or target independently of other variable regions or domains. Further possible formats of the antibody constructs according to the invention are cross bodies, maxi bodies, hetero Fc constructs, mono Fc constructs and scFc constructs.

Furthermore, the definition of the term "antibody construct" includes bivalent and polyvalent / multivalent constructs, which specifically bind to two, three or more antigenic structures, through distinct binding domains. An antibody construct can hence have more binding valences than specificities, e.g. in a case where it has two binding domains for the first target (BCMA) and one binding domain for the second target (CD3) - or vice versa - in which case the construct is trivalent and bispecific. Moreover, the definition of the term "antibody construct" includes molecules consisting of only one polypeptide chain ("single-chain polypeptide") as well as molecules consisting of two, three, four or more polypeptide chains, which chains can be either identical (homodimers, homotrimers or homo oligomers) or different (heterodimer, heterotrimer or heterooligomer). Examples for the above identified antibodies and their fragments, variants, derivatives and antibody constructs derived therefrom are described *inter alia* in Harlow and Lane, Antibodies: A laboratory manual, CSHL Press (1988); Kontermann and Dübel, Antibody Engineering, Springer, 2nd ed. 2010; and Little, Recombinant Antibodies for Immunotherapy, Cambridge University Press 2009. It is envisaged that the antibody construct of the present invention is a single chain antibody construct / a single chain polypeptide.

The term "binding domain" or "domain which binds to..." characterizes in connection with the present invention a domain of the antibody construct which immunospecifically binds to / interacts with / recognizes a given epitope on the target or antigen (here: BCMA in the case of the first domain, and CD3 in the case of the second domain). The structure and function of the first domain (binding to BCMA), and preferably also the structure and/or function of the second domain (binding to CD3), is/are based on the structure and/or function of an antibody, e.g. of a full-length immunoglobulin molecule. The "binding domain" or "domain which binds to..." may hence comprise the minimum structural requirements of an antibody which allow for immunospecific target binding. This minimum structural requirement of the first domain may *e.g*. be defined by the presence of at least three light chain CDRs (i.e. CDR1, CDR2 and CDR3 of the VL region) and/or of three heavy chain CDRs (i.e. CDR1, CDR2 and CDR3 of the VH region), preferably of all six CDRs. It is envisaged that the second domain also comprises this minimum structural requirement of an antibody which allow for the immunospecific target binding. More preferably, the second domain also comprises at least three light chain CDRs (i.e. CDR1, CDR2 and CDR3 of the VL region) and/or three heavy chain CDRs (i.e. CDR1, CDR2 and CDR3 of the VH region), preferably all six CDRs. A "domain which binds to" (or a "binding domain") may typically comprise an antibody light chain variable region (VL) and an antibody heavy chain variable region (VH); however, it does not have to comprise both, but may comprise only one of VH or VL. Fd fragments, for example, often retain some antigen-binding function of the intact antigen-binding domain.

Examples for the format of a "domain which binds to" (or a "binding domain") include, but are not limited to, full-length antibodies, fragments of full-length antibodies (such as VH, VHH, VL), (s)dAb, Fv, light chain (VL-CL), Fd (VH-CH1), heavy chain, Fab, Fab', F(ab')₂ or "r IgG" ("half antibody")), antibody variants or derivatives such as scFv, di-scFv or bi(s)-scFv, scFv-Fc, scFv-zipper, scFab, Fab₂, Fab₃, diabodies, single chain diabodies, tandem diabodies (Tandab's), tandem di-scFv, tandem tri-scFv, "minibodies" (selected from formats such as (VH-VL-CH3)₂, (scFv-CH3)₂, ((scFv)₂-CH3 + CH3)), ((scFv)₂-CH3) or (scFv-CH3-scFv)₂, multibodies such as triabodies or tetrabodies, and single domain antibodies such as nanobodies or single variable domain antibodies comprising merely one variable region, which might be VHH, VH or VL. Further examples for the format of a "domain which binds to" (or a "binding domain") include (1) an antibody fragment or variant comprising VL, VH, CL and CH1 (such as Fab); (2) an antibody fragment or variant comprising two linked Fab fragments (such as a F(ab')₂); (3) an antibody fragment or variant comprising VH and CH₁ (such as Fd); (4) an antibody fragment or variant comprising VL and CL (such as the light chain); (5) an antibody fragment or variant comprising VL and VH (such as Fv); (5) a dAb fragment (Ward et al., (1989) Nature 341 :544-546), which has a VH domain; (6) an antibody variant comprising at least three isolated CDRs of the heavy and/or the light chain; and (7) a single chain Fv (scFv). Examples for embodiments of antibody constructs or binding domains according to the invention are e.g. described in WO 00/006605, WO 2005/040220, WO 2008/119567, WO 2010/037838, WO 2013/026837, WO 2013/026833, US 2014/0308285, US 2014/0302037, W O2014/144722, WO 2014/151910, and WO 2015/048272.

The terms "(specifically or immunospecifically) binds to", "(specifically or immunospecifically) recognizes", or "(specifically or immunospecifically) reacts with" mean in accordance with this invention that an antibody construct or a binding domain interacts or (immuno-)specifically interacts with a given epitope on the target molecule (antigen), here: BCMA and CD3, respectively. This interaction or association occurs more frequently, more rapidly, with greater duration, with greater affinity, or with some combination of the aforementioned, to an epitope on the specific target than to alternative substances (non-target molecules). Because of the sequence similarity between homologous proteins in different species, an antibody construct or a binding domain that immunospecifically binds to its target (such as a human target) may, however, cross-react with homologous target molecules from different species (such as, from non-human primates, e.g. macaque). The term "specific / immunospecific binding" can hence include the binding of an antibody construct or binding domain to epitopes or structurally related epitopes in more than one species.

In the context of the present invention, the term "epitope" refers to the part or region of the antigen that is recognized / immunospecifically recognized by the binding domain. An "epitope" is antigenic, and thus the term epitope is sometimes also referred to as "antigenic structure" or "antigenic determinant". The part of the binding domain that binds to the epitope is called a paratope. Specific binding is believed to be accomplished by specific motifs in the amino acid sequence of the binding domain and the antigen. Thus, binding is achieved as a result of their primary, secondary and/or tertiary structure as well as the result of potential secondary modifications of said structures. The specific interaction of the paratope with its antigenic determinant may result in a simple binding of said site to the antigen. In some cases, the specific interaction may alternatively or additionally result in the initiation of a signal, *e.g*. due to the induction of a change of the conformation of the antigen, an oligomerization of the antigen, etc.

The epitopes of protein antigens are divided into two categories, conformational epitopes and linear epitopes, based on their structure and interaction with the paratope. A conformational epitope is composed of discontinuous sections of the antigen's amino acid sequence. These epitopes interact with the paratope based on the three-dimensional surface features and shape or tertiary structure (folding) of the antigen. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy. By contrast, linear epitopes interact with the paratope based on their primary structure. A linear epitope is formed by a continuous sequence of amino acids from the antigen and typically includes at least 3 or at least 4, and more usually, at least 5 or at least 6 or at least 7, for example, about 8 to about 10 amino acids in a unique sequence.

The interaction between the binding domain and the epitope of the target antigen implies that a binding domain exhibits appreciable or significant affinity for the epitope / the target antigen (here: BCMA and CD3, respectively). In general, it does furthermore not exhibit significant affinity for proteins or antigens other than the target antigen (here: BCMA / CD3) - notwithstanding the above discussed cross-reactivity with homologous targets e.g. from other species. "Significant affinity" includes binding with an affinity (dissociation constant, KD) of ≤10⁻⁶ M. Preferably, binding is considered specific when the binding affinity is ≤10⁻⁷ M, ≤10⁻⁸ M, ≤10⁻⁹ M, ≤10⁻¹⁰ M, or even ≤10⁻¹¹ M, or ≤10⁻¹² M. Whether a binding domain (immuno-)specifically reacts with or binds to a target can be tested readily *e.g*. by comparing the affinity of said binding domain to its desired target protein or antigen with the affinity of said binding domain to non-target proteins or antigens (here: proteins other than BCMA or CD3, respectively). Preferably, an antibody construct of the invention does not significantly bind to proteins or antigens other than BCMA or CD3, respectively (*i.e.,* the first domain does not bind to proteins other than BCMA and the second domain does not bind to proteins other than CD3). For example, it is envisaged that the antibody construct of the invention (and more specifically its first domain) does not significantly bind to, interact with, recognize or cross-react with human BAFF-R and/or human TACI.

The equilibrium dissociation constant (KD) of an antibody construct of the invention to BCMA can be determined by Scatchard or by biacore analysis, as described e.g. in WO 2013/072406. The KD values for CD3 can e.g. be determined by surface plasmon resonance analysis, as described e.g. in WO 2013/072406. It is envisaged that the antibody construct of the present invention has a KD value for BCMA and/or for CD3 in the 2-digit or 1-digit nanomolar range or in the three digit or even two digit picomolar range.

The term "does not significantly bind" means that an antibody construct or binding domain of the present invention does not bind a protein or antigen other than BCMA or CD3, *i.e.,* shows reactivity of ≤30%, preferably ≤20%, more preferably ≤10%, particularly preferably ≤9%, 8%, 7%, 6% or 5% with proteins or antigens other than BCMA or CD3, whereby binding to BCMA or CD3, respectively, is set to be 100%.

According to one embodiment of the antibody construct of the present invention, the first and/or the second domain are in the format of an scFv (single-chain variable fragment). An scFv is not actually a fragment of an antibody, but instead is a fusion protein of the variable regions of the heavy (VH) and light chains (VL) of immunoglobulins, connected with a short linker peptide. The linker is usually rich in glycine for flexibility, as well as serine or threonine for solubility. The scFv is designed to retain the specificity of the original immunoglobulin, despite removal of the constant regions and the introduction of the linker. In an scFv, the VH region and the and VL region are arranged in the order VH-VL or VL-VH (from N- to C-terminus). It is envisaged that the VH and the VL regions of the first and/or the second binding domain are connected via a linker, preferably a peptide linker. According to one embodiment of the first and/or the second domain, the VH-region is positioned N-terminally of the linker, and the VL-region is positioned C-terminally of the linker. It is furthermore envisaged that the first domain and the second domain of the antibody construct are connected via a linker, preferably a peptide linker. The linkers are preferably peptide linkers, more preferably short peptide linkers. Examples are shown in SEQ ID NOs: 686-699. In the present context, a "short" linker has between 2 and 50 amino acids, preferably between 3 and 35, between 4 and 30, between 5 and 25, between 6 and 20 or between 6 and 17amino acids. The linker between two variable regions of one binding domain may have a different length (e.g. may be longer) than the linker between the two binding domains. For example, the linker between two variable regions of one binding domain may have a length between 7 and 15 amino acids, preferably between 9 and 13, and the linker between the two binding domains may have a length between 3 and 10 amino acids, preferably between 4 and 8. It is further envisaged that the peptide linkers are glycine/serine linkers, such as those depicted in SEQ ID NOs: 687, 689-699.

It is envisaged for the antibody construct of the present invention that
a) the antibody construct is a single chain polypeptide,
b) the first domain is in the format of an scFv,
c) the second domain is in the format of an scFv, and/or
d) the first and the second domain are connected via a linker, preferably a peptide linker, more preferably a glycine/serine linker.

The first domain of the antibody construct of the invention binds to BCMA (B cell maturation antigen, TNFRSF17, CD269). More preferably, it binds to BCMA on the surface of a target cell. The "target cell" can be any prokaryotic or eukaryotic cell expressing BCMA on its surface; preferably the target cell is a cell that is part of the human or animal body, such as a specific BCMA expressing cancer or tumor cell or a cell of a BCMA positive neoplasm. It is furthermore envisaged that the first domain binds to human BCMA, preferably to human BCMA on the surface of a target cell. It is also envisaged that the first domain binds to macaque BCMA, preferably to macaque BCMA on the surface of a target cell. A preferred amino acid sequence for human BCMA is depicted in SEQ ID NO: 647, for macaque BCMA in SEQ ID NO: 648, for the extracellular domain of human BCMA in SEQ ID NO: 649, for the extracellular domain of macaque BCMA in SEQ ID NO: 650.

In one embodiment of the present invention, the first domain of the antibody construct binds to epitope cluster 3 of BCMA. More preferably, it binds to epitope cluster 3 of human BCMA. A preferred amino acid sequence for epitope cluster 3 of human BCMA is depicted in SEQ ID NO: 651. Antibody constructs having domains that bind to said epitope cluster 3 of BCMA are described in detail in WO 2013/072406. These antibody constructs are shown in WO 2013/072406 to have a very beneficial epitope / activity relationship.

A method for BCMA epitope mapping is used in WO 2013/072406 and described in the following: One or more pre-defined regions (each in the form of a contiguous amino acid stretch) within the extracellular domain of human BCMA is exchanged / replaced with the corresponding region of a rodent BCMA molecule (such as murine BCMA, but other rodent species are also conceivable, so long as the binding domain is not cross-reactive with the rodent species used). These human BCMA/ rodent (murine) BCMA chimeras are expressed on the surface of host cells (such as CHO cells). Binding of the antibody or antibody construct can be tested via FACS analysis. When the binding of the antibody or antibody construct to the chimeric molecule is entirely abolished, or when a significant binding decrease is observed, it can be concluded that the region of human BCMA which was removed from this chimeric molecule is relevant for the immunospecific epitope-paratope recognition. Said decrease in binding is preferably at least 10%, 20%, 30%, 40%, or 50%; more preferably at least 60%, 70%, or 80%, and most preferably 90%, 95% or even 100% in comparison to the binding to human (wild-type) BCMA, whereby binding to human BCMA is set to be 100%. Alternatively or additionally, the above described epitope mapping analysis can be modified by introducing one or more point mutations into the sequence of the extracellular domain of BCMA.

It is furthermore envisaged for the antibody construct of the present invention that the first domain which binds to BCMA comprises a VH region comprising CDR-H1, CDR-H2 and CDR-H3 and a VL region comprising CDR-L1, CDR-L2 and CDR-L3 selected from:
(1) CDR-H1 as depicted in SEQ ID NO: 1, CDR-H2 as depicted in SEQ ID NO: 2, CDR-H3 as depicted in SEQ ID NO: 3, CDR-L1 as depicted in SEQ ID NO: 4, CDR-L2 as depicted in SEQ ID NO: 5, and CDR-L3 as depicted in SEQ ID NO: 6;
(2) CDR-H1 as depicted in SEQ ID NO: 11, CDR-H2 as depicted in SEQ ID NO: 12, CDR-H3 as depicted in SEQ ID NO: 13, CDR-L1 as depicted in SEQ ID NO: 14, CDR-L2 as depicted in SEQ ID NO: 15, and CDR-L3 as depicted in SEQ ID NO: 16;
(3) CDR-H1 as depicted in SEQ ID NO: 21, CDR-H2 as depicted in SEQ ID NO: 22, CDR-H3 as depicted in SEQ ID NO: 23, CDR-L1 as depicted in SEQ ID NO: 24, CDR-L2 as depicted in SEQ ID NO: 25, and CDR-L3 as depicted in SEQ ID NO: 26;
(4) CDR-H1 as depicted in SEQ ID NO: 31, CDR-H2 as depicted in SEQ ID NO: 32, CDR-H3 as depicted in SEQ ID NO: 33, CDR-L1 as depicted in SEQ ID NO: 34, CDR-L2 as depicted in SEQ ID NO: 35, and CDR-L3 as depicted in SEQ ID NO: 36;
(5) CDR-H1 as depicted in SEQ ID NO: 41, CDR-H2 as depicted in SEQ ID NO: 42, CDR-H3 as depicted in SEQ ID NO: 43, CDR-L1 as depicted in SEQ ID NO: 44, CDR-L2 as depicted in SEQ ID NO: 45, and CDR-L3 as depicted in SEQ ID NO: 46;
(6) CDR-H1 as depicted in SEQ ID NO: 51, CDR-H2 as depicted in SEQ ID NO: 52, CDR-H3 as depicted in SEQ ID NO: 53, CDR-L1 as depicted in SEQ ID NO: 54, CDR-L2 as depicted in SEQ ID NO: 55, and CDR-L3 as depicted in SEQ ID NO: 56;
(7) CDR-H1 as depicted in SEQ ID NO: 61, CDR-H2 as depicted in SEQ ID NO: 62, CDR-H3 as depicted in SEQ ID NO: 63, CDR-L1 as depicted in SEQ ID NO: 64, CDR-L2 as depicted in SEQ ID NO: 65, and CDR-L3 as depicted in SEQ ID NO: 66;
(8) CDR-H1 as depicted in SEQ ID NO: 71, CDR-H2 as depicted in SEQ ID NO: 72, CDR-H3 as depicted in SEQ ID NO: 73, CDR-L1 as depicted in SEQ ID NO: 74, CDR-L2 as depicted in SEQ ID NO: 75, and CDR-L3 as depicted in SEQ ID NO: 76;
(9) CDR-H1 as depicted in SEQ ID NO: 81, CDR-H2 as depicted in SEQ ID NO: 82, CDR-H3 as depicted in SEQ ID NO: 83, CDR-L1 as depicted in SEQ ID NO: 84, CDR-L2 as depicted in SEQ ID NO: 85, and CDR-L3 as depicted in SEQ ID NO: 86;
(10) CDR-H1 as depicted in SEQ ID NO: 91, CDR-H2 as depicted in SEQ ID NO: 92, CDR-H3 as depicted in SEQ ID NO: 93, CDR-L1 as depicted in SEQ ID NO: 94, CDR-L2 as depicted in SEQ ID NO: 95, and CDR-L3 as depicted in SEQ ID NO: 96;
(11) CDR-H1 as depicted in SEQ ID NO: 101, CDR-H2 as depicted in SEQ ID NO: 102, CDR-H3 as depicted in SEQ ID NO: 103, CDR-L1 as depicted in SEQ ID NO: 104, CDR-L2 as depicted in SEQ ID NO: 105, and CDR-L3 as depicted in SEQ ID NO: 106;
(12) CDR-H1 as depicted in SEQ ID NO: 111, CDR-H2 as depicted in SEQ ID NO: 112, CDR-H3 as depicted in SEQ ID NO: 113, CDR-L1 as depicted in SEQ ID NO: 114, CDR-L2 as depicted in SEQ ID NO: 115, and CDR-L3 as depicted in SEQ ID NO: 116;
(13) CDR-H1 as depicted in SEQ ID NO: 121, CDR-H2 as depicted in SEQ ID NO: 122, CDR-H3 as depicted in SEQ ID NO: 123, CDR-L1 as depicted in SEQ ID NO: 124, CDR-L2 as depicted in SEQ ID NO: 125, and CDR-L3 as depicted in SEQ ID NO: 126;
(14) CDR-H1 as depicted in SEQ ID NO: 131, CDR-H2 as depicted in SEQ ID NO: 132, CDR-H3 as depicted in SEQ ID NO: 133, CDR-L1 as depicted in SEQ ID NO: 134, CDR-L2 as depicted in SEQ ID NO: 135, and CDR-L3 as depicted in SEQ ID NO: 136;
(15) CDR-H1 as depicted in SEQ ID NO: 141, CDR-H2 as depicted in SEQ ID NO: 142, CDR-H3 as depicted in SEQ ID NO: 143, CDR-L1 as depicted in SEQ ID NO: 144, CDR-L2 as depicted in SEQ ID NO: 145, and CDR-L3 as depicted in SEQ ID NO: 146;
(16) CDR-H1 as depicted in SEQ ID NO: 151, CDR-H2 as depicted in SEQ ID NO: 152, CDR-H3 as depicted in SEQ ID NO: 153, CDR-L1 as depicted in SEQ ID NO: 154, CDR-L2 as depicted in SEQ ID NO: 155, and CDR-L3 as depicted in SEQ ID NO: 156;
(17) CDR-H1 as depicted in SEQ ID NO: 161, CDR-H2 as depicted in SEQ ID NO: 162, CDR-H3 as depicted in SEQ ID NO: 163, CDR-L1 as depicted in SEQ ID NO: 164, CDR-L2 as depicted in SEQ ID NO: 165, and CDR-L3 as depicted in SEQ ID NO: 166;
(18) CDR-H1 as depicted in SEQ ID NO: 171, CDR-H2 as depicted in SEQ ID NO: 172, CDR-H3 as depicted in SEQ ID NO: 173, CDR-L1 as depicted in SEQ ID NO: 174, CDR-L2 as depicted in SEQ ID NO: 175, and CDR-L3 as depicted in SEQ ID NO: 176;
(19) CDR-H1 as depicted in SEQ ID NO: 181, CDR-H2 as depicted in SEQ ID NO: 182, CDR-H3 as depicted in SEQ ID NO: 183, CDR-L1 as depicted in SEQ ID NO: 184, CDR-L2 as depicted in SEQ ID NO: 185, and CDR-L3 as depicted in SEQ ID NO: 186;
(20) CDR-H1 as depicted in SEQ ID NO: 191, CDR-H2 as depicted in SEQ ID NO: 192, CDR-H3 as depicted in SEQ ID NO: 193, CDR-L1 as depicted in SEQ ID NO: 194, CDR-L2 as depicted in SEQ ID NO: 195, and CDR-L3 as depicted in SEQ ID NO: 196;
(21) CDR-H1 as depicted in SEQ ID NO: 201, CDR-H2 as depicted in SEQ ID NO: 202, CDR-H3 as depicted in SEQ ID NO: 203, CDR-L1 as depicted in SEQ ID NO: 204, CDR-L2 as depicted in SEQ ID NO: 205, and CDR-L3 as depicted in SEQ ID NO: 206;
(22) CDR-H1 as depicted in SEQ ID NO: 211, CDR-H2 as depicted in SEQ ID NO: 212, CDR-H3 as depicted in SEQ ID NO: 213, CDR-L1 as depicted in SEQ ID NO: 214, CDR-L2 as depicted in SEQ ID NO: 215, and CDR-L3 as depicted in SEQ ID NO: 216;
(23) CDR-H1 as depicted in SEQ ID NO: 221, CDR-H2 as depicted in SEQ ID NO: 222, CDR-H3 as depicted in SEQ ID NO: 223, CDR-L1 as depicted in SEQ ID NO: 224, CDR-L2 as depicted in SEQ ID NO: 225, and CDR-L3 as depicted in SEQ ID NO: 226;
(24) CDR-H1 as depicted in SEQ ID NO: 231, CDR-H2 as depicted in SEQ ID NO: 232, CDR-H3 as depicted in SEQ ID NO: 233, CDR-L1 as depicted in SEQ ID NO: 234, CDR-L2 as depicted in SEQ ID NO: 235, and CDR-L3 as depicted in SEQ ID NO: 236;
(25) CDR-H1 as depicted in SEQ ID NO: 241, CDR-H2 as depicted in SEQ ID NO: 242, CDR-H3 as depicted in SEQ ID NO: 243, CDR-L1 as depicted in SEQ ID NO: 244, CDR-L2 as depicted in SEQ ID NO: 245, and CDR-L3 as depicted in SEQ ID NO: 246;
(26) CDR-H1 as depicted in SEQ ID NO: 251, CDR-H2 as depicted in SEQ ID NO: 252, CDR-H3 as depicted in SEQ ID NO: 253, CDR-L1 as depicted in SEQ ID NO: 254, CDR-L2 as depicted in SEQ ID NO: 255, and CDR-L3 as depicted in SEQ ID NO: 256;
(27) CDR-H1 as depicted in SEQ ID NO: 261, CDR-H2 as depicted in SEQ ID NO: 262, CDR-H3 as depicted in SEQ ID NO: 263, CDR-L1 as depicted in SEQ ID NO: 264, CDR-L2 as depicted in SEQ ID NO: 265, and CDR-L3 as depicted in SEQ ID NO: 266;
(28) CDR-H1 as depicted in SEQ ID NO: 271, CDR-H2 as depicted in SEQ ID NO: 272, CDR-H3 as depicted in SEQ ID NO: 273, CDR-L1 as depicted in SEQ ID NO: 274, CDR-L2 as depicted in SEQ ID NO: 275, and CDR-L3 as depicted in SEQ ID NO: 276;
(29) CDR-H1 as depicted in SEQ ID NO: 281, CDR-H2 as depicted in SEQ ID NO: 282, CDR-H3 as depicted in SEQ ID NO: 283, CDR-L1 as depicted in SEQ ID NO: 284, CDR-L2 as depicted in SEQ ID NO: 285, and CDR-L3 as depicted in SEQ ID NO: 286;
(30) CDR-H1 as depicted in SEQ ID NO: 291, CDR-H2 as depicted in SEQ ID NO: 292, CDR-H3 as depicted in SEQ ID NO: 293, CDR-L1 as depicted in SEQ ID NO: 294, CDR-L2 as depicted in SEQ ID NO: 295, and CDR-L3 as depicted in SEQ ID NO: 296;
(31) CDR-H1 as depicted in SEQ ID NO: 301, CDR-H2 as depicted in SEQ ID NO: 302, CDR-H3 as depicted in SEQ ID NO: 303, CDR-L1 as depicted in SEQ ID NO: 304, CDR-L2 as depicted in SEQ ID NO: 305, and CDR-L3 as depicted in SEQ ID NO: 306;
(32) CDR-H1 as depicted in SEQ ID NO: 311, CDR-H2 as depicted in SEQ ID NO: 312, CDR-H3 as depicted in SEQ ID NO: 313, CDR-L1 as depicted in SEQ ID NO: 314, CDR-L2 as depicted in SEQ ID NO: 315, and CDR-L3 as depicted in SEQ ID NO: 316;
(33) CDR-H1 as depicted in SEQ ID NO: 321, CDR-H2 as depicted in SEQ ID NO: 322, CDR-H3 as depicted in SEQ ID NO: 323, CDR-L1 as depicted in SEQ ID NO: 324, CDR-L2 as depicted in SEQ ID NO: 325, and CDR-L3 as depicted in SEQ ID NO: 326;
(34) CDR-H1 as depicted in SEQ ID NO: 331, CDR-H2 as depicted in SEQ ID NO: 332, CDR-H3 as depicted in SEQ ID NO: 333, CDR-L1 as depicted in SEQ ID NO: 334, CDR-L2 as depicted in SEQ ID NO: 335, and CDR-L3 as depicted in SEQ ID NO: 336;
(35) CDR-H1 as depicted in SEQ ID NO: 341, CDR-H2 as depicted in SEQ ID NO: 342, CDR-H3 as depicted in SEQ ID NO: 343, CDR-L1 as depicted in SEQ ID NO: 344, CDR-L2 as depicted in SEQ ID NO: 345, and CDR-L3 as depicted in SEQ ID NO: 346;
(36) CDR-H1 as depicted in SEQ ID NO: 351, CDR-H2 as depicted in SEQ ID NO: 352, CDR-H3 as depicted in SEQ ID NO: 353, CDR-L1 as depicted in SEQ ID NO: 354, CDR-L2 as depicted in SEQ ID NO: 355, and CDR-L3 as depicted in SEQ ID NO: 356;
(37) CDR-H1 as depicted in SEQ ID NO: 361, CDR-H2 as depicted in SEQ ID NO: 362, CDR-H3 as depicted in SEQ ID NO: 363, CDR-L1 as depicted in SEQ ID NO: 364, CDR-L2 as depicted in SEQ ID NO: 365, and CDR-L3 as depicted in SEQ ID NO: 366;
(38) CDR-H1 as depicted in SEQ ID NO: 371, CDR-H2 as depicted in SEQ ID NO: 372, CDR-H3 as depicted in SEQ ID NO: 373, CDR-L1 as depicted in SEQ ID NO: 374, CDR-L2 as depicted in SEQ ID NO: 375, and CDR-L3 as depicted in SEQ ID NO: 376;
(39) CDR-H1 as depicted in SEQ ID NO: 381, CDR-H2 as depicted in SEQ ID NO: 382, CDR-H3 as depicted in SEQ ID NO: 383, CDR-L1 as depicted in SEQ ID NO: 384, CDR-L2 as depicted in SEQ ID NO: 385, and CDR-L3 as depicted in SEQ ID NO: 386;
(40) CDR-H1 as depicted in SEQ ID NO: 391, CDR-H2 as depicted in SEQ ID NO: 392, CDR-H3 as depicted in SEQ ID NO: 393, CDR-L1 as depicted in SEQ ID NO: 394, CDR-L2 as depicted in SEQ ID NO: 395, and CDR-L3 as depicted in SEQ ID NO: 396;
(41) CDR-H1 as depicted in SEQ ID NO: 401, CDR-H2 as depicted in SEQ ID NO: 402, CDR-H3 as depicted in SEQ ID NO: 403, CDR-L1 as depicted in SEQ ID NO: 404, CDR-L2 as depicted in SEQ ID NO: 405, and CDR-L3 as depicted in SEQ ID NO: 406;
(42) CDR-H1 as depicted in SEQ ID NO: 411, CDR-H2 as depicted in SEQ ID NO: 412, CDR-H3 as depicted in SEQ ID NO: 413, CDR-L1 as depicted in SEQ ID NO: 414, CDR-L2 as depicted in SEQ ID NO: 415, and CDR-L3 as depicted in SEQ ID NO: 416;
(43) CDR-H1 as depicted in SEQ ID NO: 421, CDR-H2 as depicted in SEQ ID NO: 422, CDR-H3 as depicted in SEQ ID NO: 423, CDR-L1 as depicted in SEQ ID NO: 424, CDR-L2 as depicted in SEQ ID NO: 425, and CDR-L3 as depicted in SEQ ID NO: 426;
(44) CDR-H1 as depicted in SEQ ID NO: 431, CDR-H2 as depicted in SEQ ID NO: 432, CDR-H3 as depicted in SEQ ID NO: 433, CDR-L1 as depicted in SEQ ID NO: 434, CDR-L2 as depicted in SEQ ID NO: 435, and CDR-L3 as depicted in SEQ ID NO: 436;
(45) CDR-H1 as depicted in SEQ ID NO: 441, CDR-H2 as depicted in SEQ ID NO: 442, CDR-H3 as depicted in SEQ ID NO: 443, CDR-L1 as depicted in SEQ ID NO: 444, CDR-L2 as depicted in SEQ ID NO: 445, and CDR-L3 as depicted in SEQ ID NO: 446;
(46) CDR-H1 as depicted in SEQ ID NO: 451, CDR-H2 as depicted in SEQ ID NO: 452, CDR-H3 as depicted in SEQ ID NO: 453, CDR-L1 as depicted in SEQ ID NO: 454, CDR-L2 as depicted in SEQ ID NO: 455, and CDR-L3 as depicted in SEQ ID NO: 456;
(47) CDR-H1 as depicted in SEQ ID NO: 461, CDR-H2 as depicted in SEQ ID NO: 462, CDR-H3 as depicted in SEQ ID NO: 463, CDR-L1 as depicted in SEQ ID NO: 464, CDR-L2 as depicted in SEQ ID NO: 465, and CDR-L3 as depicted in SEQ ID NO: 466;
(48) CDR-H1 as depicted in SEQ ID NO: 471, CDR-H2 as depicted in SEQ ID NO: 472, CDR-H3 as depicted in SEQ ID NO: 473, CDR-L1 as depicted in SEQ ID NO: 474, CDR-L2 as depicted in SEQ ID NO: 475, and CDR-L3 as depicted in SEQ ID NO: 476;
(49) CDR-H1 as depicted in SEQ ID NO: 481, CDR-H2 as depicted in SEQ ID NO: 482, CDR-H3 as depicted in SEQ ID NO: 483, CDR-L1 as depicted in SEQ ID NO: 484, CDR-L2 as depicted in SEQ ID NO: 485, and CDR-L3 as depicted in SEQ ID NO: 486;
(50) CDR-H1 as depicted in SEQ ID NO: 491, CDR-H2 as depicted in SEQ ID NO: 492, CDR-H3 as depicted in SEQ ID NO: 493, CDR-L1 as depicted in SEQ ID NO: 494, CDR-L2 as depicted in SEQ ID NO: 495, and CDR-L3 as depicted in SEQ ID NO: 496;
(51) CDR-H1 as depicted in SEQ ID NO: 501, CDR-H2 as depicted in SEQ ID NO: 502, CDR-H3 as depicted in SEQ ID NO: 503, CDR-L1 as depicted in SEQ ID NO: 504, CDR-L2 as depicted in SEQ ID NO: 505, and CDR-L3 as depicted in SEQ ID NO: 506;
(52) CDR-H1 as depicted in SEQ ID NO: 511, CDR-H2 as depicted in SEQ ID NO: 512, CDR-H3 as depicted in SEQ ID NO: 513, CDR-L1 as depicted in SEQ ID NO: 514, CDR-L2 as depicted in SEQ ID NO: 515, and CDR-L3 as depicted in SEQ ID NO: 516; and
(53) CDR-H1 as depicted in SEQ ID NO: 521, CDR-H2 as depicted in SEQ ID NO: 522, CDR-H3 as depicted in SEQ ID NO: 523, CDR-L1 as depicted in SEQ ID NO: 524, CDR-L2 as depicted in SEQ ID NO: 525, and CDR-L3 as depicted in SEQ ID NO: 526.

It is also envisaged for the antibody construct of the present invention that the first domain which binds to BCMA comprises a VL region having an amino acid sequence selected from the group consisting of those depicted in SEQ ID NOs: 8, 18, 28, 38, 48, 58, 68, 78, 88, 98, 108, 118, 128, 138, 148, 158, 168, 178, 188, 198, 208, 218, 228, 238, 248, 258, 268, 278, 288, 298, 308, 318, 328, 338, 348, 358, 368, 378, 388, 398, 408, 418, 428, 438, 448, 458, 468, 478, 488, 498, 508, 518, and 528. It is envisaged that the first domain comprises a VL region having an amino acid sequence as depicted in SEQ ID NO: 178.

It is furthermore envisaged that the first domain which binds to BCMA comprises a VH region having an amino acid sequence selected from the group consisting of those depicted in SEQ ID NOs: 7, 17, 27, 37, 47, 57, 67, 77, 87, 97, 107, 117, 127, 137, 147, 157, 167, 177, 187, 197, 207, 217, 227, 237, 247, 257, 267, 277, 287, 307, 317, 327, 337, 347, 357, 367, 377, 387, 397, 407, 417, 427, 437, 447, 457, 467, 477, 487, 497, 507, 517, and 527. It is envisaged that the first domain comprises a VH region having an amino acid sequence as depicted in SEQ ID NO: 177.

It is also envisaged for the antibody construct of the present invention that the first domain which binds to BCMA comprises a VH region and a VL region selected from the group consisting of:
(1) a VH region as depicted in SEQ ID NO: 7 and a VL region as depicted in SEQ ID NO: 8;
(2) a VH region as depicted in SEQ ID NO: 17 and a VL region as depicted in SEQ ID NO: 18;
(3) a VH region as depicted in SEQ ID NO: 27 and a VL region as depicted in SEQ ID NO: 28;
(4) a VH region as depicted in SEQ ID NO: 37 and a VL region as depicted in SEQ ID NO: 38;
(5) a VH region as depicted in SEQ ID NO: 47 and a VL region as depicted in SEQ ID NO: 48;
(6) a VH region as depicted in SEQ ID NO: 57 and a VL region as depicted in SEQ ID NO: 58;
(7) a VH region as depicted in SEQ ID NO: 67 and a VL region as depicted in SEQ ID NO: 68;
(8) a VH region as depicted in SEQ ID NO: 77 and a VL region as depicted in SEQ ID NO: 78;
(9) a VH region as depicted in SEQ ID NO: 87 and a VL region as depicted in SEQ ID NO: 88;
(10) a VH region as depicted in SEQ ID NO: 97 and a VL region as depicted in SEQ ID NO: 98;
(11) a VH region as depicted in SEQ ID NO: 107 and a VL region as depicted in SEQ ID NO: 108;
(12) a VH region as depicted in SEQ ID NO: 117 and a VL region as depicted in SEQ ID NO: 118;
(13) a VH region as depicted in SEQ ID NO: 127 and a VL region as depicted in SEQ ID NO: 128;
(14) a VH region as depicted in SEQ ID NO: 137 and a VL region as depicted in SEQ ID NO: 138;
(15) a VH region as depicted in SEQ ID NO: 147 and a VL region as depicted in SEQ ID NO: 148;
(16) a VH region as depicted in SEQ ID NO: 157 and a VL region as depicted in SEQ ID NO: 158;
(17) a VH region as depicted in SEQ ID NO: 167 and a VL region as depicted in SEQ ID NO: 168;
(18) a VH region as depicted in SEQ ID NO: 177 and a VL region as depicted in SEQ ID NO: 178;
(19) a VH region as depicted in SEQ ID NO: 187 and a VL region as depicted in SEQ ID NO: 188;
(20) a VH region as depicted in SEQ ID NO: 197 and a VL region as depicted in SEQ ID NO: 198;
(21) a VH region as depicted in SEQ ID NO: 207 and a VL region as depicted in SEQ ID NO: 208;
(22) a VH region as depicted in SEQ ID NO: 217 and a VL region as depicted in SEQ ID NO: 218;
(23) a VH region as depicted in SEQ ID NO: 227 and a VL region as depicted in SEQ ID NO: 228;
(24) a VH region as depicted in SEQ ID NO: 237 and a VL region as depicted in SEQ ID NO: 238;
(25) a VH region as depicted in SEQ ID NO: 247 and a VL region as depicted in SEQ ID NO: 248;
(26) a VH region as depicted in SEQ ID NO: 257 and a VL region as depicted in SEQ ID NO: 258;
(27) a VH region as depicted in SEQ ID NO: 267 and a VL region as depicted in SEQ ID NO: 268;
(28) a VH region as depicted in SEQ ID NO: 277 and a VL region as depicted in SEQ ID NO: 278;
(29) a VH region as depicted in SEQ ID NO: 287 and a VL region as depicted in SEQ ID NO: 288;
(30) a VH region as depicted in SEQ ID NO: 297 and a VL region as depicted in SEQ ID NO: 298;
(31) a VH region as depicted in SEQ ID NO: 307 and a VL region as depicted in SEQ ID NO: 308;
(32) a VH region as depicted in SEQ ID NO: 317 and a VL region as depicted in SEQ ID NO: 318;
(33) a VH region as depicted in SEQ ID NO: 327 and a VL region as depicted in SEQ ID NO: 328;
(34) a VH region as depicted in SEQ ID NO: 337 and a VL region as depicted in SEQ ID NO: 338;
(35) a VH region as depicted in SEQ ID NO: 347 and a VL region as depicted in SEQ ID NO: 348;
(36) a VH region as depicted in SEQ ID NO: 357 and a VL region as depicted in SEQ ID NO: 358;
(37) a VH region as depicted in SEQ ID NO: 367 and a VL region as depicted in SEQ ID NO: 368;
(38) a VH region as depicted in SEQ ID NO: 377 and a VL region as depicted in SEQ ID NO: 378;
(39) a VH region as depicted in SEQ ID NO: 387 and a VL region as depicted in SEQ ID NO: 388;
(40) a VH region as depicted in SEQ ID NO: 397 and a VL region as depicted in SEQ ID NO: 398;
(41) a VH region as depicted in SEQ ID NO: 407 and a VL region as depicted in SEQ ID NO: 408;
(42) a VH region as depicted in SEQ ID NO: 417 and a VL region as depicted in SEQ ID NO: 418;
(43) a VH region as depicted in SEQ ID NO: 427 and a VL region as depicted in SEQ ID NO: 428;
(44) a VH region as depicted in SEQ ID NO: 437 and a VL region as depicted in SEQ ID NO: 438;
(45) a VH region as depicted in SEQ ID NO: 447 and a VL region as depicted in SEQ ID NO: 448;
(46) a VH region as depicted in SEQ ID NO: 457 and a VL region as depicted in SEQ ID NO: 458;
(47) a VH region as depicted in SEQ ID NO: 467 and a VL region as depicted in SEQ ID NO: 468;
(48) a VH region as depicted in SEQ ID NO: 477 and a VL region as depicted in SEQ ID NO: 478;
(49) a VH region as depicted in SEQ ID NO: 487 and a VL region as depicted in SEQ ID NO: 488;
(50) a VH region as depicted in SEQ ID NO: 497 and a VL region as depicted in SEQ ID NO: 498;
(51) a VH region as depicted in SEQ ID NO: 507 and a VL region as depicted in SEQ ID NO: 508;
(52) a VH region as depicted in SEQ ID NO: 517 and a VL region as depicted in SEQ ID NO: 518; and
(53) a VH region as depicted in SEQ ID NO: 527 and a VL region as depicted in SEQ ID NO: 528.

It is envisaged that the first domain comprises a VH region having an amino acid sequence as depicted in SEQ ID NO: 177 and a VL region having an amino acid sequence as depicted in SEQ ID NO: 178.

It is also envisaged for the antibody construct of the present invention that the first domain which binds to BCMA comprises a polypeptide having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529. It is envisaged that the first domain comprises a polypeptide having an amino acid sequence as depicted in SEQ ID NO: 179.

"T cells" or T lymphocytes are a type of lymphocyte (itself a type of white blood cell) that play a central role in cell-mediated immunity. There are several subsets of T cells, each with a distinct function. T cells can be distinguished from other lymphocytes, such as B cells and NK cells, by the presence of a T cell receptor (TCR) on the cell surface. The TCR is responsible for recognizing antigens bound to major histocompatibility complex (MHC) molecules and is composed of two different protein chains. In 95% of the T cells, the TCR consists of an alpha (α) and beta (β) chain. When the TCR engages with antigenic peptide and MHC (peptide / MHC complex), the T lymphocyte is activated through a series of biochemical events mediated by associated enzymes, co-receptors, specialized adaptor molecules, and activated or released transcription factors.

"CD3" (cluster of differentiation 3) is a T cell co-receptor composed of four chains. In mammals, the CD3 protein complex contains a CD3γ (gamma) chain, a CD3δ (delta) chain, and two CD3ε (epsilon) chains. These four chains associate with the T cell receptor (TCR) and the so-called ζ (zeta) chain to for the "T cell receptor complex" and to generate an activation signal in T lymphocytes. The CD3γ (gamma), CD3δ (delta), and CD3ε (epsilon) chains are highly related cell-surface proteins of the immunoglobulin superfamily and each contain a single extracellular immunoglobulin domain. The intracellular tails of the CD3 molecules contain a single conserved motif known as an immunoreceptor tyrosine-based activation motif (ITAM), which is essential for the signaling capacity of the TCR. The CD3 epsilon molecule is a polypeptide which in humans is encoded by the *CD3E* gene which resides on chromosome 11.

The redirected lysis of target cells via the recruitment of T cells by an antibody construct which binds to CD3 on the T cell and to a target protein on the target cell generally involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

Cytotoxicity mediated by BCMAxCD3 antibody constructs can be measured in various ways. The "half maximal effective concentration" (EC₅₀) is commonly used as a measure of potency of a biologically active molecule such as an antibody construct of the present invention. It is expressed in molar units. In the present case of measuring cytotoxicity, the EC₅₀ value refers to the concentration of an antibody construct inducing a cytotoxic response (lysis of target cells) halfway between the baseline and the maximum. Effector cells in a cytotoxicity assay can *e.g.* be stimulated enriched (human) CD8 positive T cells or unstimulated (human) peripheral blood mononuclear cells (PBMC). The target cells should express BCMA on the cell surface. Preferably the target cells express (at least) the extracellular domain of BCMA on the cell surface. Target cells can be a cell line (such as CHO) which is stably or transiently transfected with BCMA, e.g. human BCMA. Alternatively, the target cells can be a BCMA positive natural expresser cell line, such as the human multiple myeloma cell line L363 or NCI-H929.

The effector to target cell (E:T) ratio in a cytotoxicity assay is usually about 10:1, but can also vary. Cytotoxic activity of BCMAxCD3 antibody constructs can be measured in a 51-chromium release assay (*e.g.* with an incubation time of about 18 hours) or in a in a FACS-based cytotoxicity assay (*e.g.* with an incubation time of about 48 hours). Modifications of the incubation time (cytotoxic reaction) are also envisaged. Other methods of measuring cytotoxicity are well-known and comprise MTT or MTS assays, ATP-based assays including bioluminescent assays, the sulforhodamine B (SRB) assay, WST assay, clonogenic assay and the ECIS technology.

According to one embodiment, the cytotoxic activity mediated by BCMAxCD3 antibody constructs of the present invention is measured in a FACS-based cytotoxicity assay. It is envisaged that the EC₅₀ value of the BCMAxCD3 antibody constructs is ≤5000 pM or ≤4000 pM, more preferably ≤3000 pM or ≤2000 pM, even more preferably ≤1000 pM or ≤500 pM, even more preferably ≤400 pM or ≤300 pM, even more preferably ≤200 pM, even more preferably ≤100 pM, even more preferably ≤50 pM, even more preferably ≤20 pM or ≤10 pM, and most preferably ≤5 pM. It is also envisaged that the antibody construct of the present invention has an EC50 value in the 3-digit, 2-digit or 1-digit pg/ml range, as determined in a FACS-based cytotoxicity assay. The assay may be carried out with the L363 or NCI-H929 cell line or with BCMA transfected CHO cells as target cells and stimulated enriched (human) CD8 positive T cells or unstimulated (human) PBMC as effector cells. See also WO 2013/072406.

The second domain of the antibody construct of the invention binds to CD3. More preferably, it binds to CD3 on the surface of a T cell. It is furthermore envisaged that the second domain binds to human CD3, preferably to human CD3 on the surface of a T cell. It is also envisaged that the second domain binds to CD3 epsilon. More preferably, it binds to human CD3 epsilon, e.g. to human CD3 epsilon on the surface of a T cell. A preferred amino acid sequence for the extracellular domain of human CD3 epsilon is depicted in SEQ ID NO: 653.

In one embodiment of the present invention, the second domain of the antibody construct binds to human CD3 epsilon (or human CD3 epsilon on the surface of a T cell) and to *Callithrix jacchus* or *Saimiri sciureus* CD3 epsilon. It is also envisaged that the second domain binds to an extracellular epitope of CD3 epsilon, preferably to an extracellular epitope of human CD3 epsilon. It is also envisaged that the second domain binds to an extracellular epitope of the human and the *Macaca* CD3 epsilon chain. One preferred epitope of CD3 epsilon is comprised within amino acid residues 1-27 of the human CD3 epsilon extracellular domain (see SEQ ID NO: 654). Even more specifically, the epitope comprises at least the amino acid sequence Gln-Asp-Gly-Asn-Glu. *Callithrix jacchus* is a new world primate belonging to the family of *Callitrichidae,* while *Saimiri sciureus* is a new world primate belonging to the family of *Cebidae.* Binders having such characteristics are described in detail in WO 2008/119567.

Antibodies or bispecific antibody constructs directed against (human) CD3 or specifically against CD3 epsilon are known in the art, and their CDRs, VH and VL sequences can serve as a basis for the second binding domain of the antibody construct of the invention. For example, Kung et al. reported in 1979 the development of OKT3 (Ortho Kung T3), the first mAb recognizing CD3 (specifically, the epsilon chain of CD3) on human T cells. OKT3 (muromonab) was the first monoclonal antibody of murine origin to become available for therapy in humans. Newer anti-CD3 monoclonal antibodies include otelixizumab (TRX4), teplizumab (MGA031), foralumab and visilizumab, all targeting the epsilon chain of CD3. Bispecific antibody constructs directed against a (cancer) target and CD3 are also being developed and (pre-)clinically tested, and their CD3 binding domain (CDRs, VH, VL) may serve as a basis for the second binding domain of the antibody construct of the invention. Examples include, but are not limited to, Blinatumomab, Solitomab (MT110, AMG 110), Catumaxomab, Duvortuxizumab, Ertumaxomab, Mosunetuzumab, FBTA05 (Bi20, TPBs05), CEA-TCB (RG7802, RO6958688), AFM11, and MGD006 (S80880). Other examples of CD3 binding domains are disclosed e.g. in US 7,994,289 B2, US 7,728,114 B2, US 7,381,803 B1, US 6,706,265 B1.

It is envisaged for the antibody construct of the present invention that the second domain which binds to CD3 comprises a VL region comprising CDR-L1, CDR-L2 and CDR-L3 selected from:
(a) CDR-L1 as depicted in SEQ ID NO: 542, CDR-L2 as depicted in SEQ ID NO: 543, and CDR-L3 as depicted in SEQ ID NO: 544;
(b) CDR-L1 as depicted in SEQ ID NO: 599, CDR-L2 as depicted in SEQ ID NO: 600, and CDR-L3 as depicted in SEQ ID NO: 601; and
(c) CDR-L1 as depicted in SEQ ID NO: 621, CDR-L2 as depicted in SEQ ID NO: 622, and CDR-L3 as depicted in SEQ ID NO: 623.

It is also envisaged for the antibody construct of the present invention that the second domain which binds to CD3 comprises a VH region comprising CDR-H1, CDR-H2 and CDR-H3 selected from:
(a) CDR-H1 as depicted in SEQ ID NO: 534, CDR-H2 as depicted in SEQ ID NO: 535, and CDR-H3 as depicted in SEQ ID NO: 536;
(b) CDR-H1 as depicted in SEQ ID NO: 545, CDR-H2 as depicted in SEQ ID NO: 546, and CDR-H3 as depicted in SEQ ID NO: 547;
(c) CDR-H1 as depicted in SEQ ID NO: 557, CDR-H2 as depicted in SEQ ID NO: 558, and CDR-H3 as depicted in SEQ ID NO: 559;
(d) CDR-H1 as depicted in SEQ ID NO: 568, CDR-H2 as depicted in SEQ ID NO: 569, and CDR-H3 as depicted in SEQ ID NO: 570;
(e) CDR-H1 as depicted in SEQ ID NO: 579, CDR-H2 as depicted in SEQ ID NO: 580, and CDR-H3 as depicted in SEQ ID NO: 581;
(f) CDR-H1 as depicted in SEQ ID NO: 591, CDR-H2 as depicted in SEQ ID NO: 592, and CDR-H3 as depicted in SEQ ID NO: 593;
(g) CDR-H1 as depicted in SEQ ID NO: 602, CDR-H2 as depicted in SEQ ID NO: 603, and CDR-H3 as depicted in SEQ ID NO: 604;
(h) CDR-H1 as depicted in SEQ ID NO: 613, CDR-H2 as depicted in SEQ ID NO: 614, and CDR-H3 as depicted in SEQ ID NO: 615;
(i) CDR-H1 as depicted in SEQ ID NO: 624, CDR-H2 as depicted in SEQ ID NO: 625, and CDR-H3 as depicted in SEQ ID NO: 626; and
(j) CDR-H1 as depicted in SEQ ID NO: 636, CDR-H2 as depicted in SEQ ID NO: 637, and CDR-H3 as depicted in SEQ ID NO: 638.

It is furthermore envisaged for the antibody construct of the present invention that the second domain which binds to CD3 comprises a VL region comprising CDR-L1, CDR-L2 and CDR-L3 and a VH region comprising CDR-H1, CDR-H2 and CDR-H3 selected from:
(a) CDR-L1 as depicted in SEQ ID NO: 531, CDR-L2 as depicted in SEQ ID NO: 532, CDR-L3 as depicted in SEQ ID NO: 533, CDR-H1 as depicted in SEQ ID NO: 534, CDR-H2 as depicted in SEQ ID NO: 535, and CDR-H3 as depicted in SEQ ID NO: 536;
(b) CDR-L1 as depicted in SEQ ID NO: 542, CDR-L2 as depicted in SEQ ID NO: 543, CDR-L3 as depicted in SEQ ID NO: 544, CDR-H1 as depicted in SEQ ID NO: 545, CDR-H2 as depicted in SEQ ID NO: 546, and CDR-H3 as depicted in SEQ ID NO: 547;
(c) CDR-L1 as depicted in SEQ ID NO: 554, CDR-L2 as depicted in SEQ ID NO: 555, CDR-L3 as depicted in SEQ ID NO: 556, CDR-H1 as depicted in SEQ ID NO: 557, CDR-H2 as depicted in SEQ ID NO: 558, and CDR-H3 as depicted in SEQ ID NO: 559;
(d) CDR-L1 as depicted in SEQ ID NO: 565, CDR-L2 as depicted in SEQ ID NO: 566, CDR-L3 as depicted in SEQ ID NO: 567, CDR-H1 as depicted in SEQ ID NO: 568, CDR-H2 as depicted in SEQ ID NO: 569, and CDR-H3 as depicted in SEQ ID NO: 570;
(e) CDR-L1 as depicted in SEQ ID NO: 576, CDR-L2 as depicted in SEQ ID NO: 577, CDR-L3 as depicted in SEQ ID NO: 578, CDR-H1 as depicted in SEQ ID NO: 579, CDR-H2 as depicted in SEQ ID NO: 580, and CDR-H3 as depicted in SEQ ID NO: 581;
(f) CDR-L1 as depicted in SEQ ID NO: 588, CDR-L2 as depicted in SEQ ID NO: 589, CDR-L3 as depicted in SEQ ID NO: 590, CDR-H1 as depicted in SEQ ID NO: 591, CDR-H2 as depicted in SEQ ID NO: 592, and CDR-H3 as depicted in SEQ ID NO: 593;
(g) CDR-L1 as depicted in SEQ ID NO: 599, CDR-L2 as depicted in SEQ ID NO: 600, CDR-L3 as depicted in SEQ ID NO: 601, CDR-H1 as depicted in SEQ ID NO: 602, CDR-H2 as depicted in SEQ ID NO: 603, and CDR-H3 as depicted in SEQ ID NO: 604;
(h) CDR-L1 as depicted in SEQ ID NO: 610, CDR-L2 as depicted in SEQ ID NO: 611, CDR-L3 as depicted in SEQ ID NO: 612, CDR-H1 as depicted in SEQ ID NO: 613, CDR-H2 as depicted in SEQ ID NO: 614, and CDR-H3 as depicted in SEQ ID NO: 615;
(i) CDR-L1 as depicted in SEQ ID NO: 621, CDR-L2 as depicted in SEQ ID NO: 622, CDR-L3 as depicted in SEQ ID NO: 623, CDR-H1 as depicted in SEQ ID NO: 624, CDR-H2 as depicted in SEQ ID NO: 625, and CDR-H3 as depicted in SEQ ID NO: 626; and
(j) CDR-L1 as depicted in SEQ ID NO: 633, CDR-L2 as depicted in SEQ ID NO: 634, CDR-L3 as depicted in SEQ ID NO: 635, CDR-H1 as depicted in SEQ ID NO: 636, CDR-H2 as depicted in SEQ ID NO: 637, and CDR-H3 as depicted in SEQ ID NO: 638.

It is also envisaged for the antibody construct of the present invention that the second domain which binds to CD3 comprises a VL region having an amino acid sequence selected from the group consisting of those depicted in SEQ ID NO: 550, SEQ ID NO: 551, SEQ ID NO: 584, SEQ ID NO: 585, SEQ ID NO: 629 and SEQ ID NO: 630, preferably SEQ ID NO: 629.

It is furthermore envisaged that the second domain which binds to CD3 comprises a VH region having an amino acid sequence selected from the group consisting of those depicted in SEQ ID NO: 537, SEQ ID NO: 538, SEQ ID NO: 548, SEQ ID NO: 549, SEQ ID NO: 560, SEQ ID NO: 561, SEQ ID NO: 571, SEQ ID NO: 572, SEQ ID NO: 582, SEQ ID NO: 583, SEQ ID NO: 594, SEQ ID NO: 595, SEQ ID NO: 605, SEQ ID NO: 606, SEQ ID NO: 616, SEQ ID NO: 617, SEQ ID NO: 627, SEQ ID NO: 628, SEQ ID NO: 639, SEQ ID NO: 640, and SEQ ID NO: 644.

It is also envisaged for the antibody construct of the present invention that the second domain which binds to CD3 comprises a VL region and a VH region selected from the group consisting of:
(a) a VL region as depicted in SEQ ID NO: 539 or 521 and a VH region as depicted in SEQ ID NO: 537 or 538;
(b) a VL region as depicted in SEQ ID NO: 550 or 521 and a VH region as depicted in SEQ ID NO: 548 or 549;
(c) a VL region as depicted in SEQ ID NO: 562 or 521 and a VH region as depicted in SEQ ID NO: 560 or 561;
(d) a VL region as depicted in SEQ ID NO: 573 or 521 and a VH region as depicted in SEQ ID NO: 571 or 572;
(e) a VL region as depicted in SEQ ID NO: 584 or 585 and a VH region as depicted in SEQ ID NO: 582 or 583;
(f) a VL region as depicted in SEQ ID NO: 596 or 521 and a VH region as depicted in SEQ ID NO: 594 or 595;
(g) a VL region as depicted in SEQ ID NO: 607 or 585 and a VH region as depicted in SEQ ID NO: 605 or 606;
(h) a VL region as depicted in SEQ ID NO: 618 or 521 and a VH region as depicted in SEQ ID NO: 616 or 617;
(i) a VL region as depicted in SEQ ID NO: 629 or 630 and a VH region as depicted in SEQ ID NO: 627 or 628;
(j) a VL region as depicted in SEQ ID NO: 641 or 630 and a VH region as depicted in SEQ ID NO: 639 or 640; and
(k) a VL region as depicted in SEQ ID NO: 645 and a VH region as depicted in SEQ ID NO: 644.

It is also envisaged for the antibody construct of the present invention that the second domain which binds to CD3 comprises or consists of a polypeptide having an amino acid sequence selected from the group consisting of those depicted in SEQ ID NOs: 540, 541, 552, 553, 563, 564, 574, 575, 586, 587, 597, 598, 608, 609, 619, 620, 631, 632, 642, 643, and 646, preferably SEQ ID NO: 642.

It is also envisaged that the antibody construct of the present invention competes for binding to CD3 with:
a) an antibody or antibody construct comprising a domain which binds to CD3 on the surface of a T cell, wherein said domain comprises a VH region comprising CDR-H1 as depicted in SEQ ID NO: 636, CDR-H2 as depicted in SEQ ID NO: 637, and CDR-H3 as depicted in SEQ ID NO: 638, and a VL region comprising CDR-L1 as depicted in SEQ ID NO: 633, CDR-L2 as depicted in SEQ ID NO: 634, CDR-L3 as depicted in SEQ ID NO: 635;
b) an antibody or antibody construct comprising a domain which binds to CD3 on the surface of a T cell, wherein said domain comprises a VH region as depicted in SEQ ID NO: 639, and a VL region as depicted in SEQ ID NO: 641;
c) an antibody construct comprising a domain which binds to CD3 on the surface of a T cell, wherein said domain comprises the amino acid sequence as depicted in SEQ ID NO: 642; or
d) an antibody construct having the amino acid sequence as depicted in SEQ ID NO: 661.

It is also envisaged that the antibody construct of the present invention binds to the same epitope of CD3 as:
a) an antibody or antibody construct comprising a domain which binds to CD3 on the surface of a T cell, wherein said domain comprises a VH region comprising CDR-H1 as depicted in SEQ ID NO: 636, CDR-H2 as depicted in SEQ ID NO: 637, and CDR-H3 as depicted in SEQ ID NO: 638, and a VL region comprising CDR-L1 as depicted in SEQ ID NO: 633, CDR-L2 as depicted in SEQ ID NO: 634, CDR-L3 as depicted in SEQ ID NO: 635;
b) an antibody or antibody construct comprising a domain which binds to CD3 on the surface of a T cell, wherein said domain comprises a VH region as depicted in SEQ ID NO: 639, and a VL region as depicted in SEQ ID NO: 641;
c) an antibody construct comprising a domain which binds to CD3 on the surface of a T cell, wherein said domain comprises the amino acid sequence as depicted in SEQ ID NO: 642; or
d) an antibody construct having the amino acid sequence as depicted in SEQ ID NO: 661.

It is furthermore envisaged that the antibody construct of the present invention comprises a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530. It is envisaged that the antibody construct of the present invention comprises a polypeptide having an amino acid sequence as depicted in SEQ ID NO: 180.

It is also envisaged that the antibody construct of the present invention comprises or consists of a polypeptide which has an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530, and which is linked at its N-terminus or at its C-terminus with a protein purification tag, preferably via a peptide bond (amide bond). The linking of the protein purification tag at the C-terminus of the polypeptide is preferred. It is envisaged that the protein purification tag is a short peptide. For example, the length of the short peptide may be 2-30 amino acids, 4-25 amino acids, 5-20 amino acids or 6-19 amino acids. Examples of protein purification tags include, but are not limited to, AU1 epitope (e.g. as depicted in SEQ ID NO: 666), AU5 epitope (e.g. as depicted in SEQ ID NO: 667), T7-tag (e.g. as depicted in SEQ ID NO: 668), V5-tag (e.g. as depicted in SEQ ID NO: 669), B-tag (e.g. as depicted in SEQ ID NO: 670), E2 epitope (e.g. as depicted in SEQ ID NO: 671), FLAG epitope / FLAG tag (e.g. as depicted in SEQ ID NO: 672), Glu-Glu tag (e.g. as depicted in SEQ ID NOs: 673 or 674), HA tag, Histidine affinity tag (e.g. as depicted in SEQ ID NO: 675), HSV epitope (e.g. as depicted in SEQ ID NO: 676), KT3 epitope (e.g. as depicted in SEQ ID NO: 677), Myc epitope (e.g. as depicted in SEQ ID NO: 678), polyarginine tag (5-6 Arg residues), polyaspartate tag (5-16 Asp residues), polyhistidine tag (2-10 His residues, usually 6 His residues, see e.g. SEQ ID NOs: 662-665), polyphenylalanine tag (usually 11 Phe residues), S1 tag (e.g. as depicted in SEQ ID NO: 679), S-tag (e.g. as depicted in SEQ ID NO: 680), Strep-tag (e.g. as depicted in SEQ ID NOs: 681 or 682), universal tag (e.g. as depicted in SEQ ID NO: 683), VSV-G (e.g. as depicted in SEQ ID NO: 684), Protein C (e.g. as depicted in SEQ ID NO: 685), and Protein A. A histidine tag is preferred, especially a 6x His tag (SEQ ID NO: 663).

It is also envisaged that the antibody construct of the present invention binds to the same epitope of BCMA as:
a) an antibody or antibody construct comprising a domain which binds to BCMA on the surface of a target cell, wherein said domain comprises a VH region comprising CDR-H1 as depicted in SEQ ID NO: 171, CDR-H2 as depicted in SEQ ID NO: 172, and CDR-H3 as depicted in SEQ ID NO: 173, and a VL region comprising CDR-L1 as depicted in SEQ ID NO: 174, CDR-L2 as depicted in SEQ ID NO: 175, and CDR-L3 as depicted in SEQ ID NO: 176;
b) an antibody or antibody construct comprising a domain which binds to BCMA on the surface of a target cell, wherein said domain comprises a VH region as depicted in SEQ ID NO: 177, and a VL region as depicted in SEQ ID NO: 178;
c) an antibody construct comprising a domain which binds to BCMA on the surface of a target cell, wherein said domain comprises the amino acid sequence as depicted in SEQ ID NO: 179; or
d) an antibody construct having the amino acid sequence as depicted in SEQ ID NO: 661.

Whether or not an antibody, antibody construct or binding domain binds to the same epitope of BCMA / BCMA on the surface of a target cell as another given antibody, antibody construct or binding domain can be measured by different analyses, e.g. by epitope mapping with chimeric or mutated BCMA molecules, as described in WO 2013/072406. Another possibility to identify the epitope within a target is an Alanine scanning assay (see e.g. Morrison KL & Weiss GA. Curr Opin Chem Biol. 2001 Jun;5(3):302-7), where each residue within the target (here: BCMA) to be analyzed is replaced by alanine, e.g. via site-directed mutagenesis. Alanine is used because of its non-bulky, chemically inert, methyl functional group that nevertheless mimics the secondary structure references that many of the other amino acids possess. Sometimes bulky amino acids such as valine or leucine can be used in cases where conservation of the size of mutated residues is desired. Alanine scanning is usually accomplished by site-directed mutagenesis or randomly by creating a PCR library. Furthermore, computational methods to estimate thermodynamic parameters based on theoretical alanine substitutions have been developed. The data can be tested by IR, NMR Spectroscopy, mathematical methods, bioassays, etc. The same analysis can of course be applied for other targets such as CD3.

It is also envisaged that the antibody construct of the present invention competes for binding to BCMA with:
a) an antibody or antibody construct comprising a domain which binds to BCMA on the surface of a target cell, wherein said domain comprises a VH region comprising CDR-H1 as depicted in SEQ ID NO: 171, CDR-H2 as depicted in SEQ ID NO: 172, and CDR-H3 as depicted in SEQ ID NO: 173, and a VL region comprising CDR-L1 as depicted in SEQ ID NO: 174, CDR-L2 as depicted in SEQ ID NO: 175, and CDR-L3 as depicted in SEQ ID NO: 176;
b) an antibody or antibody construct comprising a domain which binds to BCMA on the surface of a target cell, wherein said domain comprises a VH region as depicted in SEQ ID NO: 177, and a VL region as depicted in SEQ ID NO: 178;
c) an antibody construct comprising a domain which binds to BCMA on the surface of a target cell, wherein said domain comprises the amino acid sequence as depicted in SEQ ID NO: 179; or
d) an antibody construct having the amino acid sequence as depicted in SEQ ID NO: 661.

Whether or not an antibody or antibody construct competes for binding to BCMA / BCMA on the surface of a target cell with another given antibody or antibody construct can be measured in a competition assay such as a competitive ELISA or a cell-based competition assay (using either cells that naturally express BCMA or cells that were stably or transiently transformed with BCMA). Avidin-coupled microparticles (beads) can also be used. Similar to an avidin-coated ELISA plate, when reacted with a biotinylated protein, each of these beads can be used as a substrate on which an assay can be performed. Antigen is coated onto a bead and then precoated with the first antibody. The second antibody is added, and any additional binding is determined. Read-out occurs via flow cytometry. The term "competes for binding", in the present context, means that competition occurs between the two tested antibodies of at least 20%, at least 30%, at least 40%, at least 50%, at least 60%, at least 70%, at least 80% or at least 90%, as determined by any one of the assays disclosed above. The same analysis can of course be applied for other targets such as CD3.

The antibody construct described herein comprises a third domain which extends or enhances the half-life (or "serum half-life") of the antibody construct. Examples for means or domains to extend serum half-life of the antibody constructs of the invention include peptides, proteins or domains of proteins, which are fused or otherwise attached to the antibody constructs. The group of peptides, proteins or protein domains includes peptides binding to other proteins with preferred pharmacokinetic profile in the human body such as serum albumin (see WO 2009/127691). An alternative concept of such half-life extending peptides includes peptides binding to the neonatal Fc receptor (FcRn, see WO 2007/098420), which can also be used for the antibody constructs of the present invention. The concept of attaching larger domains of proteins or complete proteins includes the fusion of human serum albumin, variants or mutants of human serum albumin (see WO 2011/051489, WO 2012/059486, WO 2012/150319, WO 2013/135896, WO 2014/072481, WO 2013/075066) or domains thereof, as well as the fusion of an immunoglobulin constant region (Fc domain) and variants thereof. Such variants of Fc domains are called Fc-based domains and may be further optimized / modified in order to allow the desired pairing of dimers or multimers, to abolish Fc receptor binding (e.g. to avoid ADCC or CDC) or for other reasons. A further concept known in the art to extend the half-life of substances or molecules in the human body is the pegylation of those molecules (such as the antibody constructs of the present invention).

In one embodiment, the antibody constructs according to the invention are linked (e.g. via peptide bond) with a fusion partner (such as a protein, polypeptide or peptide) for the purpose of extending the construct's serum half-life. These fusion partners (representing the "third domain") can be selected from human serum albumin ("HSA" or "HALB") as wells as sequence variants thereof, peptides binding to HSA, peptides binding to FcRn ("FcRn BP"), or constructs comprising an (antibody derived) Fc region. In general, the fusion partners may be linked to the N-terminus or to the C-terminus of the antibody constructs according to the invention, either directly (e.g. via peptide bond) or through a peptide linker such as (GGGGS)n (wherein "n" is an integer of 2 or greater, e.g. 2 or 3 or 4). Suitable peptide linkers are depicted in SEQ ID NOs: 686-694.

According to a further embodiment, the third domain of the antibody construct of the invention comprises two polypeptide monomers, each comprising a hinge, a CH2 domain and a CH3 domain, wherein said two polypeptide monomers are fused to each other via a peptide linker. It is envisaged that said third domain comprises in an N-terminal to C-terminal order: "first polypeptide monomer - linker - second polypeptide monomer" or hinge-CH2-CH3-linker-hinge-CH2-CH3. Amino acid sequences that can be used for said third domain are depicted in SEQ ID NOs: 700-707. Each of said polypeptide monomers can have an amino acid sequence that is selected from the group consisting of SEQ ID NOs: 708-715, or that is at least 90% identical to those sequences. In another embodiment, the first and second domains of the antibody construct of the invention are fused to the third domain via a peptide linker which is for example selected from the group consisting of SEQ ID NO: 686, 687, 688, 689, 690, 691, 692, 693 or 694.

In line with the present invention, a "hinge" is an IgG hinge region. This region can be identified by analogy using the Kabat numbering, see e.g. Kabat positions 223-243. In line with the above, the minimal requirement for a "hinge" are the amino acid residues corresponding to the IgG₁ sequence stretch of D231 to P243 according to the Kabat numbering. The terms "CH2" and "CH3" (or "CH2 domain" and "CH3 domain") refer to the immunoglobulin heavy chain constant regions 2 and 3. These regions can as well be identified by analogy using the Kabat numbering, see e.g. Kabat positions 244-360 for CH2 and Kabat positions 361-478 for CH3. It is understood that there is some variation between the immunoglobulins in terms of their IgG₁ Fc region, IgG₂ Fc region, IgG₃ Fc region, IgG₄ Fc region, IgM Fc region, IgA Fc region, IgD Fc region and IgE Fc region (see, e.g., Padlan, Molecular Immunology, 31(3), 169-217 (1993)). The term Fc region refers to the last two heavy chain constant regions of IgA, IgD, and IgG, and the last three heavy chain constant regions of IgE and IgM. The Fc region can also include the flexible hinge N-terminal to these domains. For IgA and IgM, the Fc region may include the J chain. For IgG, the Fc region comprises immunoglobulin domains CH2 and CH3 and the hinge between the first two domains and CH2. Although the boundaries of the Fc region of an immunoglobulin may vary, an example for a human IgG heavy chain Fc portion comprising a functional hinge, CH2 domain and CH3 domain can be defined e.g. to comprise residues D231 (of the hinge domain) to P476 (of the C-terminus of the CH3 domain), or D231 to L476, respectively, for IgG₄, wherein the numbering is according to Kabat.

The antibody construct of the invention may hence comprise in an N- to C-terminal order:
(a) the first domain;
(b) a peptide linker having an amino acid sequence selected from the group consisting of SEQ ID NOs: 687, 693 and 694;
(c) the second domain;
(d) a peptide linker having an amino acid sequence selected from the group consisting of SEQ ID NO: 686, 687, 688, 689, 690, 691, 692, 693 or 694;
(e) the first polypeptide monomer of the third domain (comprising a hinge, a CH2 domain and a CH3 domain);
(f) a peptide linker having an amino acid sequence selected from the group consisting of SEQ ID NOs: 695, 696, 697, 698 or 699; and
(g) the second polypeptide monomer of the third domain (comprising a hinge, a CH2 domain and a CH3 domain).

It is also envisaged that the antibody construct of the invention comprises in an N- to C-terminal order:
(f) the first domain having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529; wherein the peptide linker comprised within those sequences and having SEQ ID NO: 694 can be replaced by any one of SEQ ID NOs: 686-693 and 695-699;
(g) a peptide linker having an amino acid sequence selected from the group consisting of SEQ ID NOs: 687, 693 and 694;
(h) the second domain having an amino acid sequence selected from the group consisting of SEQ ID NOs: 540, 541, 552, 553, 563, 564, 574, 575, 586, 587, 597, 598, 608, 609, 619, 620, 631, 632, 642, 643, and 646; wherein the peptide linker comprised within those sequences and having SEQ ID NO: 694 can be replaced by any one of SEQ ID NOs: 686-693 and 695-699;
(i) a peptide linker having an amino acid sequence selected from the group consisting of SEQ ID NOs: 686, 687, 688, 689, 690, 691, 692, 693, and 694; and
(j) the third domain having an amino acid sequence selected from the group consisting of SEQ ID NOs: 700-707.

Hence, in one embodiment, the antibody construct of the present invention comprises in an N- to C-terminal order:
(a) a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530;
(b) a linker having an amino acid sequence as depicted in SEQ ID NO: 686; and
(c) the third domain having an amino acid sequence as depicted in SEQ ID NO: 700.
A preferred antibody construct of the present invention comprises or consists of a polypeptide having the amino acid sequence as depicted in SEQ ID NO: 661.

The antibody construct of the present invention, or one or both of its binding domains, may be "humanized" or "human". "Humanized" antibodies, variants or fragments thereof, antibody constructs and binding domains are based on immunoglobulins of mostly human sequences, which contain (a) minimal sequence(s) derived from non-human immunoglobulin. For the most part, humanized antibodies, variants or fragments thereof, antibody constructs and binding domains are based on human immunoglobulins (recipient antibodies) in which residues from a hypervariable region or CDR are replaced by residues from a hypervariable region or CDR of a non-human species (donor antibody) such as a rodent (e.g. mouse, hamster, rat or rabbit) having the desired specificity, affinity, capacity and/or biological activity. In some instances, Fv framework region (FR) residues of the human immunoglobulin are replaced by corresponding non-human residues. Furthermore, "humanized" antibodies, variants or fragments thereof, antibody constructs and binding domains as used herein may also comprise residues which are found neither in the recipient antibody nor the donor antibody. These modifications are made to further refine and optimize antibody performance. The humanized antibodies, variants or fragments thereof, antibody constructs and binding domains may also comprise at least a portion of an immunoglobulin constant region (such as Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

Human anti-mouse antibody (HAMA) responses have led the industry to prepare chimeric or otherwise humanized antibodies / antibody constructs. It is however expected that certain human anti-chimeric antibody (HACA) responses will be observed, particularly in chronic or multi-dose utilizations of an antibody or antibody construct. Thus, it would be desirable to provide antibody constructs comprising a human binding domain against BCMA and/or a human binding domain against CD3, in order to vitiate concerns and/or effects of HAMA or HACA response.

Therefore, according to one embodiment, the antibody construct, the first binding domain and/or the second binding domain are "human". The term "human antibody", "human antibody construct" and "human binding domain" includes antibodies, antibody constructs and binding domains, respectively, having antibody-derived regions such as variable and constant regions or domains which correspond substantially to human germline immunoglobulin sequences known in the art, including, for example, those described by Kabat *et al.* (1991) (*loc. cit*.). The human antibody constructs or binding domains of the invention may include amino acid residues not encoded by human germline immunoglobulin sequences (*e.g.,* mutations introduced by random or site-specific mutagenesis *in vitro* or by somatic mutation *in vivo*), for example in the CDRs, and in particular in CDR3. The human antibody constructs or binding domains can have at least one, two, three, four, five, or more positions replaced with an amino acid residue that is not encoded by the human germline immunoglobulin sequence. The definition of human antibodies, antibody constructs and binding domains as used herein also contemplates fully human antibodies, antibody constructs and binding domains which include only non-artificially and/or genetically altered human sequences of antibodies as those can be derived by using technologies or systems such as the Xenomouse.

Antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. To avoid the use of rodent-derived antibody constructs, humanized or fully human antibody constructs can be generated through the introduction of human antibody function into a rodent so that the rodent produces fully human antibodies.

In some embodiments, the antibody construct of the invention is an "isolated" or "substantially pure" antibody construct. "Isolated" or "substantially pure", when used to describe the antibody constructs disclosed herein, means an antibody construct that has been identified, separated and/or recovered from a component of its production environment. Preferably, the antibody construct is free or substantially free of association with all other components from its production environment. Contaminant components of its production environment, such as that resulting from recombinant transfected cells, are materials that could interfere with diagnostic or therapeutic uses for the antibody construct, and may include enzymes, hormones, and other proteinaceous or non-proteinaceous compounds. It is understood that the isolated or substantially pure antibody construct may constitute from 5% to 99.9% by weight of the total protein / polypeptide content in a given sample, depending on the circumstances. The desired antibody construct may be produced at a significantly higher concentration through the use of an inducible promoter or high expression promoter. The definition includes the production of an antibody construct in a wide variety of organisms and/or host cells that are known in the art. In certain embodiments, the antibody construct will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver staining. Usually, however, an isolated antibody construct will be prepared by at least one purification step.

According to one embodiment, the entire antibody construct and/or the binding domains are in the form of one or more polypeptides or in the form of proteins. In addition to proteinaceous parts, such polypeptides or proteins may include non-proteinaceous parts (e.g. chemical linkers or chemical crosslinking agents such as glutaraldehyde).

Peptides are short chains of amino acid monomers linked by covalent peptide (amide) bonds. Hence, peptides fall under the broad chemical classes of biological oligomers and polymers. Amino acids that are part of a peptide or polypeptide chain are termed "residues" and can be consecutively numbered. All peptides except cyclic peptides have an N-terminal residue at one end and a C-terminal residue at the other end of the peptide. An oligopeptide consists of only a few amino acids (usually between two and twenty). A polypeptide is a longer, continuous, and unbranched peptide chain. Peptides are distinguished from proteins on the basis of size, and as an arbitrary benchmark can be understood to contain approximately 50 or fewer amino acids. Proteins consist of one or more polypeptides, usually arranged in a biologically functional way. While aspects of the lab techniques applied to peptides versus polypeptides and proteins differ (e.g., the specifics of electrophoresis, chromatography, etc.), the size boundaries that distinguish peptides from polypeptides and proteins are not absolute. Therefore, in the context of the present invention, the terms "peptide", "polypeptide" and "protein" may be used interchangeably, and the term "polypeptide" is often preferred.

Polypeptides may further form multimers such as dimers, trimers and higher oligomers, which consist of more than one polypeptide molecule. Polypeptide molecules forming such dimers, trimers etc. may be identical or non-identical. The corresponding structures of higher order of such multimers are, consequently, termed homo- or heterodimers, homo- or heterotrimers etc. An example for a hereteromultimer is an antibody or immunoglobulin molecule, which, in its naturally occurring form, consists of two identical light polypeptide chains and two identical heavy polypeptide chains. The terms "peptide", "polypeptide" and "protein" also refer to naturally modified peptides / polypeptides / proteins wherein the modification is accomplished e.g. by post-translational modifications like glycosylation, acetylation, phosphorylation and the like. A "peptide", "polypeptide" or "protein" when referred to herein may also be chemically modified such as pegylated. Such modifications are well known in the art.

The antibody construct of the present invention is typically formulated in a pharmaceutical composition or a formulation. Materials of a pharmaceutical composition are usually formulated in concentrations that are acceptable for the site of administration. Formulations and compositions thus may be designed in accordance with the invention for delivery by any suitable route of administration.

As used herein, the term "pharmaceutical composition" relates to a composition which is suitable for administration to a human patient. The particularly preferred pharmaceutical composition of this invention comprises one or a plurality of the antibody construct(s) of the invention, usually in a therapeutically effective amount. The pharmaceutical composition may further comprise suitable formulations of one or more (pharmaceutically effective) carriers, stabilizers, excipients, diluents, solubilizers, surfactants, emulsifiers, preservatives and/or adjuvants. Acceptable constituents of the composition are typically nontoxic to recipients at the dosages and concentrations employed. Pharmaceutical compositions of the invention include, but are not limited to, liquid, frozen, and lyophilized compositions. If the pharmaceutical composition has been lyophilized, the lyophilized material is reconstituted in an appropriate liquid prior to administration. The lyophilized material may e.g. be reconstituted in bacteriostatic water for injection (BWFI), physiological saline, phosphate buffered saline (PBS), or the same formulation the protein had been in prior to lyophilization.

The compositions may comprise a pharmaceutically acceptable carrier. In general, as used herein, "pharmaceutically acceptable carrier" means any and all aqueous and non-aqueous solutions, sterile solutions, solvents, buffers, e.g. phosphate buffered saline (PBS) solutions, water, suspensions, emulsions, such as oil/water emulsions, various types of wetting agents, liposomes, dispersion media and coatings, which are compatible with pharmaceutical administration, in particular with parenteral administration. The use of such media and agents in pharmaceutical compositions is well known in the art, and the compositions comprising such carriers can be formulated by well-known conventional methods.

Certain embodiments provide pharmaceutical compositions comprising the antibody construct of the invention and further one or more excipients. Excipients can be used for a wide variety of purposes, such as adjusting physical, chemical, or biological properties of formulations, such as adjustment of viscosity, and or processes of the invention to improve effectiveness and/or to stabilize such formulations and processes against degradation and spoilage e.g. due to stresses that occur during manufacturing, shipping, storage, pre-use preparation, administration, and thereafter. Excipients should in general be used in their lowest effective concentrations.

In certain embodiments, the pharmaceutical composition may also contain formulation materials / substances for the purpose of modifying, maintaining or preserving certain characteristics of the composition such as the pH, osmolarity, viscosity, clarity, color, isotonicity, odor, sterility, stability, rate of dissolution or release, adsorption or penetration (see, Remington's Pharmaceutical Sciences, 18" Edition, 1990, Mack Publishing Company).

As used herein, the singular forms "a", "an", and "the" include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within ±20%, preferably within ±15%, more preferably within ±10%, and most preferably within ±5% of a given value or range. It also includes the concrete value, e.g., "about 50" includes the value "50".

Throughout this specification and the claims, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of' does not exclude materials or steps that do not materially affect the basic and novel characteristics of the claim.

In each instance herein, any of the terms "comprising", "consisting essentially of' and "consisting of' may be replaced with either of the other two terms.

It should be understood that the above description and the below examples provide exemplary arrangements, but the present invention is not limited to the particular methodologies, techniques, protocols, material, reagents, substances, etc., described herein and as such can vary. The terminology used herein is for the purpose of describing particular embodiments only, and is not intended to limit the scope of the present invention, which is defined solely by the claims. Aspects of the invention are provided in the independent claims. Some optional features of the invention are provided in the dependent claims.

All publications and patents cited throughout the text of this specification (including all patents, patent applications, scientific publications, manufacturer's specifications, instructions, etc.), whether *supra* or *infra,* are referenced herein. Nothing herein is to be construed as an admission that the invention is not entitled to antedate such disclosure by virtue of prior invention. To the extent the material referenced contradicts or is inconsistent with this specification, the specification will supersede any such material.

A better understanding of the present invention and of its advantages will be obtained from the following examples, offered for illustrative purposes only. The examples are not intended and should not be construed as to limit the scope of the present invention in any way.

### EXAMPLES

Background: AMG 701, an HLE BiTE^{®} molecule which binds BCMA on Multiple Myeloma (MM) cells and CD3 on T cells, has activity in MM preclinical models. Objectives of this first-in-human study include evaluating safety and estimating the maximum tolerated dose (MTD) of AMG 701 in patients with relapsed/refractory (R/R) Multiple Myeloma (NCT03287908).

Methods: Weekly short-term IV infusions of AMG 701 were administered in 4-week cycles for as long as patients benefitted, first to single-patient cohorts (5, 15, 45 µg), then cohorts of ≥3 patients (140, 400, 800, 1200 µg), and possibly up to 10 patients in higher-dose cohorts (1600, 3000, 4500, 6500, 9000, 12000 µg). Eligible patients had MM relapsed after or intolerant to ≥2 lines with a proteasome inhibitor, IMiD, and anti-CD38 MAb (where approved and available). Excluded were patients with solely extramedullary disease (allowed in subsequent dose expansion), CNS involvement, prior BCMA-directed therapy, and immunosuppressive therapy. After a grade 3 dose-limiting toxicity (DLT) of cytokine release syndrome (CRS) at 1600 µg, for all target doses ≥1200 µg, an initial run-in dose of 800 µg was added, followed by step up to the target dose. MRD was defined as <1/105 cells, as measured per the FDA-authorized next generation sequencing assay

Results (not including the follow-up results shown in Table 2): 47 patients received AMG 701 at doses ranging from 5 to 6500 µg, with an 800 µg run-in dose used for step-up to target doses of ≥1200 µg; patients are yet to be enrolled at additional planned doses of 9000 and 12000 µg, see also Figure 1. Median age was 62 years, 21 patients (78%) had ≥5 prior lines of therapy. 12 patients are ongoing, MTD as defined per protocol has not yet been reached. No anti-AMG 701 antibodies were detected. Responses included 1 stringent complete response (sCR) at 800 µg and 4 partial responses (PRs), 2 PRs at 3000 µg, 1 PR at 4500 µg, and 1 PR and a minimal response at 6500 µg. The patient with the sCR had an initial PR at 1 month, deepening to sCR at 4.3 months; this ongoing response has lasted 13.2 months, 9.8 of which were MRD-negative. The 4 PRs started at 1-3.7 months (responses were first assessed at 1 month), and the responses lasted up to 4 months (three ongoing).

Conclusions: In this FIH study with ongoing dose escalation and MTD not yet identified, AMG 701, an HLE antibody construct targeting CD3 and BCMA, showed encouraging activity in patients with heavily pre-treated R/R MM.

**Table 1: Results of the AMG 701 FIH study. The table does not include the single-patient cohorts 1-3 of 5, 15, and 45 µg. + indicates responses are ongoing. * Save for 1 patient at 1600 µg, i.e. the patient with the DLT of CRS, all patients receiving doses of ≥1200 µg received a step dose of 800 µg prior to receiving the target dose on Day 5 or 8. The # of patients receiving the target dose on Day 5 is as follows: 1600 µg (n=3), 3000 µg (n=2), and 4500 µg (n=2); all other patients received the target dose on Day 8.**

| Cohort: target dose (µg)* | # of patients at dose | Best response | Any response (months) | |
|---|---|---|---|---|
| | | | Onset | Duration |
| 4: 140 | 3 | - | | |
| 5: 400 | 4 | - | | |
| 6: 800 | 4 | sCR | 1.0 | 13.2+ |
| 7: 1600 | 1 | - | | |
| 8: 1200 | 4 | - | | |
| 9: 1600 | 8 | - | | |
| 10: 3000 | 10 | PR | 3.7 | 3.7+ |
| | | PR | 1.8 | 4.0 |
| 11: 4500 | 6 | PR | 1.9 | 1.0+ |
| 12: 6500 | 3 | PR | 1.0 (pend PR) | NA (pend PR) |
| | | MR | | |
| 13: 9000 | 3-10 | - | | |
| 14: 12000 | 4-6 | - | | |

**Table 2: Follow-up of the results of the AMG 701 FIH study. The table does not include the single-patient cohorts 1-3 of 5, 15, and 45 µg, and the table does not include cohorts 4-9 of 140 µg to 1600 µg (see Table 1). The sCR responder of cohort 6 (800 µg) in Table 1 has a response duration of 19.6+ months.**

| Cohort | Target dose (µg) | Day to target dose | # of patients at dose | Best response | Any response (months) | |
|---|---|---|---|---|---|---|
| | | | | | Onset | Duration |
| 10 | 3000 | 8 | 11 | PR | 1.8 | 3.8 |
| | | 8 | | PR | 2.8 | 7.4 |
| | | 5 | | sCR | 1.0 | 3.9+ |
| 11 | 4500 | 8 | 7 | VGPR | 2.8 | 6.5+ |
| | | 5 | | VGPR | 1.9 | 4.7+ |
| 12 | 6500 | 8 | 9 | PR | 1.0 | 2.8+ |
| | | 8 | | VGPR | 1.9 | 5.8+ |
| | | 3 | | CR | 1.0 | 1.9+ |
| | | 3 | | VGPR | 1.0 | 6.5+ |

| | | | | | | |
|---|---|---|---|---|---|---|
| + indicates responses are ongoing. All patients shown in this Table 2 received a step dose of 800 µg prior to receiving the target dose on day 3, 5 or 8. | | | | | | |

The IMWG response criteria for a complete response (see also http://imwg.myeloma.org/internationalmyeloma-working-group-imwg-uniform-response-criteria-for-multiple-myeloma/) are:
- Negative M protein immunofixation on the serum and urine,
- Disappearance of any soft tissue plasmacytomas, and
- < 5% plasma cells in bone marrow

The IMWG response criteria for a partial response are:
- M protein electrophoresis: ≥ 50% reduction of serum M-protein and reduction in 24 hours urinary M-protein by ≥90% or to <200 mg/24 h
- Free light chains (FLC): If the serum and urine M protein are unmeasurable, a ≥ 50% decrease in the difference between involved and uninvolved FLC levels is required in place of the M-protein criteria
- If serum and urine M protein are not measurable, and serum free light assay is also not measurable: ≥ 50% reduction in plasma cells is required in place of M protein, provided baseline bone marrow plasma cell percentage was ≥ 30%
- In addition to the above listed criteria, if present at baseline, a ≥ 50% reduction in the size of soft tissue plasmacytomas is also required

The IMWG response criteria for a very good partial response are:
- Serum and urine M protein detectable by immunofixation but not on electrophoresis or ≥ 90% reduction in serum M-protein plus urine M-protein level < 100 mg/24 h

## Claims

1. An antibody construct comprising a first domain which binds to BCMA, a second domain which binds to CD3 and a third domain which extends the plasma half-life of the antibody construct, for use in the treatment or amelioration of a BCMA positive neoplasm in a human, wherein the neoplasm is a B cell neoplasm or a plasma cell neoplasm, wherein the antibody construct is administered at a minimum dose of 800 µg/day in at least one cycle, wherein one cycle comprises at least three individual administrations of the antibody construct, wherein one individual administration is the administration on one day.

2. The antibody construct for use according to claim 1,
(I) which is administered for 2, 3, 4, 5, 6, 7, 8, 9, 10, 11, 12, 13, 14, 15, 16, 17, 18, 19, 20, 21, 22, 23, 24, 25 or more cycles;
(II) wherein one cycle has about 25 to about 30 days, preferably about 26 or 27 to about 29 days, and more preferably about 28 days;
(III) wherein the antibody construct is administered at a dose of 800 µg/day to 12 mg per day, such as 1000 µg, 1200 µg, 1500 µg, 1600 µg, 2000 µg, 2500 µg, 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg or 12 mg per day;
(IV) wherein the antibody construct is administered in one or two dose steps during the first cycle, wherein a first dose is preferably between 800 µg/day and 1200 µg/day, an optional second dose is between 2500 µg/day and 5000 µg/day, preferably about 3000 µg/day or about 4500 µg/day, and a last dose (target dose) is between 6500 µg/day and 12 mg/day, such as 6500 µg/day, 7000 µg/day, 7500 µg/day, 8000 µg/day, 8500 µg/day, 9000 µg/day, 9500 µg/day, 10 mg/day, 11 mg/day or 12 mg/day; and/or
(V) wherein the first cycle comprises or consists of three to six individual administrations, preferably four or five individual administrations of the antibody construct.

3. The antibody construct for use according to claim 2, wherein the antibody construct is administered during the second cycle and optionally during any subsequent cycle at constant doses.

4. The antibody construct for use according to any one of the preceding claims,
(I) which is administered parenterally, preferably intravenously, and more preferably via intravenous bolus injection, bolus infusion or short-term intravenous infusion;
(II) wherein the BCMA positive neoplasm is selected from the group consisting of multiple myeloma, relapsed and/or refractory multiple myeloma, heavy chain multiple myeloma, light chain multiple myeloma, extramedullary myeloma, plasmacytoma, plasma cell leukemia, Waldenström's macroglobulinemia, and smoldering myeloma; and/or
(III) wherein
a) the antibody construct is a single chain polypeptide,
b) the first domain is in the format of an scFv,
c) the second domain is in the format of an scFv, and/or
d) the first domain and the second domain are connected via a linker, preferably a peptide linker, more preferably a glycine/serine linker.

5. The antibody construct for use according to any one of the preceding claims,
(I) which competes for binding to BCMA with or which binds to the same epitope of BCMA as:
a) an antibody or antibody construct comprising a domain which binds to BCMA on the surface of a target cell, wherein said domain comprises a VH region comprising CDR-H1 as depicted in SEQ ID NO: 171, CDR-H2 as depicted in SEQ ID NO: 172, and CDR-H3 as depicted in SEQ ID NO: 173, and a VL region comprising CDR-L1 as depicted in SEQ ID NO: 174, CDR-L2 as depicted in SEQ ID NO: 175, and CDR-L3 as depicted in SEQ ID NO: 176;
b) an antibody or antibody construct comprising a domain which binds to BCMA on the surface of a target cell, wherein said domain comprises a VH region as depicted in SEQ ID NO: 177, and a VL region as depicted in SEQ ID NO: 178;
c) an antibody construct comprising a domain which binds to BCMA on the surface of a target cell, wherein said domain comprises the amino acid sequence as depicted in SEQ ID NO: 179; or
d) an antibody construct having the amino acid sequence as depicted in SEQ ID NO: 661; and/or
(II) which competes for binding to CD3 with or which binds to the same epitope of CD3 as:
a) an antibody or antibody construct comprising a domain which binds to CD3 on the surface of a T cell, wherein said domain comprises a VH region comprising CDR-H1 as depicted in SEQ ID NO: 636, CDR-H2 as depicted in SEQ ID NO: 637, and CDR-H3 as depicted in SEQ ID NO: 638, and a VL region comprising CDR-L1 as depicted in SEQ ID NO: 633, CDR-L2 as depicted in SEQ ID NO: 634, CDR-L3 as depicted in SEQ ID NO: 635;
b) an antibody or antibody construct comprising a domain which binds to CD3 on the surface of a T cell, wherein said domain comprises a VH region as depicted in SEQ ID NO: 639, and a VL region as depicted in SEQ ID NO: 641;
c) an antibody construct comprising a domain which binds to CD3 on the surface of a T cell, wherein said domain comprises the amino acid sequence as depicted in SEQ ID NO: 642; or
d) an antibody construct having the amino acid sequence as depicted in SEQ ID NO: 661.

6. The antibody construct for use according to any one of the preceding claims, wherein the first domain which binds to BCMA comprises a VH region comprising CDR-H1, CDR-H2 and CDR-H3 and a VL region comprising CDR-L1, CDR-L2 and CDR-L3 selected from:
(1) CDR-H1 as depicted in SEQ ID NO: 1, CDR-H2 as depicted in SEQ ID NO: 2, CDR-H3 as depicted in SEQ ID NO: 3, CDR-L1 as depicted in SEQ ID NO: 4, CDR-L2 as depicted in SEQ ID NO: 5, and CDR-L3 as depicted in SEQ ID NO: 6;
(2) CDR-H1 as depicted in SEQ ID NO: 11, CDR-H2 as depicted in SEQ ID NO: 12, CDR-H3 as depicted in SEQ ID NO: 13, CDR-L1 as depicted in SEQ ID NO: 14, CDR-L2 as depicted in SEQ ID NO: 15, and CDR-L3 as depicted in SEQ ID NO: 16;
(3) CDR-H1 as depicted in SEQ ID NO: 21, CDR-H2 as depicted in SEQ ID NO: 22, CDR-H3 as depicted in SEQ ID NO: 23, CDR-L1 as depicted in SEQ ID NO: 24, CDR-L2 as depicted in SEQ ID NO: 25, and CDR-L3 as depicted in SEQ ID NO: 26;
(4) CDR-H1 as depicted in SEQ ID NO: 31, CDR-H2 as depicted in SEQ ID NO: 32, CDR-H3 as depicted in SEQ ID NO: 33, CDR-L1 as depicted in SEQ ID NO: 34, CDR-L2 as depicted in SEQ ID NO: 35, and CDR-L3 as depicted in SEQ ID NO: 36;
(5) CDR-H1 as depicted in SEQ ID NO: 41, CDR-H2 as depicted in SEQ ID NO: 42, CDR-H3 as depicted in SEQ ID NO: 43, CDR-L1 as depicted in SEQ ID NO: 44, CDR-L2 as depicted in SEQ ID NO: 45, and CDR-L3 as depicted in SEQ ID NO: 46;
(6) CDR-H1 as depicted in SEQ ID NO: 51, CDR-H2 as depicted in SEQ ID NO: 52, CDR-H3 as depicted in SEQ ID NO: 53, CDR-L1 as depicted in SEQ ID NO: 54, CDR-L2 as depicted in SEQ ID NO: 55, and CDR-L3 as depicted in SEQ ID NO: 56;
(7) CDR-H1 as depicted in SEQ ID NO: 61, CDR-H2 as depicted in SEQ ID NO: 62, CDR-H3 as depicted in SEQ ID NO: 63, CDR-L1 as depicted in SEQ ID NO: 64, CDR-L2 as depicted in SEQ ID NO: 65, and CDR-L3 as depicted in SEQ ID NO: 66;
(8) CDR-H1 as depicted in SEQ ID NO: 71, CDR-H2 as depicted in SEQ ID NO: 72, CDR-H3 as depicted in SEQ ID NO: 73, CDR-L1 as depicted in SEQ ID NO: 74, CDR-L2 as depicted in SEQ ID NO: 75, and CDR-L3 as depicted in SEQ ID NO: 76;
(9) CDR-H1 as depicted in SEQ ID NO: 81, CDR-H2 as depicted in SEQ ID NO: 82, CDR-H3 as depicted in SEQ ID NO: 83, CDR-L1 as depicted in SEQ ID NO: 84, CDR-L2 as depicted in SEQ ID NO: 85, and CDR-L3 as depicted in SEQ ID NO: 86;
(10) CDR-H1 as depicted in SEQ ID NO: 91, CDR-H2 as depicted in SEQ ID NO: 92, CDR-H3 as depicted in SEQ ID NO: 93, CDR-L1 as depicted in SEQ ID NO: 94, CDR-L2 as depicted in SEQ ID NO: 95, and CDR-L3 as depicted in SEQ ID NO: 96;
(11) CDR-H1 as depicted in SEQ ID NO: 101, CDR-H2 as depicted in SEQ ID NO: 102, CDR-H3 as depicted in SEQ ID NO: 103, CDR-L1 as depicted in SEQ ID NO: 104, CDR-L2 as depicted in SEQ ID NO: 105, and CDR-L3 as depicted in SEQ ID NO: 106;
(12) CDR-H1 as depicted in SEQ ID NO: 111, CDR-H2 as depicted in SEQ ID NO: 112, CDR-H3 as depicted in SEQ ID NO: 113, CDR-L1 as depicted in SEQ ID NO: 114, CDR-L2 as depicted in SEQ ID NO: 115, and CDR-L3 as depicted in SEQ ID NO: 116;
(13) CDR-H1 as depicted in SEQ ID NO: 121, CDR-H2 as depicted in SEQ ID NO: 122, CDR-H3 as depicted in SEQ ID NO: 123, CDR-L1 as depicted in SEQ ID NO: 124, CDR-L2 as depicted in SEQ ID NO: 125, and CDR-L3 as depicted in SEQ ID NO: 126;
(14) CDR-H1 as depicted in SEQ ID NO: 131, CDR-H2 as depicted in SEQ ID NO: 132, CDR-H3 as depicted in SEQ ID NO: 133, CDR-L1 as depicted in SEQ ID NO: 134, CDR-L2 as depicted in SEQ ID NO: 135, and CDR-L3 as depicted in SEQ ID NO: 136;
(15) CDR-H1 as depicted in SEQ ID NO: 141, CDR-H2 as depicted in SEQ ID NO: 142, CDR-H3 as depicted in SEQ ID NO: 143, CDR-L1 as depicted in SEQ ID NO: 144, CDR-L2 as depicted in SEQ ID NO: 145, and CDR-L3 as depicted in SEQ ID NO: 146;
(16) CDR-H1 as depicted in SEQ ID NO: 151, CDR-H2 as depicted in SEQ ID NO: 152, CDR-H3 as depicted in SEQ ID NO: 153, CDR-L1 as depicted in SEQ ID NO: 154, CDR-L2 as depicted in SEQ ID NO: 155, and CDR-L3 as depicted in SEQ ID NO: 156;
(17) CDR-H1 as depicted in SEQ ID NO: 161, CDR-H2 as depicted in SEQ ID NO: 162, CDR-H3 as depicted in SEQ ID NO: 163, CDR-L1 as depicted in SEQ ID NO: 164, CDR-L2 as depicted in SEQ ID NO: 165, and CDR-L3 as depicted in SEQ ID NO: 166;
(18) CDR-H1 as depicted in SEQ ID NO: 171, CDR-H2 as depicted in SEQ ID NO: 172, CDR-H3 as depicted in SEQ ID NO: 173, CDR-L1 as depicted in SEQ ID NO: 174, CDR-L2 as depicted in SEQ ID NO: 175, and CDR-L3 as depicted in SEQ ID NO: 176;
(19) CDR-H1 as depicted in SEQ ID NO: 181, CDR-H2 as depicted in SEQ ID NO: 182, CDR-H3 as depicted in SEQ ID NO: 183, CDR-L1 as depicted in SEQ ID NO: 184, CDR-L2 as depicted in SEQ ID NO: 185, and CDR-L3 as depicted in SEQ ID NO: 186;
(20) CDR-H1 as depicted in SEQ ID NO: 191, CDR-H2 as depicted in SEQ ID NO: 192, CDR-H3 as depicted in SEQ ID NO: 193, CDR-L1 as depicted in SEQ ID NO: 194, CDR-L2 as depicted in SEQ ID NO: 195, and CDR-L3 as depicted in SEQ ID NO: 196;
(21) CDR-H1 as depicted in SEQ ID NO: 201, CDR-H2 as depicted in SEQ ID NO: 202, CDR-H3 as depicted in SEQ ID NO: 203, CDR-L1 as depicted in SEQ ID NO: 204, CDR-L2 as depicted in SEQ ID NO: 205, and CDR-L3 as depicted in SEQ ID NO: 206;
(22) CDR-H1 as depicted in SEQ ID NO: 211, CDR-H2 as depicted in SEQ ID NO: 212, CDR-H3 as depicted in SEQ ID NO: 213, CDR-L1 as depicted in SEQ ID NO: 214, CDR-L2 as depicted in SEQ ID NO: 215, and CDR-L3 as depicted in SEQ ID NO: 216;
(23) CDR-H1 as depicted in SEQ ID NO: 221, CDR-H2 as depicted in SEQ ID NO: 222, CDR-H3 as depicted in SEQ ID NO: 223, CDR-L1 as depicted in SEQ ID NO: 224, CDR-L2 as depicted in SEQ ID NO: 225, and CDR-L3 as depicted in SEQ ID NO: 226;
(24) CDR-H1 as depicted in SEQ ID NO: 231, CDR-H2 as depicted in SEQ ID NO: 232, CDR-H3 as depicted in SEQ ID NO: 233, CDR-L1 as depicted in SEQ ID NO: 234, CDR-L2 as depicted in SEQ ID NO: 235, and CDR-L3 as depicted in SEQ ID NO: 236;
(25) CDR-H1 as depicted in SEQ ID NO: 241, CDR-H2 as depicted in SEQ ID NO: 242, CDR-H3 as depicted in SEQ ID NO: 243, CDR-L1 as depicted in SEQ ID NO: 244, CDR-L2 as depicted in SEQ ID NO: 245, and CDR-L3 as depicted in SEQ ID NO: 246;
(26) CDR-H1 as depicted in SEQ ID NO: 251, CDR-H2 as depicted in SEQ ID NO: 252, CDR-H3 as depicted in SEQ ID NO: 253, CDR-L1 as depicted in SEQ ID NO: 254, CDR-L2 as depicted in SEQ ID NO: 255, and CDR-L3 as depicted in SEQ ID NO: 256;
(27) CDR-H1 as depicted in SEQ ID NO: 261, CDR-H2 as depicted in SEQ ID NO: 262, CDR-H3 as depicted in SEQ ID NO: 263, CDR-L1 as depicted in SEQ ID NO: 264, CDR-L2 as depicted in SEQ ID NO: 265, and CDR-L3 as depicted in SEQ ID NO: 266;
(28) CDR-H1 as depicted in SEQ ID NO: 271, CDR-H2 as depicted in SEQ ID NO: 272, CDR-H3 as depicted in SEQ ID NO: 273, CDR-L1 as depicted in SEQ ID NO: 274, CDR-L2 as depicted in SEQ ID NO: 275, and CDR-L3 as depicted in SEQ ID NO: 276;
(29) CDR-H1 as depicted in SEQ ID NO: 281, CDR-H2 as depicted in SEQ ID NO: 282, CDR-H3 as depicted in SEQ ID NO: 283, CDR-L1 as depicted in SEQ ID NO: 284, CDR-L2 as depicted in SEQ ID NO: 285, and CDR-L3 as depicted in SEQ ID NO: 286;
(30) CDR-H1 as depicted in SEQ ID NO: 291, CDR-H2 as depicted in SEQ ID NO: 292, CDR-H3 as depicted in SEQ ID NO: 293, CDR-L1 as depicted in SEQ ID NO: 294, CDR-L2 as depicted in SEQ ID NO: 295, and CDR-L3 as depicted in SEQ ID NO: 296;
(31) CDR-H1 as depicted in SEQ ID NO: 301, CDR-H2 as depicted in SEQ ID NO: 302, CDR-H3 as depicted in SEQ ID NO: 303, CDR-L1 as depicted in SEQ ID NO: 304, CDR-L2 as depicted in SEQ ID NO: 305, and CDR-L3 as depicted in SEQ ID NO: 306;
(32) CDR-H1 as depicted in SEQ ID NO: 311, CDR-H2 as depicted in SEQ ID NO: 312, CDR-H3 as depicted in SEQ ID NO: 313, CDR-L1 as depicted in SEQ ID NO: 314, CDR-L2 as depicted in SEQ ID NO: 315, and CDR-L3 as depicted in SEQ ID NO: 316;
(33) CDR-H1 as depicted in SEQ ID NO: 321, CDR-H2 as depicted in SEQ ID NO: 322, CDR-H3 as depicted in SEQ ID NO: 323, CDR-L1 as depicted in SEQ ID NO: 324, CDR-L2 as depicted in SEQ ID NO: 325, and CDR-L3 as depicted in SEQ ID NO: 326;
(34) CDR-H1 as depicted in SEQ ID NO: 331, CDR-H2 as depicted in SEQ ID NO: 332, CDR-H3 as depicted in SEQ ID NO: 333, CDR-L1 as depicted in SEQ ID NO: 334, CDR-L2 as depicted in SEQ ID NO: 335, and CDR-L3 as depicted in SEQ ID NO: 336;
(35) CDR-H1 as depicted in SEQ ID NO: 341, CDR-H2 as depicted in SEQ ID NO: 342, CDR-H3 as depicted in SEQ ID NO: 343, CDR-L1 as depicted in SEQ ID NO: 344, CDR-L2 as depicted in SEQ ID NO: 345, and CDR-L3 as depicted in SEQ ID NO: 346;
(36) CDR-H1 as depicted in SEQ ID NO: 351, CDR-H2 as depicted in SEQ ID NO: 352, CDR-H3 as depicted in SEQ ID NO: 353, CDR-L1 as depicted in SEQ ID NO: 354, CDR-L2 as depicted in SEQ ID NO: 355, and CDR-L3 as depicted in SEQ ID NO: 356;
(37) CDR-H1 as depicted in SEQ ID NO: 361, CDR-H2 as depicted in SEQ ID NO: 362, CDR-H3 as depicted in SEQ ID NO: 363, CDR-L1 as depicted in SEQ ID NO: 364, CDR-L2 as depicted in SEQ ID NO: 365, and CDR-L3 as depicted in SEQ ID NO: 366;
(38) CDR-H1 as depicted in SEQ ID NO: 371, CDR-H2 as depicted in SEQ ID NO: 372, CDR-H3 as depicted in SEQ ID NO: 373, CDR-L1 as depicted in SEQ ID NO: 374, CDR-L2 as depicted in SEQ ID NO: 375, and CDR-L3 as depicted in SEQ ID NO: 376;
(39) CDR-H1 as depicted in SEQ ID NO: 381, CDR-H2 as depicted in SEQ ID NO: 382, CDR-H3 as depicted in SEQ ID NO: 383, CDR-L1 as depicted in SEQ ID NO: 384, CDR-L2 as depicted in SEQ ID NO: 385, and CDR-L3 as depicted in SEQ ID NO: 386;
(40) CDR-H1 as depicted in SEQ ID NO: 391, CDR-H2 as depicted in SEQ ID NO: 392, CDR-H3 as depicted in SEQ ID NO: 393, CDR-L1 as depicted in SEQ ID NO: 394, CDR-L2 as depicted in SEQ ID NO: 395, and CDR-L3 as depicted in SEQ ID NO: 396;
(41) CDR-H1 as depicted in SEQ ID NO: 401, CDR-H2 as depicted in SEQ ID NO: 402, CDR-H3 as depicted in SEQ ID NO: 403, CDR-L1 as depicted in SEQ ID NO: 404, CDR-L2 as depicted in SEQ ID NO: 405, and CDR-L3 as depicted in SEQ ID NO: 406;
(42) CDR-H1 as depicted in SEQ ID NO: 411, CDR-H2 as depicted in SEQ ID NO: 412, CDR-H3 as depicted in SEQ ID NO: 413, CDR-L1 as depicted in SEQ ID NO: 414, CDR-L2 as depicted in SEQ ID NO: 415, and CDR-L3 as depicted in SEQ ID NO: 416;
(43) CDR-H1 as depicted in SEQ ID NO: 421, CDR-H2 as depicted in SEQ ID NO: 422, CDR-H3 as depicted in SEQ ID NO: 423, CDR-L1 as depicted in SEQ ID NO: 424, CDR-L2 as depicted in SEQ ID NO: 425, and CDR-L3 as depicted in SEQ ID NO: 426;
(44) CDR-H1 as depicted in SEQ ID NO: 431, CDR-H2 as depicted in SEQ ID NO: 432, CDR-H3 as depicted in SEQ ID NO: 433, CDR-L1 as depicted in SEQ ID NO: 434, CDR-L2 as depicted in SEQ ID NO: 435, and CDR-L3 as depicted in SEQ ID NO: 436;
(45) CDR-H1 as depicted in SEQ ID NO: 441, CDR-H2 as depicted in SEQ ID NO: 442, CDR-H3 as depicted in SEQ ID NO: 443, CDR-L1 as depicted in SEQ ID NO: 444, CDR-L2 as depicted in SEQ ID NO: 445, and CDR-L3 as depicted in SEQ ID NO: 446;
(46) CDR-H1 as depicted in SEQ ID NO: 451, CDR-H2 as depicted in SEQ ID NO: 452, CDR-H3 as depicted in SEQ ID NO: 453, CDR-L1 as depicted in SEQ ID NO: 454, CDR-L2 as depicted in SEQ ID NO: 455, and CDR-L3 as depicted in SEQ ID NO: 456;
(47) CDR-H1 as depicted in SEQ ID NO: 461, CDR-H2 as depicted in SEQ ID NO: 462, CDR-H3 as depicted in SEQ ID NO: 463, CDR-L1 as depicted in SEQ ID NO: 464, CDR-L2 as depicted in SEQ ID NO: 465, and CDR-L3 as depicted in SEQ ID NO: 466;
(48) CDR-H1 as depicted in SEQ ID NO: 471, CDR-H2 as depicted in SEQ ID NO: 472, CDR-H3 as depicted in SEQ ID NO: 473, CDR-L1 as depicted in SEQ ID NO: 474, CDR-L2 as depicted in SEQ ID NO: 475, and CDR-L3 as depicted in SEQ ID NO: 476;
(49) CDR-H1 as depicted in SEQ ID NO: 481, CDR-H2 as depicted in SEQ ID NO: 482, CDR-H3 as depicted in SEQ ID NO: 483, CDR-L1 as depicted in SEQ ID NO: 484, CDR-L2 as depicted in SEQ ID NO: 485, and CDR-L3 as depicted in SEQ ID NO: 486;
(50) CDR-H1 as depicted in SEQ ID NO: 491, CDR-H2 as depicted in SEQ ID NO: 492, CDR-H3 as depicted in SEQ ID NO: 493, CDR-L1 as depicted in SEQ ID NO: 494, CDR-L2 as depicted in SEQ ID NO: 495, and CDR-L3 as depicted in SEQ ID NO: 496;
(51) CDR-H1 as depicted in SEQ ID NO: 501, CDR-H2 as depicted in SEQ ID NO: 502, CDR-H3 as depicted in SEQ ID NO: 503, CDR-L1 as depicted in SEQ ID NO: 504, CDR-L2 as depicted in SEQ ID NO: 505, and CDR-L3 as depicted in SEQ ID NO: 506;
(52) CDR-H1 as depicted in SEQ ID NO: 511, CDR-H2 as depicted in SEQ ID NO: 512, CDR-H3 as depicted in SEQ ID NO: 513, CDR-L1 as depicted in SEQ ID NO: 514, CDR-L2 as depicted in SEQ ID NO: 515, and CDR-L3 as depicted in SEQ ID NO: 516; and
(53) CDR-H1 as depicted in SEQ ID NO: 521, CDR-H2 as depicted in SEQ ID NO: 522, CDR-H3 as depicted in SEQ ID NO: 523, CDR-L1 as depicted in SEQ ID NO: 524, CDR-L2 as depicted in SEQ ID NO: 525, and CDR-L3 as depicted in SEQ ID NO: 526.

7. The antibody construct for use according to any one of the preceding claims,
(I) wherein the first domain which binds to BCMA comprises a VH region having an amino acid sequence selected from the group consisting of those depicted in SEQ ID NOs: 7, 17, 27, 37, 47, 57, 67, 77, 87, 97, 107, 117, 127, 137, 147, 157, 167, 177, 187, 197, 207, 217, 227, 237, 247, 257, 267, 277, 287, 307, 317, 327, 337, 347, 357, 367, 377, 387, 397, 407, 417, 427, 437, 447, 457, 467, 477, 487, 497, 507, 517, and 527; and/or
(II) The antibody construct for use according to any one of the preceding claims, wherein the first domain which binds to BCMA comprises a VL region having an amino acid sequence selected from the group consisting of those depicted in SEQ ID NOs: 8, 18, 28, 38, 48, 58, 68, 78, 88, 98, 108, 118, 128, 138, 148, 158, 168, 178, 188, 198, 208, 218, 228, 238, 248, 258, 268, 278, 288, 298, 308, 318, 328, 338, 348, 358, 368, 378, 388, 398, 408, 418, 428, 438, 448, 458, 468, 478, 488, 498, 508, 518, and 528.

8. The antibody construct for use according to any one of the preceding claims, wherein the first domain which binds to BCMA comprises a VH region and a VL region selected from the group consisting of:
(1) a VH region as depicted in SEQ ID NO: 7 and a VL region as depicted in SEQ ID NO: 8;
(2) a VH region as depicted in SEQ ID NO: 17 and a VL region as depicted in SEQ ID NO: 18;
(3) a VH region as depicted in SEQ ID NO: 27 and a VL region as depicted in SEQ ID NO: 28;
(4) a VH region as depicted in SEQ ID NO: 37 and a VL region as depicted in SEQ ID NO: 38;
(5) a VH region as depicted in SEQ ID NO: 47 and a VL region as depicted in SEQ ID NO: 48;
(6) a VH region as depicted in SEQ ID NO: 57 and a VL region as depicted in SEQ ID NO: 58;
(7) a VH region as depicted in SEQ ID NO: 67 and a VL region as depicted in SEQ ID NO: 68;
(8) a VH region as depicted in SEQ ID NO: 77 and a VL region as depicted in SEQ ID NO: 78;
(9) a VH region as depicted in SEQ ID NO: 87 and a VL region as depicted in SEQ ID NO: 88;
(10) a VH region as depicted in SEQ ID NO: 97 and a VL region as depicted in SEQ ID NO: 98;
(11) a VH region as depicted in SEQ ID NO: 107 and a VL region as depicted in SEQ ID NO: 108;
(12) a VH region as depicted in SEQ ID NO: 117 and a VL region as depicted in SEQ ID NO: 118;
(13) a VH region as depicted in SEQ ID NO: 127 and a VL region as depicted in SEQ ID NO: 128;
(14) a VH region as depicted in SEQ ID NO: 137 and a VL region as depicted in SEQ ID NO: 138;
(15) a VH region as depicted in SEQ ID NO: 147 and a VL region as depicted in SEQ ID NO: 148;
(16) a VH region as depicted in SEQ ID NO: 157 and a VL region as depicted in SEQ ID NO: 158;
(17) a VH region as depicted in SEQ ID NO: 167 and a VL region as depicted in SEQ ID NO: 168;
(18) a VH region as depicted in SEQ ID NO: 177 and a VL region as depicted in SEQ ID NO: 178;
(19) a VH region as depicted in SEQ ID NO: 187 and a VL region as depicted in SEQ ID NO: 188;
(20) a VH region as depicted in SEQ ID NO: 197 and a VL region as depicted in SEQ ID NO: 198;
(21) a VH region as depicted in SEQ ID NO: 207 and a VL region as depicted in SEQ ID NO: 208;
(22) a VH region as depicted in SEQ ID NO: 217 and a VL region as depicted in SEQ ID NO: 218;
(23) a VH region as depicted in SEQ ID NO: 227 and a VL region as depicted in SEQ ID NO: 228;
(24) a VH region as depicted in SEQ ID NO: 237 and a VL region as depicted in SEQ ID NO: 238;
(25) a VH region as depicted in SEQ ID NO: 247 and a VL region as depicted in SEQ ID NO: 248;
(26) a VH region as depicted in SEQ ID NO: 257 and a VL region as depicted in SEQ ID NO: 258;
(27) a VH region as depicted in SEQ ID NO: 267 and a VL region as depicted in SEQ ID NO: 268;
(28) a VH region as depicted in SEQ ID NO: 277 and a VL region as depicted in SEQ ID NO: 278;
(29) a VH region as depicted in SEQ ID NO: 287 and a VL region as depicted in SEQ ID NO: 288;
(30) a VH region as depicted in SEQ ID NO: 297 and a VL region as depicted in SEQ ID NO: 298;
(31) a VH region as depicted in SEQ ID NO: 307 and a VL region as depicted in SEQ ID NO: 308;
(32) a VH region as depicted in SEQ ID NO: 317 and a VL region as depicted in SEQ ID NO: 318;
(33) a VH region as depicted in SEQ ID NO: 327 and a VL region as depicted in SEQ ID NO: 328;
(34) a VH region as depicted in SEQ ID NO: 337 and a VL region as depicted in SEQ ID NO: 338;
(35) a VH region as depicted in SEQ ID NO: 347 and a VL region as depicted in SEQ ID NO: 348;
(36) a VH region as depicted in SEQ ID NO: 357 and a VL region as depicted in SEQ ID NO: 358;
(37) a VH region as depicted in SEQ ID NO: 367 and a VL region as depicted in SEQ ID NO: 368;
(38) a VH region as depicted in SEQ ID NO: 377 and a VL region as depicted in SEQ ID NO: 378;
(39) a VH region as depicted in SEQ ID NO: 387 and a VL region as depicted in SEQ ID NO: 388;
(40) a VH region as depicted in SEQ ID NO: 397 and a VL region as depicted in SEQ ID NO: 398;
(41) a VH region as depicted in SEQ ID NO: 407 and a VL region as depicted in SEQ ID NO: 408;
(42) a VH region as depicted in SEQ ID NO: 417 and a VL region as depicted in SEQ ID NO: 418;
(43) a VH region as depicted in SEQ ID NO: 427 and a VL region as depicted in SEQ ID NO: 428;
(44) a VH region as depicted in SEQ ID NO: 437 and a VL region as depicted in SEQ ID NO: 438;
(45) a VH region as depicted in SEQ ID NO: 447 and a VL region as depicted in SEQ ID NO: 448;
(46) a VH region as depicted in SEQ ID NO: 457 and a VL region as depicted in SEQ ID NO: 458;
(47) a VH region as depicted in SEQ ID NO: 467 and a VL region as depicted in SEQ ID NO: 468;
(48) a VH region as depicted in SEQ ID NO: 477 and a VL region as depicted in SEQ ID NO: 478;
(49) a VH region as depicted in SEQ ID NO: 487 and a VL region as depicted in SEQ ID NO: 488;
(50) a VH region as depicted in SEQ ID NO: 497 and a VL region as depicted in SEQ ID NO: 498;
(51) a VH region as depicted in SEQ ID NO: 507 and a VL region as depicted in SEQ ID NO: 508;
(52) a VH region as depicted in SEQ ID NO: 517 and a VL region as depicted in SEQ ID NO: 518; and
(53) a VH region as depicted in SEQ ID NO: 527 and a VL region as depicted in SEQ ID NO: 528.

9. The antibody construct for use according to any one of the preceding claims,
(I) wherein the first domain which binds to BCMA comprises or consists of a polypeptide having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529;
(II) wherein the second domain which binds to CD3 comprises a VL region comprising CDR-L1, CDR-L2 and CDR-L3 selected from the group consisting of:
(a) CDR-L1 as depicted in SEQ ID NO: 542, CDR-L2 as depicted in SEQ ID NO: 543, and CDR-L3 as depicted in SEQ ID NO: 544;
(b) CDR-L1 as depicted in SEQ ID NO: 599, CDR-L2 as depicted in SEQ ID NO: 600, and CDR-L3 as depicted in SEQ ID NO: 601; and
(c) CDR-L1 as depicted in SEQ ID NO: 621, CDR-L2 as depicted in SEQ ID NO: 622, and CDR-L3 as depicted in SEQ ID NO: 623;
(III) wherein the second domain which binds to CD3 comprises a VH region comprising CDR-H1, CDR-H2 and CDR-H3 selected from the group consisting of:
(a) CDR-H1 as depicted in SEQ ID NO: 534, CDR-H2 as depicted in SEQ ID NO: 535, and CDR-H3 as depicted in SEQ ID NO: 536;
(b) CDR-H1 as depicted in SEQ ID NO: 545, CDR-H2 as depicted in SEQ ID NO: 546, and CDR-H3 as depicted in SEQ ID NO: 547;
(c) CDR-H1 as depicted in SEQ ID NO: 557, CDR-H2 as depicted in SEQ ID NO: 558, and CDR-H3 as depicted in SEQ ID NO: 559;
(d) CDR-H1 as depicted in SEQ ID NO: 568, CDR-H2 as depicted in SEQ ID NO: 569, and CDR-H3 as depicted in SEQ ID NO: 570;
(e) CDR-H1 as depicted in SEQ ID NO: 579, CDR-H2 as depicted in SEQ ID NO: 580, and CDR-H3 as depicted in SEQ ID NO: 581;
(f) CDR-H1 as depicted in SEQ ID NO: 591, CDR-H2 as depicted in SEQ ID NO: 592, and CDR-H3 as depicted in SEQ ID NO: 593;
(g) CDR-H1 as depicted in SEQ ID NO: 602, CDR-H2 as depicted in SEQ ID NO: 603, and CDR-H3 as depicted in SEQ ID NO: 604;
(h) CDR-H1 as depicted in SEQ ID NO: 613, CDR-H2 as depicted in SEQ ID NO: 614, and CDR-H3 as depicted in SEQ ID NO: 615;
(i) CDR-H1 as depicted in SEQ ID NO: 624, CDR-H2 as depicted in SEQ ID NO: 625, and CDR-H3 as depicted in SEQ ID NO: 626; and
(j) CDR-H1 as depicted in SEQ ID NO: 636, CDR-H2 as depicted in SEQ ID NO: 637, and CDR-H3 as depicted in SEQ ID NO: 638; and/or
(IV) wherein the second domain which binds to CD3 comprises a VL region comprising CDR-L1, CDR-L2 and CDR-L3 and a VH region comprising CDR-H1, CDR-H2 and CDR-H3 selected from the group consisting of
(a) CDR-L1 as depicted in SEQ ID NO: 531, CDR-L2 as depicted in SEQ ID NO: 532, CDR-L3 as depicted in SEQ ID NO: 533, CDR-H1 as depicted in SEQ ID NO: 534, CDR-H2 as depicted in SEQ ID NO: 535, and CDR-H3 as depicted in SEQ ID NO: 536;
(b) CDR-L1 as depicted in SEQ ID NO: 542, CDR-L2 as depicted in SEQ ID NO: 543, CDR-L3 as depicted in SEQ ID NO: 544, CDR-H1 as depicted in SEQ ID NO: 545, CDR-H2 as depicted in SEQ ID NO: 546, and CDR-H3 as depicted in SEQ ID NO: 547;
(c) CDR-L1 as depicted in SEQ ID NO: 554, CDR-L2 as depicted in SEQ ID NO: 555, CDR-L3 as depicted in SEQ ID NO: 556, CDR-H1 as depicted in SEQ ID NO: 557, CDR-H2 as depicted in SEQ ID NO: 558, and CDR-H3 as depicted in SEQ ID NO: 559;
(d) CDR-L1 as depicted in SEQ ID NO: 565, CDR-L2 as depicted in SEQ ID NO: 566, CDR-L3 as depicted in SEQ ID NO: 567, CDR-H1 as depicted in SEQ ID NO: 568, CDR-H2 as depicted in SEQ ID NO: 569, and CDR-H3 as depicted in SEQ ID NO: 570;
(e) CDR-L1 as depicted in SEQ ID NO: 576, CDR-L2 as depicted in SEQ ID NO: 577, CDR-L3 as depicted in SEQ ID NO: 578, CDR-H1 as depicted in SEQ ID NO: 579, CDR-H2 as depicted in SEQ ID NO: 580, and CDR-H3 as depicted in SEQ ID NO: 581;
(f) CDR-L1 as depicted in SEQ ID NO: 588, CDR-L2 as depicted in SEQ ID NO: 589, CDR-L3 as depicted in SEQ ID NO: 590, CDR-H1 as depicted in SEQ ID NO: 591, CDR-H2 as depicted in SEQ ID NO: 592, and CDR-H3 as depicted in SEQ ID NO: 593;
(g) CDR-L1 as depicted in SEQ ID NO: 599, CDR-L2 as depicted in SEQ ID NO: 600, CDR-L3 as depicted in SEQ ID NO: 601, CDR-H1 as depicted in SEQ ID NO: 602, CDR-H2 as depicted in SEQ ID NO: 603, and CDR-H3 as depicted in SEQ ID NO: 604;
(h) CDR-L1 as depicted in SEQ ID NO: 610, CDR-L2 as depicted in SEQ ID NO: 611, CDR-L3 as depicted in SEQ ID NO: 612, CDR-H1 as depicted in SEQ ID NO: 613, CDR-H2 as depicted in SEQ ID NO: 614, and CDR-H3 as depicted in SEQ ID NO: 615;
(i) CDR-L1 as depicted in SEQ ID NO: 621, CDR-L2 as depicted in SEQ ID NO: 622, CDR-L3 as depicted in SEQ ID NO: 623, CDR-H1 as depicted in SEQ ID NO: 624, CDR-H2 as depicted in SEQ ID NO: 625, and CDR-H3 as depicted in SEQ ID NO: 626; and
(j) CDR-L1 as depicted in SEQ ID NO: 633, CDR-L2 as depicted in SEQ ID NO: 634, CDR-L3 as depicted in SEQ ID NO: 635, CDR-H1 as depicted in SEQ ID NO: 636, CDR-H2 as depicted in SEQ ID NO: 637, and CDR-H3 as depicted in SEQ ID NO: 638.

10. The antibody construct for use according to any one of the preceding claims,
(I) wherein the second domain which binds to CD3 comprises a VL region having an amino acid sequence selected from the group consisting of those depicted in SEQ ID NO: 550, SEQ ID NO: 551, SEQ ID NO: 584, SEQ ID NO: 585, SEQ ID NO: 629 and SEQ ID NO: 630; and/or
(II) wherein the second domain which binds to CD3 comprises a VH region having an amino acid sequence selected from the group consisting of those depicted in SEQ ID NO: 537, SEQ ID NO: 538, SEQ ID NO: 548, SEQ ID NO: 549, SEQ ID NO: 560, SEQ ID NO: 561, SEQ ID NO: 571, SEQ ID NO: 572, SEQ ID NO: 582, SEQ ID NO: 583, SEQ ID NO: 594, SEQ ID NO: 595, SEQ ID NO: 605, SEQ ID NO: 606, SEQ ID NO: 616, SEQ ID NO: 617, SEQ ID NO: 627, SEQ ID NO: 628, SEQ ID NO: 639, SEQ ID NO: 640, and SEQ ID NO: 644.

11. The antibody construct for use according to any one of the preceding claims, wherein the second domain which binds to CD3 comprises a VL region and a VH region selected from the group consisting of:
(a) a VL region as depicted in SEQ ID NO: 539 or 521 and a VH region as depicted in SEQ ID NO: 537 or 538;
(b) a VL region as depicted in SEQ ID NO: 550 or 521 and a VH region as depicted in SEQ ID NO: 548 or 549;
(c) a VL region as depicted in SEQ ID NO: 562 or 521 and a VH region as depicted in SEQ ID NO: 560 or 561;
(d) a VL region as depicted in SEQ ID NO: 573 or 521 and a VH region as depicted in SEQ ID NO: 571 or 572;
(e) a VL region as depicted in SEQ ID NO: 584 or 585 and a VH region as depicted in SEQ ID NO: 582 or 583;
(f) a VL region as depicted in SEQ ID NO: 596 or 521 and a VH region as depicted in SEQ ID NO: 594 or 595;
(g) a VL region as depicted in SEQ ID NO: 607 or 585 and a VH region as depicted in SEQ ID NO: 605 or 606;
(h) a VL region as depicted in SEQ ID NO: 618 or 521 and a VH region as depicted in SEQ ID NO: 616 or 617;
(i) a VL region as depicted in SEQ ID NO: 629 or 630 and a VH region as depicted in SEQ ID NO: 627 or 628;
(j) a VL region as depicted in SEQ ID NO: 641 or 630 and a VH region as depicted in SEQ ID NO: 639 or 640; and
(k) a VL region as depicted in SEQ ID NO: 645 and a VH region as depicted in SEQ ID NO: 644.

12. The antibody construct for use according to any one of the preceding claims,
(I) wherein the second domain which binds to CD3 comprises or consists of a polypeptide having an amino acid sequence selected from the group consisting of those depicted in SEQ ID NOs: 540, 541, 552, 553, 563, 564, 574, 575, 586, 587, 597, 598, 608, 609, 619, 620, 631, 632, 642, 643, and 646; and/or
(II) which comprises a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530.

13. The antibody construct for use according to any one of the preceding claims, comprising in an N- to C-terminal order:
(a) the first domain;
(b) a peptide linker having an amino acid sequence selected from the group consisting of SEQ ID NOs: 687, 693 and 694;
(c) the second domain;
(d) a peptide linker having an amino acid sequence selected from the group consisting of SEQ ID NO: 686, 687, 688, 689, 690, 691, 692, 693 or 694;
(e) a first polypeptide monomer (comprising a hinge, a CH2 domain and a CH3 domain);
(f) a peptide linker having an amino acid sequence selected from the group consisting of SEQ ID NOs: 695, 696, 697, 698 or 699; and
(g) a second polypeptide monomer (comprising a hinge, a CH2 domain and a CH3 domain).

14. The antibody construct for use according to any one of the preceding claims, comprising in an N- to C-terminal order:
(a) the first domain having an amino acid sequence selected from the group consisting of SEQ ID NOs: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, and 529; wherein the peptide linker comprised within those sequences and having SEQ ID NO: 694 can be replaced by any one of SEQ ID NOs: 686-693 and 695-699;
(b) a peptide linker having an amino acid sequence selected from the group consisting of SEQ ID NOs: 687, 693 and 694;
(c) the second domain having an amino acid sequence selected from the group consisting of SEQ ID NOs: 540, 541, 552, 553, 563, 564, 574, 575, 586, 587, 597, 598, 608, 609, 619, 620, 631, 632, 642, 643, and 646; wherein the peptide linker comprised within those sequences and having SEQ ID NO: 694 can be replaced by any one of SEQ ID NOs: 686-693 and 695-699;
(d) a peptide linker having an amino acid sequence selected from the group consisting of SEQ ID NOs: 686, 687, 688, 689, 690, 691, 692, 693, and 694; and
(e) the third domain having an amino acid sequence selected from the group consisting of SEQ ID NOs: 700-707.

15. The antibody construct for use according to any one of the preceding claims,
(I) comprising in an N- to C-terminal order:
(a) a polypeptide having an amino acid sequence selected from the group consisting of those depicted in SEQ ID NOs: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, and 530;
(b) a linker having an amino acid sequence as depicted in SEQ ID NO: 686; and
(c) a polypeptide having an amino acid sequence as depicted in SEQ ID NO: 700;
(II) which has a molecular weight of about 75 to about 200 kDa, about 80 to about 175 kDa, about 85 to about 150 kDa, about 90 to about 130 kDa, about 95 to about 120 kDa, and preferably about 100 to about 115 kDa or about 105 to about 110 kDa; and/or
(III) which has an elimination half-life (T_{1/2}) of about 3 to about 14 days, about 4 to about 12 days, about 3 or 4 to about 10 days, about 3 or 4 to about 8 days, or about 5 to about 7 days.

## Patentansprüche

1. Antikörperkonstrukt, umfassend eine erste Domäne, die an BCMA bindet, eine zweite Domäne, die an CD3 bindet, und eine dritte Domäne, die die Plasmahalbwertszeit des Antikörperkonstrukts verlängert, zur Verwendung bei der Behandlung oder Linderung einer BCMA-positiven Neoplasie bei einem Menschen, wobei es sich bei der Neoplasie um eine B-Zell-Neoplasie oder eine Plasmazell-Neoplasie handelt, wobei das Antikörperkonstrukt in einer Minimaldosis von 800 µg/Tag in wenigstens einem Zyklus verabreicht wird, wobei ein Zyklus wenigstens drei einzelne Verabreichungen des Antikörperkonstrukts umfasst, wobei es sich bei der einzelnen Verabreichung um die Verabreichung an einem Tag handelt.

2. Antikörperkonstrukt zur Verwendung nach Anspruch 1,
(I) das über 2, 3, 4, 5, 6, 7, 8, 9, 10, 11, 12, 13, 14, 15, 16, 17, 18, 19, 20, 21, 22, 23, 24, 25 oder mehr Zyklen verabreicht wird;
(II) wobei ein Zyklus etwa 25 bis etwa 30 Tage, bevorzugt etwa 26 oder 27 bis etwa 29 Tage und bevorzugter etwa 28 Tage aufweist;
(III) wobei das Antikörperkonstrukt in einer Dosis von 800 µg/Tag bis 12 mg pro Tag, wie 1000 µg, 1200 µg, 1500 µg, 1600 µg, 2000 µg, 2500 µg, 3000 µg, 3500 µg, 4000 µg, 4500 µg, 5000 µg, 5500 µg, 6000 µg, 6500 µg, 7000 µg, 7500 µg, 8000 µg, 8500 µg, 9000 µg, 9500 µg, 10 mg, 11 mg oder 12 mg pro Tag, verabreicht wird;
(IV) wobei das Antikörperkonstrukt beim ersten Zyklus in einem Dosisschritt oder in zwei Dosisschritten verabreicht wird, wobei eine erste Dosis bevorzugt zwischen 800 µg/Tag und 1200 µg/Tag liegt, eine optionale zweite Dosis zwischen 2500 µg/Tag und 5000 µg/Tag, bevorzugt bei etwa 3000 µg/Tag oder etwa 4500 µg/Tag liegt und eine letzte Dosis (Zieldosis) zwischen 6500 µg/Tag und 12 mg/Tag, wie bei 6500 µg/Tag, 7000 µg/Tag, 7500 µg/Tag, 8000 µg/Tag, 8500 µg/Tag, 9000 µg/Tag, 9500 µg/Tag, 10 mg/Tag, 11 mg/Tag oder 12 mg/Tag, liegt; und/oder
(V) wobei der erste Zyklus drei bis sechs einzelne Verabreichungen, bevorzugt vier oder fünf einzelne Verabreichungen des Antikörperkonstrukts umfasst bzw. daraus besteht.

3. Antikörperkonstrukt zur Verwendung nach Anspruch 2, wobei das Antikörperkonstrukt beim zweiten Zyklus und gegebenenfalls bei allen nachfolgenden Zyklen jeweils in konstanten Dosen verabreicht wird.

4. Antikörperkonstrukt zur Verwendung nach einem der vorhergehenden Ansprüche,
(I) das parenteral, bevorzugt intravenös und bevorzugter über intravenöse Bolusinjektion, Bolusinfusion oder kurzzeitige intravenöse Infusion verabreicht wird;
(II) wobei die BCMA-positive Neoplasie aus der Gruppe bestehend aus multiplem Myelom, rezidiviertem und/oder refraktärem multiplem Myelom, multiplem Myelom der schweren Kette, multiplem Myelom der leichten Kette, extramedullärem Myelom, Plasmazytom, Plasmazell-Leukämie, Morbus Waldenström und schwelendem Myelom ausgewählt ist; und/oder
(III) wobei
a) das Antikörperkonstrukt ein Einzelkette-Polypeptid ist,
b) die erste Domäne im Format eines scFv vorliegt,
c) die zweite Domäne im Format eines scFv vorliegt und/oder
d) die erste Domäne und die zweite Domäne über einen Linker, bevorzugt einen Peptidlinker, bevorzugter einen Glycin/Serin-Linker verbunden sind.

5. Antikörperkonstrukt zur Verwendung nach einem der vorhergehenden Ansprüche,
(I) das um die Bindung an BCMA mit Folgendem konkurriert oder das an das gleiche Epitop von BCMA wie Folgendes bindet:
a) ein Antikörper oder Antikörperkonstrukt, der bzw. das eine Domäne umfasst, die an BCMA auf der Oberfläche einer Zielzelle bindet, wobei die Domäne eine VH-Region, die CDR-H1 gemäß SEQ ID NO: 171, CDR-H2 gemäß SEQ ID NO: 172 und CDR-H3 gemäß SEQ ID NO: 173 umfasst, und eine VL-Region, die CDR-L1 gemäß SEQ ID NO: 174, CDR-L2 gemäß SEQ ID NO: 175 und CDR-L3 gemäß SEQ ID NO: 176 umfasst, umfasst;
b) ein Antikörper oder Antikörperkonstrukt, der bzw. das eine Domäne umfasst, die an BCMA auf der Oberfläche einer Zielzelle bindet, wobei die Domäne eine VH-Region gemäß SEQ ID NO: 177 und eine VL-Region gemäß SEQ ID NO: 178 umfasst;
c) ein Antikörperkonstrukt, das eine Domäne umfasst, die an BCMA auf der Oberfläche einer Zielzelle bindet, wobei die Domäne die Aminosäuresequenz gemäß SEQ ID NO: 179 umfasst; oder
d) ein Antikörperkonstrukt mit der Aminosäuresequenz gemäß SEQ ID NO: 661; und/oder
(II) das um die Bindung an CD3 mit Folgendem konkurriert oder das an das gleiche Epitop von CD3 wie Folgendes bindet:
a) ein Antikörper oder Antikörperkonstrukt, der bzw. das eine Domäne umfasst, die an CD3 auf der Oberfläche einer T-Zelle bindet, wobei die Domäne eine VH-Region, die CDR-H1 gemäß SEQ ID NO: 636, CDR-H2 gemäß SEQ ID NO: 637 und CDR-H3 gemäß SEQ ID NO: 638 umfasst, und eine VL-Region, die CDR-L1 gemäß SEQ ID NO: 633, CDR-L2 gemäß SEQ ID NO: 634, CDR-L3 gemäß SEQ ID NO: 635 umfasst, umfasst;
b) ein Antikörper oder Antikörperkonstrukt, der bzw. das eine Domäne umfasst, die an CD3 auf der Oberfläche einer T-Zelle bindet, wobei die Domäne eine VH-Region gemäß SEQ ID NO: 639 und eine VL-Region gemäß SEQ ID NO: 641 umfasst;
c) ein Antikörperkonstrukt, das eine Domäne umfasst, die an CD3 auf der Oberfläche einer T-Zelle bindet, wobei die Domäne die Aminosäuresequenz gemäß SEQ ID NO: 642 umfasst; oder
d) ein Antikörperkonstrukt mit der Aminosäuresequenz gemäß SEQ ID NO: 661.

6. Antikörperkonstrukt zur Verwendung nach einem der vorhergehenden Ansprüche, wobei die erste Domäne, die an BCMA bindet, eine CDR-H1, CDR-H2 und CDR-H3 umfassende VH-Region und eine CDR-L1, CDR-L2 und CDR-L3 umfassende VL-Region umfasst, jeweils ausgewählt aus:
(1) CDR-H1 gemäß SEQ ID NO: 1, CDR-H2 gemäß SEQ ID NO: 2, CDR-H3 gemäß SEQ ID NO: 3, CDR-L1 gemäß SEQ ID NO: 4, CDR-L2 gemäß SEQ ID NO: 5 und CDR-L3 gemäß SEQ ID NO: 6;
(2) CDR-H1 gemäß SEQ ID NO: 11, CDR-H2 gemäß SEQ ID NO: 12, CDR-H3 gemäß SEQ ID NO: 13, CDR-L1 gemäß SEQ ID NO: 14, CDR-L2 gemäß SEQ ID NO: 15 und CDR-L3 gemäß SEQ ID NO: 16;
(3) CDR-H1 gemäß SEQ ID NO: 21, CDR-H2 gemäß SEQ ID NO: 22, CDR-H3 gemäß SEQ ID NO: 23, CDR-L1 gemäß SEQ ID NO: 24, CDR-L2 gemäß SEQ ID NO: 25 und CDR-L3 gemäß SEQ ID NO: 26;
(4) CDR-H1 gemäß SEQ ID NO: 31, CDR-H2 gemäß SEQ ID NO: 32, CDR-H3 gemäß SEQ ID NO: 33, CDR-L1 gemäß SEQ ID NO: 34, CDR-L2 gemäß SEQ ID NO: 35 und CDR-L3 gemäß SEQ ID NO: 36;
(5) CDR-H1 gemäß SEQ ID NO: 41, CDR-H2 gemäß SEQ ID NO: 42, CDR-H3 gemäß SEQ ID NO: 43, CDR-L1 gemäß SEQ ID NO: 44, CDR-L2 gemäß SEQ ID NO: 45 und CDR-L3 gemäß SEQ ID NO: 46;
(6) CDR-H1 gemäß SEQ ID NO: 51, CDR-H2 gemäß SEQ ID NO: 52, CDR-H3 gemäß SEQ ID NO: 53, CDR-L1 gemäß SEQ ID NO: 54, CDR-L2 gemäß SEQ ID NO: 55 und CDR-L3 gemäß SEQ ID NO: 56;
(7) CDR-H1 gemäß SEQ ID NO: 61, CDR-H2 gemäß SEQ ID NO: 62, CDR-H3 gemäß SEQ ID NO: 63, CDR-L1 gemäß SEQ ID NO: 64, CDR-L2 gemäß SEQ ID NO: 65 und CDR-L3 gemäß SEQ ID NO: 66;
(8) CDR-H1 gemäß SEQ ID NO: 71, CDR-H2 gemäß SEQ ID NO: 72, CDR-H3 gemäß SEQ ID NO: 73, CDR-L1 gemäß SEQ ID NO: 74, CDR-L2 gemäß SEQ ID NO: 75 und CDR-L3 gemäß SEQ ID NO: 76;
(9) CDR-H1 gemäß SEQ ID NO: 81, CDR-H2 gemäß SEQ ID NO: 82, CDR-H3 gemäß SEQ ID NO: 83, CDR-L1 gemäß SEQ ID NO: 84, CDR-L2 gemäß SEQ ID NO: 85 und CDR-L3 gemäß SEQ ID NO: 86;
(10) CDR-H1 gemäß SEQ ID NO: 91, CDR-H2 gemäß SEQ ID NO: 92, CDR-H3 gemäß SEQ ID NO: 93, CDR-L1 gemäß SEQ ID NO: 94, CDR-L2 gemäß SEQ ID NO: 95 und CDR-L3 gemäß SEQ ID NO: 96;
(11) CDR-H1 gemäß SEQ ID NO: 101, CDR-H2 gemäß SEQ ID NO: 102, CDR-H3 gemäß SEQ ID NO: 103, CDR-L1 gemäß SEQ ID NO: 104, CDR-L2 gemäß SEQ ID NO: 105 und CDR-L3 gemäß SEQ ID NO: 106;
(12) CDR-H1 gemäß SEQ ID NO: 111, CDR-H2 gemäß SEQ ID NO: 112, CDR-H3 gemäß SEQ ID NO: 113, CDR-L1 gemäß SEQ ID NO: 114, CDR-L2 gemäß SEQ ID NO: 115 und CDR-L3 gemäß SEQ ID NO: 116;
(13) CDR-H1 gemäß SEQ ID NO: 121, CDR-H2 gemäß SEQ ID NO: 122, CDR-H3 gemäß SEQ ID NO: 123, CDR-L1 gemäß SEQ ID NO: 124, CDR-L2 gemäß SEQ ID NO: 125 und CDR-L3 gemäß SEQ ID NO: 126;
(14) CDR-H1 gemäß SEQ ID NO: 131, CDR-H2 gemäß SEQ ID NO: 132, CDR-H3 gemäß SEQ ID NO: 133, CDR-L1 gemäß SEQ ID NO: 134, CDR-L2 gemäß SEQ ID NO: 135 und CDR-L3 gemäß SEQ ID NO: 136;
(15) CDR-H1 gemäß SEQ ID NO: 141, CDR-H2 gemäß SEQ ID NO: 142, CDR-H3 gemäß SEQ ID NO: 143, CDR-L1 gemäß SEQ ID NO: 144, CDR-L2 gemäß SEQ ID NO: 145 und CDR-L3 gemäß SEQ ID NO: 146;
(16) CDR-H1 gemäß SEQ ID NO: 151, CDR-H2 gemäß SEQ ID NO: 152, CDR-H3 gemäß SEQ ID NO: 153, CDR-L1 gemäß SEQ ID NO: 154, CDR-L2 gemäß SEQ ID NO: 155 und CDR-L3 gemäß SEQ ID NO: 156;
(17) CDR-H1 gemäß SEQ ID NO: 161, CDR-H2 gemäß SEQ ID NO: 162, CDR-H3 gemäß SEQ ID NO: 163, CDR-L1 gemäß SEQ ID NO: 164, CDR-L2 gemäß SEQ ID NO: 165 und CDR-L3 gemäß SEQ ID NO: 166;
(18) CDR-H1 gemäß SEQ ID NO: 171, CDR-H2 gemäß SEQ ID NO: 172, CDR-H3 gemäß SEQ ID NO: 173, CDR-L1 gemäß SEQ ID NO: 174, CDR-L2 gemäß SEQ ID NO: 175 und CDR-L3 gemäß SEQ ID NO: 176;
(19) CDR-H1 gemäß SEQ ID NO: 181, CDR-H2 gemäß SEQ ID NO: 182, CDR-H3 gemäß SEQ ID NO: 183, CDR-L1 gemäß SEQ ID NO: 184, CDR-L2 gemäß SEQ ID NO: 185 und CDR-L3 gemäß SEQ ID NO: 186;
(20) CDR-H1 gemäß SEQ ID NO: 191, CDR-H2 gemäß SEQ ID NO: 192, CDR-H3 gemäß SEQ ID NO: 193, CDR-L1 gemäß SEQ ID NO: 194, CDR-L2 gemäß SEQ ID NO: 195 und CDR-L3 gemäß SEQ ID NO: 196;
(21) CDR-H1 gemäß SEQ ID NO: 201, CDR-H2 gemäß SEQ ID NO: 202, CDR-H3 gemäß SEQ ID NO: 203, CDR-L1 gemäß SEQ ID NO: 204, CDR-L2 gemäß SEQ ID NO: 205 und CDR-L3 gemäß SEQ ID NO: 206;
(22) CDR-H1 gemäß SEQ ID NO: 211, CDR-H2 gemäß SEQ ID NO: 212, CDR-H3 gemäß SEQ ID NO: 213, CDR-L1 gemäß SEQ ID NO: 214, CDR-L2 gemäß SEQ ID NO: 215 und CDR-L3 gemäß SEQ ID NO: 216;
(23) CDR-H1 gemäß SEQ ID NO: 221, CDR-H2 gemäß SEQ ID NO: 222, CDR-H3 gemäß SEQ ID NO: 223, CDR-L1 gemäß SEQ ID NO: 224, CDR-L2 gemäß SEQ ID NO: 225 und CDR-L3 gemäß SEQ ID NO: 226;
(24) CDR-H1 gemäß SEQ ID NO: 231, CDR-H2 gemäß SEQ ID NO: 232, CDR-H3 gemäß SEQ ID NO: 233, CDR-L1 gemäß SEQ ID NO: 234, CDR-L2 gemäß SEQ ID NO: 235 und CDR-L3 gemäß SEQ ID NO: 236;
(25) CDR-H1 gemäß SEQ ID NO: 241, CDR-H2 gemäß SEQ ID NO: 242, CDR-H3 gemäß SEQ ID NO: 243, CDR-L1 gemäß SEQ ID NO: 244, CDR-L2 gemäß SEQ ID NO: 245 und CDR-L3 gemäß SEQ ID NO: 246;
(26) CDR-H1 gemäß SEQ ID NO: 251, CDR-H2 gemäß SEQ ID NO: 252, CDR-H3 gemäß SEQ ID NO: 253, CDR-L1 gemäß SEQ ID NO: 254, CDR-L2 gemäß SEQ ID NO: 255 und CDR-L3 gemäß SEQ ID NO: 256;
(27) CDR-H1 gemäß SEQ ID NO: 261, CDR-H2 gemäß SEQ ID NO: 262, CDR-H3 gemäß SEQ ID NO: 263, CDR-L1 gemäß SEQ ID NO: 264, CDR-L2 gemäß SEQ ID NO: 265 und CDR-L3 gemäß SEQ ID NO: 266;
(28) CDR-H1 gemäß SEQ ID NO: 271, CDR-H2 gemäß SEQ ID NO: 272, CDR-H3 gemäß SEQ ID NO: 273, CDR-L1 gemäß SEQ ID NO: 274, CDR-L2 gemäß SEQ ID NO: 275 und CDR-L3 gemäß SEQ ID NO: 276;
(29) CDR-H1 gemäß SEQ ID NO: 281, CDR-H2 gemäß SEQ ID NO: 282, CDR-H3 gemäß SEQ ID NO: 283, CDR-L1 gemäß SEQ ID NO: 284, CDR-L2 gemäß SEQ ID NO: 285 und CDR-L3 gemäß SEQ ID NO: 286;
(30) CDR-H1 gemäß SEQ ID NO: 291, CDR-H2 gemäß SEQ ID NO: 292, CDR-H3 gemäß SEQ ID NO: 293, CDR-L1 gemäß SEQ ID NO: 294, CDR-L2 gemäß SEQ ID NO: 295 und CDR-L3 gemäß SEQ ID NO: 296;
(31) CDR-H1 gemäß SEQ ID NO: 301, CDR-H2 gemäß SEQ ID NO: 302, CDR-H3 gemäß SEQ ID NO: 303, CDR-L1 gemäß SEQ ID NO: 304, CDR-L2 gemäß SEQ ID NO: 305 und CDR-L3 gemäß SEQ ID NO: 306;
(32) CDR-H1 gemäß SEQ ID NO: 311, CDR-H2 gemäß SEQ ID NO: 312, CDR-H3 gemäß SEQ ID NO: 313, CDR-L1 gemäß SEQ ID NO: 314, CDR-L2 gemäß SEQ ID NO: 315 und CDR-L3 gemäß SEQ ID NO: 316;
(33) CDR-H1 gemäß SEQ ID NO: 321, CDR-H2 gemäß SEQ ID NO: 322, CDR-H3 gemäß SEQ ID NO: 323, CDR-L1 gemäß SEQ ID NO: 324, CDR-L2 gemäß SEQ ID NO: 325 und CDR-L3 gemäß SEQ ID NO: 326;
(34) CDR-H1 gemäß SEQ ID NO: 331, CDR-H2 gemäß SEQ ID NO: 332, CDR-H3 gemäß SEQ ID NO: 333, CDR-L1 gemäß SEQ ID NO: 334, CDR-L2 gemäß SEQ ID NO: 335 und CDR-L3 gemäß SEQ ID NO: 336;
(35) CDR-H1 gemäß SEQ ID NO: 341, CDR-H2 gemäß SEQ ID NO: 342, CDR-H3 gemäß SEQ ID NO: 343, CDR-L1 gemäß SEQ ID NO: 344, CDR-L2 gemäß SEQ ID NO: 345 und CDR-L3 gemäß SEQ ID NO: 346;
(36) CDR-H1 gemäß SEQ ID NO: 351, CDR-H2 gemäß SEQ ID NO: 352, CDR-H3 gemäß SEQ ID NO: 353, CDR-L1 gemäß SEQ ID NO: 354, CDR-L2 gemäß SEQ ID NO: 355 und CDR-L3 gemäß SEQ ID NO: 356;
(37) CDR-H1 gemäß SEQ ID NO: 361, CDR-H2 gemäß SEQ ID NO: 362, CDR-H3 gemäß SEQ ID NO: 363, CDR-L1 gemäß SEQ ID NO: 364, CDR-L2 gemäß SEQ ID NO: 365 und CDR-L3 gemäß SEQ ID NO: 366;
(38) CDR-H1 gemäß SEQ ID NO: 371, CDR-H2 gemäß SEQ ID NO: 372, CDR-H3 gemäß SEQ ID NO: 373, CDR-L1 gemäß SEQ ID NO: 374, CDR-L2 gemäß SEQ ID NO: 375 und CDR-L3 gemäß SEQ ID NO: 376;
(39) CDR-H1 gemäß SEQ ID NO: 381, CDR-H2 gemäß SEQ ID NO: 382, CDR-H3 gemäß SEQ ID NO: 383, CDR-L1 gemäß SEQ ID NO: 384, CDR-L2 gemäß SEQ ID NO: 385 und CDR-L3 gemäß SEQ ID NO: 386;
(40) CDR-H1 gemäß SEQ ID NO: 391, CDR-H2 gemäß SEQ ID NO: 392, CDR-H3 gemäß SEQ ID NO: 393, CDR-L1 gemäß SEQ ID NO: 394, CDR-L2 gemäß SEQ ID NO: 395 und CDR-L3 gemäß SEQ ID NO: 396;
(41) CDR-H1 gemäß SEQ ID NO: 401, CDR-H2 gemäß SEQ ID NO: 402, CDR-H3 gemäß SEQ ID NO: 403, CDR-L1 gemäß SEQ ID NO: 404, CDR-L2 gemäß SEQ ID NO: 405 und CDR-L3 gemäß SEQ ID NO: 406;
(42) CDR-H1 gemäß SEQ ID NO: 411, CDR-H2 gemäß SEQ ID NO: 412, CDR-H3 gemäß SEQ ID NO: 413, CDR-L1 gemäß SEQ ID NO: 414, CDR-L2 gemäß SEQ ID NO: 415 und CDR-L3 gemäß SEQ ID NO: 416;
(43) CDR-H1 gemäß SEQ ID NO: 421, CDR-H2 gemäß SEQ ID NO: 422, CDR-H3 gemäß SEQ ID NO: 423, CDR-L1 gemäß SEQ ID NO: 424, CDR-L2 gemäß SEQ ID NO: 425 und CDR-L3 gemäß SEQ ID NO: 426;
(44) CDR-H1 gemäß SEQ ID NO: 431, CDR-H2 gemäß SEQ ID NO: 432, CDR-H3 gemäß SEQ ID NO: 433, CDR-L1 gemäß SEQ ID NO: 434, CDR-L2 gemäß SEQ ID NO: 435 und CDR-L3 gemäß SEQ ID NO: 436;
(45) CDR-H1 gemäß SEQ ID NO: 441, CDR-H2 gemäß SEQ ID NO: 442, CDR-H3 gemäß SEQ ID NO: 443, CDR-L1 gemäß SEQ ID NO: 444, CDR-L2 gemäß SEQ ID NO: 445 und CDR-L3 gemäß SEQ ID NO: 446;
(46) CDR-H1 gemäß SEQ ID NO: 451, CDR-H2 gemäß SEQ ID NO: 452, CDR-H3 gemäß SEQ ID NO: 453, CDR-L1 gemäß SEQ ID NO: 454, CDR-L2 gemäß SEQ ID NO: 455 und CDR-L3 gemäß SEQ ID NO: 456;
(47) CDR-H1 gemäß SEQ ID NO: 461, CDR-H2 gemäß SEQ ID NO: 462, CDR-H3 gemäß SEQ ID NO: 463, CDR-L1 gemäß SEQ ID NO: 464, CDR-L2 gemäß SEQ ID NO: 465 und CDR-L3 gemäß SEQ ID NO: 466;
(48) CDR-H1 gemäß SEQ ID NO: 471, CDR-H2 gemäß SEQ ID NO: 472, CDR-H3 gemäß SEQ ID NO: 473, CDR-L1 gemäß SEQ ID NO: 474, CDR-L2 gemäß SEQ ID NO: 475 und CDR-L3 gemäß SEQ ID NO: 476;
(49) CDR-H1 gemäß SEQ ID NO: 481, CDR-H2 gemäß SEQ ID NO: 482, CDR-H3 gemäß SEQ ID NO: 483, CDR-L1 gemäß SEQ ID NO: 484, CDR-L2 gemäß SEQ ID NO: 485 und CDR-L3 gemäß SEQ ID NO: 486;
(50) CDR-H1 gemäß SEQ ID NO: 491, CDR-H2 gemäß SEQ ID NO: 492, CDR-H3 gemäß SEQ ID NO: 493, CDR-L1 gemäß SEQ ID NO: 494, CDR-L2 gemäß SEQ ID NO: 495 und CDR-L3 gemäß SEQ ID NO: 496;
(51) CDR-H1 gemäß SEQ ID NO: 501, CDR-H2 gemäß SEQ ID NO: 502, CDR-H3 gemäß SEQ ID NO: 503, CDR-L1 gemäß SEQ ID NO: 504, CDR-L2 gemäß SEQ ID NO: 505 und CDR-L3 gemäß SEQ ID NO: 506;
(52) CDR-H1 gemäß SEQ ID NO: 511, CDR-H2 gemäß SEQ ID NO: 512, CDR-H3 gemäß SEQ ID NO: 513, CDR-L1 gemäß SEQ ID NO: 514, CDR-L2 gemäß SEQ ID NO: 515 und CDR-L3 gemäß SEQ ID NO: 516; und
(53) CDR-H1 gemäß SEQ ID NO: 521, CDR-H2 gemäß SEQ ID NO: 522, CDR-H3 gemäß SEQ ID NO: 523, CDR-L1 gemäß SEQ ID NO: 524, CDR-L2 gemäß SEQ ID NO: 525 und CDR-L3 gemäß SEQ ID NO: 526.

7. Antikörperkonstrukt zur Verwendung nach einem der vorhergehenden Ansprüche,
(I) wobei die erste Domäne, die an BCMA bindet, eine VH-Region mit einer Aminosäuresequenz, die aus der Gruppe bestehend aus denen unter SEQ ID NO: 7, 17, 27, 37, 47, 57, 67, 77, 87, 97, 107, 117, 127, 137, 147, 157, 167, 177, 187, 197, 207, 217, 227, 237, 247, 257, 267, 277, 287, 307, 317, 327, 337, 347, 357, 367, 377, 387, 397, 407, 417, 427, 437, 447, 457, 467, 477, 487, 497, 507, 517 und 527 ausgewählt ist, umfasst; und/oder
(II) das Antikörperkonstrukt zur Verwendung nach einem der vorhergehenden Ansprüche, wobei die erste Domäne, die an BCMA bindet, eine VL-Region mit einer Aminosäuresequenz, die aus der Gruppe bestehend aus denen unter SEQ ID NO: 8, 18, 28, 38, 48, 58, 68, 78, 88, 98, 108, 118, 128, 138, 148, 158, 168, 178, 188, 198, 208, 218, 228, 238, 248, 258, 268, 278, 288, 298, 308, 318, 328, 338, 348, 358, 368, 378, 388, 398, 408, 418, 428, 438, 448, 458, 468, 478, 488, 498, 508, 518 und 528 ausgewählt ist, umfasst.

8. Antikörperkonstrukt zur Verwendung nach einem der vorhergehenden Ansprüche, wobei die erste Domäne, die an BCMA bindet, eine VH-Region und eine VL-Region umfasst, die ausgewählt sind aus der Gruppe bestehend aus:
(1) einer VH-Region gemäß SEQ ID NO: 7 und einer VL-Region gemäß SEQ ID NO: 8;
(2) einer VH-Region gemäß SEQ ID NO: 17 und einer VL-Region gemäß SEQ ID NO: 18;
(3) einer VH-Region gemäß SEQ ID NO: 27 und einer VL-Region gemäß SEQ ID NO: 28;
(4) einer VH-Region gemäß SEQ ID NO: 37 und einer VL-Region gemäß SEQ ID NO: 38;
(5) einer VH-Region gemäß SEQ ID NO: 47 und einer VL-Region gemäß SEQ ID NO: 48;
(6) einer VH-Region gemäß SEQ ID NO: 57 und einer VL-Region gemäß SEQ ID NO: 58;
(7) einer VH-Region gemäß SEQ ID NO: 67 und einer VL-Region gemäß SEQ ID NO: 68;
(8) einer VH-Region gemäß SEQ ID NO: 77 und einer VL-Region gemäß SEQ ID NO: 78;
(9) einer VH-Region gemäß SEQ ID NO: 87 und einer VL-Region gemäß SEQ ID NO: 88;
(10) einer VH-Region gemäß SEQ ID NO: 97 und einer VL-Region gemäß SEQ ID NO: 98;
(11) einer VH-Region gemäß SEQ ID NO: 107 und einer VL-Region gemäß SEQ ID NO: 108;
(12) einer VH-Region gemäß SEQ ID NO: 117 und einer VL-Region gemäß SEQ ID NO: 118;
(13) einer VH-Region gemäß SEQ ID NO: 127 und einer VL-Region gemäß SEQ ID NO: 128;
(14) einer VH-Region gemäß SEQ ID NO: 137 und einer VL-Region gemäß SEQ ID NO: 138;
(15) einer VH-Region gemäß SEQ ID NO: 147 und einer VL-Region gemäß SEQ ID NO: 148;
(16) einer VH-Region gemäß SEQ ID NO: 157 und einer VL-Region gemäß SEQ ID NO: 158;
(17) einer VH-Region gemäß SEQ ID NO: 167 und einer VL-Region gemäß SEQ ID NO: 168;
(18) einer VH-Region gemäß SEQ ID NO: 177 und einer VL-Region gemäß SEQ ID NO: 178;
(19) einer VH-Region gemäß SEQ ID NO: 187 und einer VL-Region gemäß SEQ ID NO: 188;
(20) einer VH-Region gemäß SEQ ID NO: 197 und einer VL-Region gemäß SEQ ID NO: 198;
(21) einer VH-Region gemäß SEQ ID NO: 207 und einer VL-Region gemäß SEQ ID NO: 208;
(22) einer VH-Region gemäß SEQ ID NO: 217 und einer VL-Region gemäß SEQ ID NO: 218;
(23) einer VH-Region gemäß SEQ ID NO: 227 und einer VL-Region gemäß SEQ ID NO: 228;
(24) einer VH-Region gemäß SEQ ID NO: 237 und einer VL-Region gemäß SEQ ID NO: 238;
(25) einer VH-Region gemäß SEQ ID NO: 247 und einer VL-Region gemäß SEQ ID NO: 248;
(26) einer VH-Region gemäß SEQ ID NO: 257 und einer VL-Region gemäß SEQ ID NO: 258;
(27) einer VH-Region gemäß SEQ ID NO: 267 und einer VL-Region gemäß SEQ ID NO: 268;
(28) einer VH-Region gemäß SEQ ID NO: 277 und einer VL-Region gemäß SEQ ID NO: 278;
(29) einer VH-Region gemäß SEQ ID NO: 287 und einer VL-Region gemäß SEQ ID NO: 288;
(30) einer VH-Region gemäß SEQ ID NO: 297 und einer VL-Region gemäß SEQ ID NO: 298;
(31) einer VH-Region gemäß SEQ ID NO: 307 und einer VL-Region gemäß SEQ ID NO: 308;
(32) einer VH-Region gemäß SEQ ID NO: 317 und einer VL-Region gemäß SEQ ID NO: 318;
(33) einer VH-Region gemäß SEQ ID NO: 327 und einer VL-Region gemäß SEQ ID NO: 328;
(34) einer VH-Region gemäß SEQ ID NO: 337 und einer VL-Region gemäß SEQ ID NO: 338;
(35) einer VH-Region gemäß SEQ ID NO: 347 und einer VL-Region gemäß SEQ ID NO: 348;
(36) einer VH-Region gemäß SEQ ID NO: 357 und einer VL-Region gemäß SEQ ID NO: 358;
(37) einer VH-Region gemäß SEQ ID NO: 367 und einer VL-Region gemäß SEQ ID NO: 368;
(38) einer VH-Region gemäß SEQ ID NO: 377 und einer VL-Region gemäß SEQ ID NO: 378;
(39) einer VH-Region gemäß SEQ ID NO: 387 und einer VL-Region gemäß SEQ ID NO: 388;
(40) einer VH-Region gemäß SEQ ID NO: 397 und einer VL-Region gemäß SEQ ID NO: 398;
(41) einer VH-Region gemäß SEQ ID NO: 407 und einer VL-Region gemäß SEQ ID NO: 408;
(42) einer VH-Region gemäß SEQ ID NO: 417 und einer VL-Region gemäß SEQ ID NO: 418;
(43) einer VH-Region gemäß SEQ ID NO: 427 und einer VL-Region gemäß SEQ ID NO: 428;
(44) einer VH-Region gemäß SEQ ID NO: 437 und einer VL-Region gemäß SEQ ID NO: 438;
(45) einer VH-Region gemäß SEQ ID NO: 447 und einer VL-Region gemäß SEQ ID NO: 448;
(46) einer VH-Region gemäß SEQ ID NO: 457 und einer VL-Region gemäß SEQ ID NO: 458;
(47) einer VH-Region gemäß SEQ ID NO: 467 und einer VL-Region gemäß SEQ ID NO: 468;
(48) einer VH-Region gemäß SEQ ID NO: 477 und einer VL-Region gemäß SEQ ID NO: 478;
(49) einer VH-Region gemäß SEQ ID NO: 487 und einer VL-Region gemäß SEQ ID NO: 488;
(50) einer VH-Region gemäß SEQ ID NO: 497 und einer VL-Region gemäß SEQ ID NO: 498;
(51) einer VH-Region gemäß SEQ ID NO: 507 und einer VL-Region gemäß SEQ ID NO: 508;
(52) einer VH-Region gemäß SEQ ID NO: 517 und einer VL-Region gemäß SEQ ID NO: 518; und
(53) einer VH-Region gemäß SEQ ID NO: 527 und einer VL-Region gemäß SEQ ID NO: 528.

9. Antikörperkonstrukt zur Verwendung nach einem der vorhergehenden Ansprüche,
(I) wobei die erste Domäne, die an BCMA bindet, ein Polypeptid mit einer Aminosäuresequenz, die aus der Gruppe bestehend aus SEQ ID NO: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519 und 529 ausgewählt ist, umfasst bzw. daraus besteht;
(II) wobei die zweite Domäne, die an CD3 bindet, eine CDR-L1, CDR-L2 und CDR-L3 umfassende VL-Region umfasst, jeweils ausgewählt aus der Gruppe bestehend aus:
(a) CDR-L1 gemäß SEQ ID NO: 542, CDR-L2 gemäß SEQ ID NO: 543 und CDR-L3 gemäß SEQ ID NO: 544;
(b) CDR-L1 gemäß SEQ ID NO: 599, CDR-L2 gemäß SEQ ID NO: 600 und CDR-L3 gemäß SEQ ID NO: 601; und
(c) CDR-L1 gemäß SEQ ID NO: 621, CDR-L2 gemäß SEQ ID NO: 622 und CDR-L3 gemäß SEQ ID NO: 623;
(III) wobei die zweite Domäne, die an CD3 bindet, eine CDR-H1, CDR-H2 und CDR-H3 umfassende VH-Region umfasst, jeweils ausgewählt aus der Gruppe bestehend aus:
(a) CDR-H1 gemäß SEQ ID NO: 534, CDR-H2 gemäß SEQ ID NO: 535 und CDR-H3 gemäß SEQ ID NO: 536;
(b) CDR-H1 gemäß SEQ ID NO: 545, CDR-H2 gemäß SEQ ID NO: 546 und CDR-H3 gemäß SEQ ID NO: 547;
(c) CDR-H1 gemäß SEQ ID NO: 557, CDR-H2 gemäß SEQ ID NO: 558 und CDR-H3 gemäß SEQ ID NO: 559;
(d) CDR-H1 gemäß SEQ ID NO: 568, CDR-H2 gemäß SEQ ID NO: 569 und CDR-H3 gemäß SEQ ID NO: 570;
(e) CDR-H1 gemäß SEQ ID NO: 579, CDR-H2 gemäß SEQ ID NO: 580 und CDR-H3 gemäß SEQ ID NO: 581;
(f) CDR-H1 gemäß SEQ ID NO: 591, CDR-H2 gemäß SEQ ID NO: 592 und CDR-H3 gemäß SEQ ID NO: 593;
(g) CDR-H1 gemäß SEQ ID NO: 602, CDR-H2 gemäß SEQ ID NO: 603 und CDR-H3 gemäß SEQ ID NO: 604;
(h) CDR-H1 gemäß SEQ ID NO: 613, CDR-H2 gemäß SEQ ID NO: 614 und CDR-H3 gemäß SEQ ID NO: 615;
(i) CDR-H1 gemäß SEQ ID NO: 624, CDR-H2 gemäß SEQ ID NO: 625 und CDR-H3 gemäß SEQ ID NO: 626; und
(j) CDR-H1 gemäß SEQ ID NO: 636, CDR-H2 gemäß SEQ ID NO: 637 und CDR-H3 gemäß SEQ ID NO: 638; und/oder
(IV) wobei die zweite Domäne, die an CD3 bindet, eine CDR-L1, CDR-L2 und CDR-L3 umfassende VL-Region und eine CDR-H1, CDR-H2 und CDR-H3 umfassende VH-Region umfasst, jeweils ausgewählt aus der Gruppe bestehend aus:
(a) CDR-L1 gemäß SEQ ID NO: 531, CDR-L2 gemäß SEQ ID NO: 532, CDR-L3 gemäß SEQ ID NO: 533, CDR-H1 gemäß SEQ ID NO: 534, CDR-H2 gemäß SEQ ID NO: 535 und CDR-H3 gemäß SEQ ID NO: 536;
(b) CDR-L1 gemäß SEQ ID NO: 542, CDR-L2 gemäß SEQ ID NO: 543, CDR-L3 gemäß SEQ ID NO: 544, CDR-H1 gemäß SEQ ID NO: 545, CDR-H2 gemäß SEQ ID NO: 546 und CDR-H3 gemäß SEQ ID NO: 547;
(c) CDR-L1 gemäß SEQ ID NO: 554, CDR-L2 gemäß SEQ ID NO: 555, CDR-L3 gemäß SEQ ID NO: 556, CDR-H1 gemäß SEQ ID NO: 557, CDR-H2 gemäß SEQ ID NO: 558 und CDR-H3 gemäß SEQ ID NO: 559;
(d) CDR-L1 gemäß SEQ ID NO: 565, CDR-L2 gemäß SEQ ID NO: 566, CDR-L3 gemäß SEQ ID NO: 567, CDR-H1 gemäß SEQ ID NO: 568, CDR-H2 gemäß SEQ ID NO: 569 und CDR-H3 gemäß SEQ ID NO: 570;
(e) CDR-L1 gemäß SEQ ID NO: 576, CDR-L2 gemäß SEQ ID NO: 577, CDR-L3 gemäß SEQ ID NO: 578, CDR-H1 gemäß SEQ ID NO: 579, CDR-H2 gemäß SEQ ID NO: 580 und CDR-H3 gemäß SEQ ID NO: 581;
(f) CDR-L1 gemäß SEQ ID NO: 588, CDR-L2 gemäß SEQ ID NO: 589, CDR-L3 gemäß SEQ ID NO: 590, CDR-H1 gemäß SEQ ID NO: 591, CDR-H2 gemäß SEQ ID NO: 592 und CDR-H3 gemäß SEQ ID NO: 593;
(g) CDR-L1 gemäß SEQ ID NO: 599, CDR-L2 gemäß SEQ ID NO: 600, CDR-L3 gemäß SEQ ID NO: 601, CDR-H1 gemäß SEQ ID NO: 602, CDR-H2 gemäß SEQ ID NO: 603 und CDR-H3 gemäß SEQ ID NO: 604;
(h) CDR-L1 gemäß SEQ ID NO: 610, CDR-L2 gemäß SEQ ID NO: 611, CDR-L3 gemäß SEQ ID NO: 612, CDR-H1 gemäß SEQ ID NO: 613, CDR-H2 gemäß SEQ ID NO: 614 und CDR-H3 gemäß SEQ ID NO: 615;
(i) CDR-L1 gemäß SEQ ID NO: 621, CDR-L2 gemäß SEQ ID NO: 622, CDR-L3 gemäß SEQ ID NO: 623, CDR-H1 gemäß SEQ ID NO: 624, CDR-H2 gemäß SEQ ID NO: 625 und CDR-H3 gemäß SEQ ID NO: 626; und
(j) CDR-L1 gemäß SEQ ID NO: 633, CDR-L2 gemäß SEQ ID NO: 634, CDR-L3 gemäß SEQ ID NO: 635, CDR-H1 gemäß SEQ ID NO: 636, CDR-H2 gemäß SEQ ID NO: 637 und CDR-H3 gemäß SEQ ID NO: 638.

10. Antikörperkonstrukt zur Verwendung nach einem der vorhergehenden Ansprüche,
(I) wobei die zweite Domäne, die an CD3 bindet, eine VL-Region mit einer Aminosäuresequenz, die aus der Gruppe bestehend aus denen unter SEQ ID NO: 550, SEQ ID NO: 551, SEQ ID NO: 584, SEQ ID NO: 585, SEQ ID NO: 629 und SEQ ID NO: 630 ausgewählt ist, umfasst; und/oder
(II) wobei die zweite Domäne, die an CD3 bindet, eine VH-Region mit einer Aminosäuresequenz, die aus der Gruppe bestehend aus denen unter SEQ ID NO: 537, SEQ ID NO: 538, SEQ ID NO: 548, SEQ ID NO: 549, SEQ ID NO: 560, SEQ ID NO: 561, SEQ ID NO: 571, SEQ ID NO: 572, SEQ ID NO: 582, SEQ ID NO: 583, SEQ ID NO: 594, SEQ ID NO: 595, SEQ ID NO: 605, SEQ ID NO: 606, SEQ ID NO: 616, SEQ ID NO: 617, SEQ ID NO: 627, SEQ ID NO: 628, SEQ ID NO: 639, SEQ ID NO: 640 und SEQ ID NO: 644 ausgewählt ist, umfasst.

11. Antikörperkonstrukt zur Verwendung nach einem der vorhergehenden Ansprüche, wobei die zweite Domäne, die an CD3 bindet, eine VL-Region und eine VH-Region umfasst, die ausgewählt sind aus der Gruppe bestehend aus:
(a) einer VL-Region gemäß SEQ ID NO: 539 oder 521 und einer VH-Region gemäß SEQ ID NO: 537 oder 538;
(b) einer VL-Region gemäß SEQ ID NO: 550 oder 521 und einer VH-Region gemäß SEQ ID NO: 548 oder 549;
(c) einer VL-Region gemäß SEQ ID NO: 562 oder 521 und einer VH-Region gemäß SEQ ID NO: 560 oder 561;
(d) einer VL-Region gemäß SEQ ID NO: 573 oder 521 und einer VH-Region gemäß SEQ ID NO: 571 oder 572;
(e) einer VL-Region gemäß SEQ ID NO: 584 oder 585 und einer VH-Region gemäß SEQ ID NO: 582 oder 583;
(f) einer VL-Region gemäß SEQ ID NO: 596 oder 521 und einer VH-Region gemäß SEQ ID NO: 594 oder 595;
(g) einer VL-Region gemäß SEQ ID NO: 607 oder 585 und einer VH-Region gemäß SEQ ID NO: 605 oder 606;
(h) einer VL-Region gemäß SEQ ID NO: 618 oder 521 und einer VH-Region gemäß SEQ ID NO: 616 oder 617;
(i) einer VL-Region gemäß SEQ ID NO: 629 oder 630 und einer VH-Region gemäß SEQ ID NO: 627 oder 628;
(j) einer VL-Region gemäß SEQ ID NO: 641 oder 630 und einer VH-Region gemäß SEQ ID NO: 639 oder 640; und
(k) einer VL-Region gemäß SEQ ID NO: 645 und einer VH-Region gemäß SEQ ID NO: 644.

12. Antikörperkonstrukt zur Verwendung nach einem der vorhergehenden Ansprüche,
(I) wobei die zweite Domäne, die an CD3 bindet, ein Polypeptid mit einer Aminosäuresequenz, die aus der Gruppe bestehend aus denen unter SEQ ID NO: 540, 541, 552, 553, 563, 564, 574, 575, 586, 587, 597, 598, 608, 609, 619, 620, 631, 632, 642, 643 und 646 ausgewählt ist, umfasst bzw. daraus besteht; und/oder
(II) das ein Polypeptid mit einer Aminosäuresequenz, die aus der Gruppe bestehend aus denen unter SEQ ID NO: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520 und 530 ausgewählt ist, umfasst.

13. Antikörperkonstrukt zur Verwendung nach einem der vorhergehenden Ansprüche, umfassend in einer Reihenfolge vom N- zum C-Terminus:
(a) die erste Domäne;
(b) einen Peptidlinker mit einer Aminosäuresequenz, die aus der Gruppe bestehend aus SEQ ID NO: 687, 693 und 694 ausgewählt ist;
(c) die zweite Domäne;
(d) einen Peptidlinker mit einer Aminosäuresequenz, die aus der Gruppe bestehend aus SEQ ID NO: 686, 687, 688, 689, 690, 691, 692, 693 oder 694 ausgewählt ist;
(e) ein erstes Polypeptidmonomer (umfassend eine Hinge, eine CH2-Domäne und eine CH3-Domäne);
(f) einen Peptidlinker mit einer Aminosäuresequenz, die aus der Gruppe bestehend aus SEQ ID NO: 695, 696, 697, 698 oder 699 ausgewählt ist; und
(g) ein zweites Polypeptidmonomer (umfassend eine Hinge, eine CH2-Domäne und eine CH3-Domäne).

14. Antikörperkonstrukt zur Verwendung nach einem der vorhergehenden Ansprüche, umfassend in einer Reihenfolge vom N- zum C-Terminus:
(a) die erste Domäne mit einer Aminosäuresequenz, die aus der Gruppe bestehend aus SEQ ID NO: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519 und 529 ausgewählt ist; wobei der Peptidlinker, der sich in diesen Sequenzen befindet und SEQ ID NO: 694 aufweist, durch einen unter SEQ ID NO: 686-693 und 695-699 ersetzt werden kann;
(b) einen Peptidlinker mit einer Aminosäuresequenz, die aus der Gruppe bestehend aus SEQ ID NO: 687, 693 und 694 ausgewählt ist;
(c) die zweite Domäne mit einer Aminosäuresequenz, die aus der Gruppe bestehend aus SEQ ID NOs: 540, 541, 552, 553, 563, 564, 574, 575, 586, 587, 597, 598, 608, 609, 619, 620, 631, 632, 642, 643 und 646 ausgewählt ist; wobei der Peptidlinker, der sich in diesen Sequenzen befindet und SEQ ID NO: 694 aufweist, durch einen unter SEQ ID NO: 686-693 und 695-699 ersetzt werden kann;
(d) einen Peptidlinker mit einer Aminosäuresequenz, die aus der Gruppe bestehend aus SEQ ID NO: 686, 687, 688, 689, 690, 691, 692, 693 und 694 ausgewählt ist; und
(e) die dritte Domäne mit einer Aminosäuresequenz, die aus der Gruppe bestehend aus SEQ ID NO: 700-707 ausgewählt ist.

15. Antikörperkonstrukt zur Verwendung nach einem der vorhergehenden Ansprüche,
(I) umfassend in einer Reihenfolge vom N- zum C-Terminus:
(a) ein Polypeptid mit einer Aminosäuresequenz, die aus der Gruppe bestehend aus denen unter SEQ ID NO: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520 und 530 ausgewählt ist;
(b) einen Linker mit einer Aminosäuresequenz gemäß SEQ ID NO: 686; und
(c) ein Polypeptid mit einer Aminosäuresequenz gemäß SEQ ID NO: 700;
(II) das ein Molekulargewicht von etwa 75 bis etwa 200 kDa, etwa 80 bis etwa 175 kDa, etwa 85 bis etwa 150 kDa, etwa 90 bis etwa 130 kDa, etwa 95 bis etwa 120 kDa und bevorzugt etwa 100 bis etwa 115 kDa oder etwa 105 bis etwa 110 kDa aufweist; und/oder
(III) das eine Ausscheidungshalbwertszeit (T_{1/2}) von etwa 3 bis etwa 14 Tagen, etwa 4 bis etwa 12 Tagen, etwa 3 oder 4 bis etwa 10 Tagen, etwa 3 oder 4 bis etwa 8 Tagen oder etwa 5 bis etwa 7 Tagen aufweist.

## Revendications

1. Construction d'anticorps comprenant un premier domaine qui se lie à BCMA, un deuxième domaine qui se lie à CD3 et un troisième domaine qui prolonge la demi-vie plasmatique de la construction d'anticorps, pour une utilisation dans le traitement ou l'amélioration d'un néoplasme positif pour BCMA chez un humain, le néoplasme étant un néoplasme de cellules B ou un néoplasme de cellules plasmatiques, la construction d'anticorps étant administrée à une dose minimale de 800 µg/jour en au moins un cycle, un cycle comprenant au moins trois administrations individuelles de la construction d'anticorps, une administration individuelle étant l'administration sur un jour.

2. Construction d'anticorps pour une utilisation selon la revendication 1,
(I) qui est administrée pendant 2, 3, 4, 5, 6, 7, 8, 9, 10, 11, 12, 13, 14, 15, 16, 17, 18, 19, 20, 21, 22, 23, 24, 25 cycles ou plus ;
(II) un cycle ayant environ 25 à environ 30 jours, préférablement environ 26 ou 27 à environ 29 jours et plus préférablement environ 28 jours ;
(III) la construction d'anticorps étant administrée à une dose de 800 µg/jour à 12 mg par jour, telle que 1 000 pg, 1 200 pg, 1 500 pg, 1 600 pg, 2 000 pg, 2 500 pg, 3 000 pg, 3 500 pg, 4 000 pg, 4 500 pg, 5 000 pg, 5 500 pg, 6 000 pg, 6 500 pg, 7 000 pg, 7 500 µg, 8 000 µg, 8 500 µg, 9 000 µg, 9 500 µg, 10 mg, 11 mg ou 12 mg par jour ;
(IV) la construction d'anticorps étant administrée en une ou deux étapes de dosage pendant le premier cycle, une première dose étant préférablement entre 800 µg/jour et 1 200 µg/jour, une deuxième dose éventuelle étant entre 2 500 µg/jour et 5 000 µg/jour, préférablement environ 3 000 µg/jour ou environ 4 500 µg/jour, et une dernière dose (dose cible) étant entre 6 500 µg/jour et 12 mg/jour, telle que 6 500 µg/jour, 7 000 µg/jour, 7 500 µg/jour, 8 000 µg/jour, 8 500 µg/jour, 9 000 µg/jour, 9 500 µg/jour, 10 mg/jour, 11 mg/jour ou 12 mg/jour ; et/ou
(V) le premier cycle comprenant ou étant constitué de trois à six administrations individuelles, préférablement quatre ou cinq administrations individuelles de la construction d'anticorps.

3. Construction d'anticorps pour une utilisation selon la revendication 2, la construction d'anticorps étant administrée pendant le deuxième cycle et éventuellement pendant un quelconque cycle subséquent à des doses constantes.

4. Construction d'anticorps pour une utilisation selon l'une quelconque des revendications précédentes,
(I) qui est administrée de manière parentérale, préférablement de manière intraveineuse et plus préférablement via une injection de bolus intraveineuse, une perfusion de bolus ou une perfusion intraveineuse à court terme ;
(II) le néoplasme positif pour BCMA étant choisi dans le groupe constitué par un myélome multiple, un myélome multiple récidivant et/ou réfractaire, un myélome multiple à chaîne lourde, un myélome multiple à chaîne légère, un myélome extramédullaire, un plasmacyme, une leucémie plasmocytaire, une macroglobulinémie de Waldenström et un myélome couvant ; et/ou
(III)
a) la construction d'anticorps étant un polypeptide monocaténaire,
b) le premier domaine étant dans le format d'un scFv,
c) le deuxième domaine étant dans le format d'un scFv, et/ou
d) le premier domaine et le deuxième domaine étant reliés via un lieur, préférablement un lieur peptidique, plus préférablement un lieur glycine/sérine.

5. Construction d'anticorps pour une utilisation selon l'une quelconque des revendications précédentes,
(I) qui est en compétition pour la liaison à BCMA avec ou qui se lie au même épitope de BCMA que :
a) un anticorps ou une construction d'anticorps comprenant un domaine qui se lie à BCMA à la surface d'une cellule cible, ledit domaine comprenant une région VH comprenant CDR-H1 comme décrite dans la SEQ ID NO: 171, CDR-H2 comme décrite dans la SEQ ID NO: 172, et CDR-H3 comme décrite dans la SEQ ID NO: 173, et une région VL comprenant CDR-L1 comme décrite dans la SEQ ID NO: 174, CDR-L2 comme décrite dans la SEQ ID NO: 175, et CDR-L3 comme décrite dans la SEQ ID NO: 176 ;
b) un anticorps ou une construction d'anticorps comprenant un domaine qui se lie à BCMA à la surface d'une cellule cible, ledit domaine comprenant une région VH comme décrite dans la SEQ ID NO: 177, et une région VL comme décrite dans la SEQ ID NO: 178 ;
c) une construction d'anticorps comprenant un domaine qui se lie à BCMA à la surface d'une cellule cible, ledit domaine comprenant la séquence d'acides aminés comme décrite dans la SEQ ID NO: 179 ; ou
d) une construction d'anticorps ayant la séquence d'acides aminés comme décrite dans la SEQ ID NO: 661 ; et/ou
(II) qui est en compétition pour la liaison à CD3 avec ou qui se lie au même épitope de CD3 que :
a) un anticorps ou une construction d'anticorps comprenant un domaine qui se lie à CD3 sur la surface d'une cellule T, ledit domaine comprenant une région VH comprenant CDR-H1 comme décrite dans la SEQ ID NO: 636, CDR-H2 comme décrite dans la SEQ ID NO: 637, et CDR-H3 comme décrite dans la SEQ ID NO: 638, et une région VL comprenant CDR-L1 comme décrite dans la SEQ ID NO: 633, CDR-L2 comme décrite dans la SEQ ID NO: 634, CDR-L3 comme décrite dans la SEQ ID NO: 635 ;
b) un anticorps ou une construction d'anticorps comprenant un domaine qui se lie à CD3 sur la surface d'une cellule T, ledit domaine comprenant une région VH comme décrite dans la SEQ ID NO: 639, et une région VL comme décrite dans la SEQ ID NO: 641 ;
c) une construction d'anticorps comprenant un domaine qui se lie à CD3 sur la surface d'une cellule T, ledit domaine comprenant la séquence d'acides aminés comme décrite dans la SEQ ID NO: 642 ; ou
d) une construction d'anticorps ayant la séquence d'acides aminés comme décrite dans la SEQ ID NO: 661.

6. Construction d'anticorps pour une utilisation selon l'une quelconque des revendications précédentes, le premier domaine qui se lie à BCMA comprenant une région VH comprenant CDR-H1, CDR-H2 et CDR-H3 et une région VL comprenant CDR-L1, CDR-L2 et CDR-L3 choisies parmi :
(1) CDR-H1 comme décrite dans la SEQ ID NO: 1, CDR-H2 comme décrite dans la SEQ ID NO: 2, CDR-H3 comme décrite dans la SEQ ID NO: 3, CDR-L1 comme décrite dans la SEQ ID NO: 4, CDR-L2 comme décrite dans la SEQ ID NO: 5, et CDR-L3 comme décrite dans la SEQ ID NO: 6 ;
(2) CDR-H1 comme décrite dans la SEQ ID NO: 11, CDR-H2 comme décrite dans la SEQ ID NO: 12, CDR-H3 comme décrite dans la SEQ ID NO: 13, CDR-L1 comme décrite dans la SEQ ID NO: 14, CDR-L2 comme décrite dans la SEQ ID NO: 15, et CDR-L3 comme décrite dans la SEQ ID NO: 16 ;
(3) CDR-H1 comme décrite dans la SEQ ID NO: 21, CDR-H2 comme décrite dans la SEQ ID NO: 22, CDR-H3 comme décrite dans la SEQ ID NO: 23, CDR-L1 comme décrite dans la SEQ ID NO: 24, CDR-L2 comme décrite dans la SEQ ID NO: 25, et CDR-L3 comme décrite dans la SEQ ID NO: 26 ;
(4) CDR-H1 comme décrite dans la SEQ ID NO: 31, CDR-H2 comme décrite dans la SEQ ID NO: 32, CDR-H3 comme décrite dans la SEQ ID NO: 33, CDR-L1 comme décrite dans la SEQ ID NO: 34, CDR-L2 comme décrite dans la SEQ ID NO: 35, et CDR-L3 comme décrite dans la SEQ ID NO: 36 ;
(5) CDR-H1 comme décrite dans la SEQ ID NO: 41, CDR-H2 comme décrite dans la SEQ ID NO: 42, CDR-H3 comme décrite dans la SEQ ID NO: 43, CDR-L1 comme décrite dans la SEQ ID NO: 44, CDR-L2 comme décrite dans la SEQ ID NO: 45, et CDR-L3 comme décrite dans la SEQ ID NO: 46 ;
(6) CDR-H1 comme décrite dans la SEQ ID NO: 51, CDR-H2 comme décrite dans la SEQ ID NO: 52, CDR-H3 comme décrite dans la SEQ ID NO: 53, CDR-L1 comme décrite dans la SEQ ID NO: 54, CDR-L2 comme décrite dans la SEQ ID NO: 55, et CDR-L3 comme décrite dans la SEQ ID NO: 56 ;
(7) CDR-H1 comme décrite dans la SEQ ID NO: 61, CDR-H2 comme décrite dans la SEQ ID NO: 62, CDR-H3 comme décrite dans la SEQ ID NO: 63, CDR-L1 comme décrite dans la SEQ ID NO: 64, CDR-L2 comme décrite dans la SEQ ID NO: 65, et CDR-L3 comme décrite dans la SEQ ID NO: 66 ;
(8) CDR-H1 comme décrite dans la SEQ ID NO: 71, CDR-H2 comme décrite dans la SEQ ID NO: 72, CDR-H3 comme décrite dans la SEQ ID NO: 73, CDR-L1 comme décrite dans la SEQ ID NO: 74, CDR-L2 comme décrite dans la SEQ ID NO: 75, et CDR-L3 comme décrite dans la SEQ ID NO: 76 ;
(9) CDR-H1 comme décrite dans la SEQ ID NO: 81, CDR-H2 comme décrite dans la SEQ ID NO: 82, CDR-H3 comme décrite dans la SEQ ID NO: 83, CDR-L1 comme décrite dans la SEQ ID NO: 84, CDR-L2 comme décrite dans la SEQ ID NO: 85, et CDR-L3 comme décrite dans la SEQ ID NO: 86 ;
(10) CDR-H1 comme décrite dans la SEQ ID NO: 91, CDR-H2 comme décrite dans la SEQ ID NO: 92, CDR-H3 comme décrite dans la SEQ ID NO: 93, CDR-L1 comme décrite dans la SEQ ID NO: 94, CDR-L2 comme décrite dans la SEQ ID NO: 95, et CDR-L3 comme décrite dans la SEQ ID NO: 96 ;
(11) CDR-H1 comme décrite dans la SEQ ID NO: 101, CDR-H2 comme décrite dans la SEQ ID NO: 102, CDR-H3 comme décrite dans la SEQ ID NO: 103, CDR-L1 comme décrite dans la SEQ ID NO: 104, CDR-L2 comme décrite dans la SEQ ID NO: 105, et CDR-L3 comme décrite dans la SEQ ID NO: 106 ;
(12) CDR-H1 comme décrite dans la SEQ ID NO: 111, CDR-H2 comme décrite dans la SEQ ID NO: 112, CDR-H3 comme décrite dans la SEQ ID NO: 113, CDR-L1 comme décrite dans la SEQ ID NO: 114, CDR-L2 comme décrite dans la SEQ ID NO: 115, et CDR-L3 comme décrite dans la SEQ ID NO: 116 ;
(13) CDR-H1 comme décrite dans la SEQ ID NO: 121, CDR-H2 comme décrite dans la SEQ ID NO: 122, CDR-H3 comme décrite dans la SEQ ID NO: 123, CDR-L1 comme décrite dans la SEQ ID NO: 124, CDR-L2 comme décrite dans la SEQ ID NO: 125, et CDR-L3 comme décrite dans la SEQ ID NO: 126 ;
(14) CDR-H1 comme décrite dans la SEQ ID NO: 131, CDR-H2 comme décrite dans la SEQ ID NO: 132, CDR-H3 comme décrite dans la SEQ ID NO: 133, CDR-L1 comme décrite dans la SEQ ID NO: 134, CDR-L2 comme décrite dans la SEQ ID NO: 135, et CDR-L3 comme décrite dans la SEQ ID NO: 136 ;
(15) CDR-H1 comme décrite dans la SEQ ID NO: 141, CDR-H2 comme décrite dans la SEQ ID NO: 142, CDR-H3 comme décrite dans la SEQ ID NO: 143, CDR-L1 comme décrite dans la SEQ ID NO: 144, CDR-L2 comme décrite dans la SEQ ID NO: 145, et CDR-L3 comme décrite dans la SEQ ID NO: 146 ;
(16) CDR-H1 comme décrite dans la SEQ ID NO: 151, CDR-H2 comme décrite dans la SEQ ID NO: 152, CDR-H3 comme décrite dans la SEQ ID NO: 153, CDR-L1 comme décrite dans la SEQ ID NO: 154, CDR-L2 comme décrite dans la SEQ ID NO: 155, et CDR-L3 comme décrite dans la SEQ ID NO: 156 ;
(17) CDR-H1 comme décrite dans la SEQ ID NO: 161, CDR-H2 comme décrite dans la SEQ ID NO: 162, CDR-H3 comme décrite dans la SEQ ID NO: 163, CDR-L1 comme décrite dans la SEQ ID NO: 164, CDR-L2 comme décrite dans la SEQ ID NO: 165, et CDR-L3 comme décrite dans la SEQ ID NO: 166 ;
(18) CDR-H1 comme décrite dans la SEQ ID NO: 171, CDR-H2 comme décrite dans la SEQ ID NO: 172, CDR-H3 comme décrite dans la SEQ ID NO: 173, CDR-L1 comme décrite dans la SEQ ID NO: 174, CDR-L2 comme décrite dans la SEQ ID NO: 175, et CDR-L3 comme décrite dans la SEQ ID NO: 176 ;
(19) CDR-H1 comme décrite dans la SEQ ID NO: 181, CDR-H2 comme décrite dans la SEQ ID NO: 182, CDR-H3 comme décrite dans la SEQ ID NO: 183, CDR-L1 comme décrite dans la SEQ ID NO: 184, CDR-L2 comme décrite dans la SEQ ID NO: 185, et CDR-L3 comme décrite dans la SEQ ID NO: 186 ;
(20) CDR-H1 comme décrite dans la SEQ ID NO: 191, CDR-H2 comme décrite dans la SEQ ID NO: 192, CDR-H3 comme décrite dans la SEQ ID NO: 193, CDR-L1 comme décrite dans la SEQ ID NO: 194, CDR-L2 comme décrite dans la SEQ ID NO: 195, et CDR-L3 comme décrite dans la SEQ ID NO: 196 ;
(21) CDR-H1 comme décrite dans la SEQ ID NO: 201, CDR-H2 comme décrite dans la SEQ ID NO: 202, CDR-H3 comme décrite dans la SEQ ID NO: 203, CDR-L1 comme décrite dans la SEQ ID NO: 204, CDR-L2 comme décrite dans la SEQ ID NO: 205, et CDR-L3 comme décrite dans la SEQ ID NO: 206 ;
(22) CDR-H1 comme décrite dans la SEQ ID NO: 211, CDR-H2 comme décrite dans la SEQ ID NO: 212, CDR-H3 comme décrite dans la SEQ ID NO: 213, CDR-L1 comme décrite dans la SEQ ID NO: 214, CDR-L2 comme décrite dans la SEQ ID NO: 215, et CDR-L3 comme décrite dans la SEQ ID NO: 216 ;
(23) CDR-H1 comme décrite dans la SEQ ID NO: 221, CDR-H2 comme décrite dans la SEQ ID NO: 222, CDR-H3 comme décrite dans la SEQ ID NO: 223, CDR-L1 comme décrite dans la SEQ ID NO: 224, CDR-L2 comme décrite dans la SEQ ID NO: 225, et CDR-L3 comme décrite dans la SEQ ID NO: 226 ;
(24) CDR-H1 comme décrite dans la SEQ ID NO: 231, CDR-H2 comme décrite dans la SEQ ID NO: 232, CDR-H3 comme décrite dans la SEQ ID NO: 233, CDR-L1 comme décrite dans la SEQ ID NO: 234, CDR-L2 comme décrite dans la SEQ ID NO: 235, et CDR-L3 comme décrite dans la SEQ ID NO: 236 ;
(25) CDR-H1 comme décrite dans la SEQ ID NO: 241, CDR-H2 comme décrite dans la SEQ ID NO: 242, CDR-H3 comme décrite dans la SEQ ID NO: 243, CDR-L1 comme décrite dans la SEQ ID NO: 244, CDR-L2 comme décrite dans la SEQ ID NO: 245, et CDR-L3 comme décrite dans la SEQ ID NO: 246 ;
(26) CDR-H1 comme décrite dans la SEQ ID NO: 251, CDR-H2 comme décrite dans la SEQ ID NO: 252, CDR-H3 comme décrite dans la SEQ ID NO: 253, CDR-L1 comme décrite dans la SEQ ID NO: 254, CDR-L2 comme décrite dans la SEQ ID NO: 255, et CDR-L3 comme décrite dans la SEQ ID NO: 256 ;
(27) CDR-H1 comme décrite dans la SEQ ID NO: 261, CDR-H2 comme décrite dans la SEQ ID NO: 262, CDR-H3 comme décrite dans la SEQ ID NO: 263, CDR-L1 comme décrite dans la SEQ ID NO: 264, CDR-L2 comme décrite dans la SEQ ID NO: 265, et CDR-L3 comme décrite dans la SEQ ID NO: 266 ;
(28) CDR-H1 comme décrite dans la SEQ ID NO: 271, CDR-H2 comme décrite dans la SEQ ID NO: 272, CDR-H3 comme décrite dans la SEQ ID NO: 273, CDR-L1 comme décrite dans la SEQ ID NO: 274, CDR-L2 comme décrite dans la SEQ ID NO: 275, et CDR-L3 comme décrite dans la SEQ ID NO: 276 ;
(29) CDR-H1 comme décrite dans la SEQ ID NO: 281, CDR-H2 comme décrite dans la SEQ ID NO: 282, CDR-H3 comme décrite dans la SEQ ID NO: 283, CDR-L1 comme décrite dans la SEQ ID NO: 284, CDR-L2 comme décrite dans la SEQ ID NO: 285, et CDR-L3 comme décrite dans la SEQ ID NO: 286 ;
(30) CDR-H1 comme décrite dans la SEQ ID NO: 291, CDR-H2 comme décrite dans la SEQ ID NO: 292, CDR-H3 comme décrite dans la SEQ ID NO: 293, CDR-L1 comme décrite dans la SEQ ID NO: 294, CDR-L2 comme décrite dans la SEQ ID NO: 295, et CDR-L3 comme décrite dans la SEQ ID NO: 296 ;
(31) CDR-H1 comme décrite dans la SEQ ID NO: 301, CDR-H2 comme décrite dans la SEQ ID NO: 302, CDR-H3 comme décrite dans la SEQ ID NO: 303, CDR-L1 comme décrite dans la SEQ ID NO: 304, CDR-L2 comme décrite dans la SEQ ID NO: 305, et CDR-L3 comme décrite dans la SEQ ID NO: 306 ;
(32) CDR-H1 comme décrite dans la SEQ ID NO: 311, CDR-H2 comme décrite dans la SEQ ID NO: 312, CDR-H3 comme décrite dans la SEQ ID NO: 313, CDR-L1 comme décrite dans la SEQ ID NO: 314, CDR-L2 comme décrite dans la SEQ ID NO: 315, et CDR-L3 comme décrite dans la SEQ ID NO: 316 ;
(33) CDR-H1 comme décrite dans la SEQ ID NO: 321, CDR-H2 comme décrite dans la SEQ ID NO: 322, CDR-H3 comme décrite dans la SEQ ID NO: 323, CDR-L1 comme décrite dans la SEQ ID NO: 324, CDR-L2 comme décrite dans la SEQ ID NO: 325, et CDR-L3 comme décrite dans la SEQ ID NO: 326 ;
(34) CDR-H1 comme décrite dans la SEQ ID NO: 331, CDR-H2 comme décrite dans la SEQ ID NO: 332, CDR-H3 comme décrite dans la SEQ ID NO: 333, CDR-L1 comme décrite dans la SEQ ID NO: 334, CDR-L2 comme décrite dans la SEQ ID NO: 335, et CDR-L3 comme décrite dans la SEQ ID NO: 336 ;
(35) CDR-H1 comme décrite dans la SEQ ID NO: 341, CDR-H2 comme décrite dans la SEQ ID NO: 342, CDR-H3 comme décrite dans la SEQ ID NO: 343, CDR-L1 comme décrite dans la SEQ ID NO: 344, CDR-L2 comme décrite dans la SEQ ID NO: 345, et CDR-L3 comme décrite dans la SEQ ID NO: 346 ;
(36) CDR-H1 comme décrite dans la SEQ ID NO: 351, CDR-H2 comme décrite dans la SEQ ID NO: 352, CDR-H3 comme décrite dans la SEQ ID NO: 353, CDR-L1 comme décrite dans la SEQ ID NO: 354, CDR-L2 comme décrite dans la SEQ ID NO: 355, et CDR-L3 comme décrite dans la SEQ ID NO: 356 ;
(37) CDR-H1 comme décrite dans la SEQ ID NO: 361, CDR-H2 comme décrite dans la SEQ ID NO: 362, CDR-H3 comme décrite dans la SEQ ID NO: 363, CDR-L1 comme décrite dans la SEQ ID NO: 364, CDR-L2 comme décrite dans la SEQ ID NO: 365, et CDR-L3 comme décrite dans la SEQ ID NO: 366 ;
(38) CDR-H1 comme décrite dans la SEQ ID NO: 371, CDR-H2 comme décrite dans la SEQ ID NO: 372, CDR-H3 comme décrite dans la SEQ ID NO: 373, CDR-L1 comme décrite dans la SEQ ID NO: 374, CDR-L2 comme décrite dans la SEQ ID NO: 375, et CDR-L3 comme décrite dans la SEQ ID NO: 376 ;
(39) CDR-H1 comme décrite dans la SEQ ID NO: 381, CDR-H2 comme décrite dans la SEQ ID NO: 382, CDR-H3 comme décrite dans la SEQ ID NO: 383, CDR-L1 comme décrite dans la SEQ ID NO: 384, CDR-L2 comme décrite dans la SEQ ID NO: 385, et CDR-L3 comme décrite dans la SEQ ID NO: 386 ;
(40) CDR-H1 comme décrite dans la SEQ ID NO: 391, CDR-H2 comme décrite dans la SEQ ID NO: 392, CDR-H3 comme décrite dans la SEQ ID NO: 393, CDR-L1 comme décrite dans la SEQ ID NO: 394, CDR-L2 comme décrite dans la SEQ ID NO: 395, et CDR-L3 comme décrite dans la SEQ ID NO: 396 ;
(41) CDR-H1 comme décrite dans la SEQ ID NO: 401, CDR-H2 comme décrite dans la SEQ ID NO: 402, CDR-H3 comme décrite dans la SEQ ID NO: 403, CDR-L1 comme décrite dans la SEQ ID NO: 404, CDR-L2 comme décrite dans la SEQ ID NO: 405, et CDR-L3 comme décrite dans la SEQ ID NO: 406 ;
(42) CDR-H1 comme décrite dans la SEQ ID NO: 411, CDR-H2 comme décrite dans la SEQ ID NO: 412, CDR-H3 comme décrite dans la SEQ ID NO: 413, CDR-L1 comme décrite dans la SEQ ID NO: 414, CDR-L2 comme décrite dans la SEQ ID NO: 415, et CDR-L3 comme décrite dans la SEQ ID NO: 416 ;
(43) CDR-H1 comme décrite dans la SEQ ID NO: 421, CDR-H2 comme décrite dans la SEQ ID NO: 422, CDR-H3 comme décrite dans la SEQ ID NO: 423, CDR-L1 comme décrite dans la SEQ ID NO: 424, CDR-L2 comme décrite dans la SEQ ID NO: 425, et CDR-L3 comme décrite dans la SEQ ID NO: 426 ;
(44) CDR-H1 comme décrite dans la SEQ ID NO: 431, CDR-H2 comme décrite dans la SEQ ID NO: 432, CDR-H3 comme décrite dans la SEQ ID NO: 433, CDR-L1 comme décrite dans la SEQ ID NO: 434, CDR-L2 comme décrite dans la SEQ ID NO: 435, et CDR-L3 comme décrite dans la SEQ ID NO: 436 ;
(45) CDR-H1 comme décrite dans la SEQ ID NO: 441, CDR-H2 comme décrite dans la SEQ ID NO: 442, CDR-H3 comme décrite dans la SEQ ID NO: 443, CDR-L1 comme décrite dans la SEQ ID NO: 444, CDR-L2 comme décrite dans la SEQ ID NO: 445, et CDR-L3 comme décrite dans la SEQ ID NO: 446 ;
(46) CDR-H1 comme décrite dans la SEQ ID NO: 451, CDR-H2 comme décrite dans la SEQ ID NO: 452, CDR-H3 comme décrite dans la SEQ ID NO: 453, CDR-L1 comme décrite dans la SEQ ID NO: 454, CDR-L2 comme décrite dans la SEQ ID NO: 455, et CDR-L3 comme décrite dans la SEQ ID NO: 456 ;
(47) CDR-H1 comme décrite dans la SEQ ID NO: 461, CDR-H2 comme décrite dans la SEQ ID NO: 462, CDR-H3 comme décrite dans la SEQ ID NO: 463, CDR-L1 comme décrite dans la SEQ ID NO: 464, CDR-L2 comme décrite dans la SEQ ID NO: 465, et CDR-L3 comme décrite dans la SEQ ID NO: 466 ;
(48) CDR-H1 comme décrite dans la SEQ ID NO: 471, CDR-H2 comme décrite dans la SEQ ID NO: 472, CDR-H3 comme décrite dans la SEQ ID NO: 473, CDR-L1 comme décrite dans la SEQ ID NO: 474, CDR-L2 comme décrite dans la SEQ ID NO: 475, et CDR-L3 comme décrite dans la SEQ ID NO: 476 ;
(49) CDR-H1 comme décrite dans la SEQ ID NO: 481, CDR-H2 comme décrite dans la SEQ ID NO: 482, CDR-H3 comme décrite dans la SEQ ID NO: 483, CDR-L1 comme décrite dans la SEQ ID NO: 484, CDR-L2 comme décrite dans la SEQ ID NO: 485, et CDR-L3 comme décrite dans la SEQ ID NO: 486 ;
(50) CDR-H1 comme décrite dans la SEQ ID NO: 491, CDR-H2 comme décrite dans la SEQ ID NO: 492, CDR-H3 comme décrite dans la SEQ ID NO: 493, CDR-L1 comme décrite dans la SEQ ID NO: 494, CDR-L2 comme décrite dans la SEQ ID NO: 495, et CDR-L3 comme décrite dans la SEQ ID NO: 496 ;
(51) CDR-H1 comme décrite dans la SEQ ID NO: 501, CDR-H2 comme décrite dans la SEQ ID NO: 502, CDR-H3 comme décrite dans la SEQ ID NO: 503, CDR-L1 comme décrite dans la SEQ ID NO: 504, CDR-L2 comme décrite dans la SEQ ID NO: 505, et CDR-L3 comme décrite dans la SEQ ID NO: 506 ;
(52) CDR-H1 comme décrite dans la SEQ ID NO: 511, CDR-H2 comme décrite dans la SEQ ID NO: 512, CDR-H3 comme décrite dans la SEQ ID NO: 513, CDR-L1 comme décrite dans la SEQ ID NO: 514, CDR-L2 comme décrite dans la SEQ ID NO: 515, et CDR-L3 comme décrite dans la SEQ ID NO: 516 ; et
(53) CDR-H1 comme décrite dans la SEQ ID NO: 521, CDR-H2 comme décrite dans la SEQ ID NO: 522, CDR-H3 comme décrite dans la SEQ ID NO: 523, CDR-L1 comme décrite dans la SEQ ID NO: 524, CDR-L2 comme décrite dans la SEQ ID NO: 525, et CDR-L3 comme décrite dans la SEQ ID NO: 526.

7. Construction d'anticorps pour une utilisation selon l'une quelconque des revendications précédentes,
(I) le premier domaine qui se lie à BCMA comprenant une région VH ayant une séquence d'acides aminés choisie dans le groupe constitué par celles décrites dans les SEQ ID NO: 7, 17, 27, 37, 47, 57, 67, 77, 87, 97, 107, 117, 127, 137, 147, 157, 167, 177, 187, 197, 207, 217, 227, 237, 247, 257, 267, 277, 287, 307, 317, 327, 337, 347, 357, 367, 377, 387, 397, 407, 417, 427, 437, 447, 457, 467, 477, 487, 497, 507, 517, et 527 ; et/ou
(II) Construction d'anticorps pour une utilisation selon l'une quelconque des revendications précédentes, le premier domaine qui se lie à BCMA comprenant une région VL ayant une séquence d'acides aminés choisie dans le groupe constitué par celles décrites dans les SEQ ID NO: 8, 18, 28, 38, 48, 58, 68, 78, 88, 98, 108, 118, 128, 138, 148, 158, 168, 178, 188, 198, 208, 218, 228, 238, 248, 258, 268, 278, 288, 298, 308, 318, 328, 338, 348, 358, 368, 378, 388, 398, 408, 418, 428, 438, 448, 458, 468, 478, 488, 498, 508, 518, et 528.

8. Construction d'anticorps pour une utilisation selon l'une quelconque des revendications précédentes, le premier domaine qui se lie à BCMA comprenant une région VH et une région VL choisies dans le groupe constitué par :
(1) une région VH comme décrite dans la SEQ ID NO: 7 et une région VL comme décrite dans la SEQ ID NO: 8 ;
(2) une région VH comme décrite dans la SEQ ID NO: 17 et une région VL comme décrite dans la SEQ ID NO: 18 ;
(3) une région VH comme décrite dans la SEQ ID NO: 27 et une région VL comme décrite dans la SEQ ID NO: 28 ;
(4) une région VH comme décrite dans la SEQ ID NO: 37 et une région VL comme décrite dans la SEQ ID NO: 38 ;
(5) une région VH comme décrite dans la SEQ ID NO: 47 et une région VL comme décrite dans la SEQ ID NO: 48 ;
(6) une région VH comme décrite dans la SEQ ID NO: 57 et une région VL comme décrite dans la SEQ ID NO: 58 ;
(7) une région VH comme décrite dans la SEQ ID NO: 67 et une région VL comme décrite dans la SEQ ID NO: 68 ;
(8) une région VH comme décrite dans la SEQ ID NO: 77 et une région VL comme décrite dans la SEQ ID NO: 78 ;
(9) une région VH comme décrite dans la SEQ ID NO: 87 et une région VL comme décrite dans la SEQ ID NO: 88 ;
(10) une région VH comme décrite dans la SEQ ID NO: 97 et une région VL comme décrite dans la SEQ ID NO: 98 ;
(11) une région VH comme décrite dans la SEQ ID NO: 107 et une région VL comme décrite dans la SEQ ID NO: 108 ;
(12) une région VH comme décrite dans la SEQ ID NO: 117 et une région VL comme décrite dans la SEQ ID NO: 118 ;
(13) une région VH comme décrite dans la SEQ ID NO: 127 et une région VL comme décrite dans la SEQ ID NO: 128 ;
(14) une région VH comme décrite dans la SEQ ID NO: 137 et une région VL comme décrite dans la SEQ ID NO: 138 ;
(15) une région VH comme décrite dans la SEQ ID NO: 147 et une région VL comme décrite dans la SEQ ID NO: 148 ;
(16) une région VH comme décrite dans la SEQ ID NO: 157 et une région VL comme décrite dans la SEQ ID NO: 158 ;
(17) une région VH comme décrite dans la SEQ ID NO: 167 et une région VL comme décrite dans la SEQ ID NO: 168 ;
(18) une région VH comme décrite dans la SEQ ID NO: 177 et une région VL comme décrite dans la SEQ ID NO: 178 ;
(19) une région VH comme décrite dans la SEQ ID NO: 187 et une région VL comme décrite dans la SEQ ID NO: 188 ;
(20) une région VH comme décrite dans la SEQ ID NO: 197 et une région VL comme décrite dans la SEQ ID NO: 198 ;
(21) une région VH comme décrite dans la SEQ ID NO: 207 et une région VL comme décrite dans la SEQ ID NO: 208 ;
(22) une région VH comme décrite dans la SEQ ID NO: 217 et une région VL comme décrite dans la SEQ ID NO: 218 ;
(23) une région VH comme décrite dans la SEQ ID NO: 227 et une région VL comme décrite dans la SEQ ID NO: 228 ;
(24) une région VH comme décrite dans la SEQ ID NO: 237 et une région VL comme décrite dans la SEQ ID NO: 238 ;
(25) une région VH comme décrite dans la SEQ ID NO: 247 et une région VL comme décrite dans la SEQ ID NO: 248 ;
(26) une région VH comme décrite dans la SEQ ID NO: 257 et une région VL comme décrite dans la SEQ ID NO: 258 ;
(27) une région VH comme décrite dans la SEQ ID NO: 267 et une région VL comme décrite dans la SEQ ID NO: 268 ;
(28) une région VH comme décrite dans la SEQ ID NO: 277 et une région VL comme décrite dans la SEQ ID NO: 278 ;
(29) une région VH comme décrite dans la SEQ ID NO: 287 et une région VL comme décrite dans la SEQ ID NO: 288 ;
(30) une région VH comme décrite dans la SEQ ID NO: 297 et une région VL comme décrite dans la SEQ ID NO: 298 ;
(31) une région VH comme décrite dans la SEQ ID NO: 307 et une région VL comme décrite dans la SEQ ID NO: 308 ;
(32) une région VH comme décrite dans la SEQ ID NO: 317 et une région VL comme décrite dans la SEQ ID NO: 318 ;
(33) une région VH comme décrite dans la SEQ ID NO: 327 et une région VL comme décrite dans la SEQ ID NO: 328 ;
(34) une région VH comme décrite dans la SEQ ID NO: 337 et une région VL comme décrite dans la SEQ ID NO: 338 ;
(35) une région VH comme décrite dans la SEQ ID NO: 347 et une région VL comme décrite dans la SEQ ID NO: 348 ;
(36) une région VH comme décrite dans la SEQ ID NO: 357 et une région VL comme décrite dans la SEQ ID NO: 358 ;
(37) une région VH comme décrite dans la SEQ ID NO: 367 et une région VL comme décrite dans la SEQ ID NO: 368 ;
(38) une région VH comme décrite dans la SEQ ID NO: 377 et une région VL comme décrite dans la SEQ ID NO: 378 ;
(39) une région VH comme décrite dans la SEQ ID NO: 387 et une région VL comme décrite dans la SEQ ID NO: 388 ;
(40) une région VH comme décrite dans la SEQ ID NO: 397 et une région VL comme décrite dans la SEQ ID NO: 398 ;
(41) une région VH comme décrite dans la SEQ ID NO: 407 et une région VL comme décrite dans la SEQ ID NO: 408 ;
(42) une région VH comme décrite dans la SEQ ID NO: 417 et une région VL comme décrite dans la SEQ ID NO: 418 ;
(43) une région VH comme décrite dans la SEQ ID NO: 427 et une région VL comme décrite dans la SEQ ID NO: 428 ;
(44) une région VH comme décrite dans la SEQ ID NO: 437 et une région VL comme décrite dans la SEQ ID NO: 438 ;
(45) une région VH comme décrite dans la SEQ ID NO: 447 et une région VL comme décrite dans la SEQ ID NO: 448 ;
(46) une région VH comme décrite dans la SEQ ID NO: 457 et une région VL comme décrite dans la SEQ ID NO: 458 ;
(47) une région VH comme décrite dans la SEQ ID NO: 467 et une région VL comme décrite dans la SEQ ID NO: 468 ;
(48) une région VH comme décrite dans la SEQ ID NO: 477 et une région VL comme décrite dans la SEQ ID NO: 478 ;
(49) une région VH comme décrite dans la SEQ ID NO: 487 et une région VL comme décrite dans la SEQ ID NO: 488 ;
(50) une région VH comme décrite dans la SEQ ID NO: 497 et une région VL comme décrite dans la SEQ ID NO: 498 ;
(51) une région VH comme décrite dans la SEQ ID NO: 507 et une région VL comme décrite dans la SEQ ID NO: 508 ;
(52) une région VH comme décrite dans la SEQ ID NO: 517 et une région VL comme décrite dans la SEQ ID NO: 518 ; et
(53) une région VH comme décrite dans la SEQ ID NO: 527 et une région VL comme décrite dans la SEQ ID NO: 528.

9. Construction d'anticorps pour une utilisation selon l'une quelconque des revendications précédentes,
(I) le premier domaine qui se lie à BCMA comprenant ou étant constitué d'un polypeptide ayant une séquence d'acides aminés choisie dans le groupe constitué par les SEQ ID NO: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, et 529 ;
(II) le deuxième domaine qui se lie à CD3 comprenant une région VL comprenant CDR-L1, CDR-L2 et CDR-L3 choisies dans le groupe constitué par :
(a) CDR-L1 comme décrite dans la SEQ ID NO: 542, CDR-L2 comme décrite dans la SEQ ID NO: 543, et CDR-L3 comme décrite dans la SEQ ID NO: 544 ;
(b) CDR-L1 comme décrite dans la SEQ ID NO: 599, CDR-L2 comme décrite dans la SEQ ID NO: 600, et CDR-L3 comme décrite dans la SEQ ID NO: 601 ; et
(c) CDR-L1 comme décrite dans la SEQ ID NO: 621, CDR-L2 comme décrite dans la SEQ ID NO: 622, et CDR-L3 comme décrite dans la SEQ ID NO: 623 ;
(III) le deuxième domaine qui se lie à CD3 comprenant une région VH comprenant CDR-H1, CDR-H2 et CDR-H3 choisies dans le groupe constitué par :
(a) CDR-H1 comme décrite dans la SEQ ID NO: 534, CDR-H2 comme décrite dans la SEQ ID NO: 535, et CDR-H3 comme décrite dans la SEQ ID NO: 536 ;
(b) CDR-H1 comme décrite dans la SEQ ID NO: 545, CDR-H2 comme décrite dans la SEQ ID NO: 546, et CDR-H3 comme décrite dans la SEQ ID NO: 547 ;
(c) CDR-H1 comme décrite dans la SEQ ID NO: 557, CDR-H2 comme décrite dans la SEQ ID NO: 558, et CDR-H3 comme décrite dans la SEQ ID NO: 559 ;
(d) CDR-H1 comme décrite dans la SEQ ID NO: 568, CDR-H2 comme décrite dans la SEQ ID NO: 569, et CDR-H3 comme décrite dans la SEQ ID NO: 570 ;
(e) CDR-H1 comme décrite dans la SEQ ID NO: 579, CDR-H2 comme décrite dans la SEQ ID NO: 580, et CDR-H3 comme décrite dans la SEQ ID NO: 581 ;
(f) CDR-H1 comme décrite dans la SEQ ID NO: 591, CDR-H2 comme décrite dans la SEQ ID NO: 592, et CDR-H3 comme décrite dans la SEQ ID NO: 593 ;
(g) CDR-H1 comme décrite dans la SEQ ID NO: 602, CDR-H2 comme décrite dans la SEQ ID NO: 603, et CDR-H3 comme décrite dans la SEQ ID NO: 604 ;
(h) CDR-H1 comme décrite dans la SEQ ID NO: 613, CDR-H2 comme décrite dans la SEQ ID NO: 614, et CDR-H3 comme décrite dans la SEQ ID NO: 615 ;
(i) CDR-H1 comme décrite dans la SEQ ID NO: 624, CDR-H2 comme décrite dans la SEQ ID NO: 625, et CDR-H3 comme décrite dans la SEQ ID NO: 626 ; et
(j) CDR-H1 comme décrite dans la SEQ ID NO: 636, CDR-H2 comme décrite dans la SEQ ID NO: 637, et CDR-H3 comme décrite dans la SEQ ID NO: 638 ; et/ou
(IV) le deuxième domaine qui se lie à CD3 comprenant une région VL comprenant CDR-L1, CDR-L2 et CDR-L3 et une région VH comprenant CDR-H1, CDR-H2 et CDR-H3 choisies dans le groupe constitué par :
(a) CDR-L1 comme décrite dans la SEQ ID NO: 531, CDR-L2 comme décrite dans la SEQ ID NO: 532, CDR-L3 comme décrite dans la SEQ ID NO: 533, CDR-H1 comme décrite dans la SEQ ID NO: 534, CDR-H2 comme décrite dans la SEQ ID NO: 535, et CDR-H3 comme décrite dans la SEQ ID NO: 536 ;
(b) CDR-L1 comme décrite dans la SEQ ID NO: 542, CDR-L2 comme décrite dans la SEQ ID NO: 543, CDR-L3 comme décrite dans la SEQ ID NO: 544, CDR-H1 comme décrite dans la SEQ ID NO: 545, CDR-H2 comme décrite dans la SEQ ID NO: 546, et CDR-H3 comme décrite dans la SEQ ID NO: 547 ;
(c) CDR-L1 comme décrite dans la SEQ ID NO: 554, CDR-L2 comme décrite dans la SEQ ID NO: 555, CDR-L3 comme décrite dans la SEQ ID NO: 556, CDR-H1 comme décrite dans la SEQ ID NO: 557, CDR-H2 comme décrite dans la SEQ ID NO: 558, et CDR-H3 comme décrite dans la SEQ ID NO: 559 ;
(d) CDR-L1 comme décrite dans la SEQ ID NO: 565, CDR-L2 comme décrite dans la SEQ ID NO: 566, CDR-L3 comme décrite dans la SEQ ID NO: 567, CDR-H1 comme décrite dans la SEQ ID NO: 568, CDR-H2 comme décrite dans la SEQ ID NO: 569, et CDR-H3 comme décrite dans la SEQ ID NO: 570 ;
(e) CDR-L1 comme décrite dans la SEQ ID NO: 576, CDR-L2 comme décrite dans la SEQ ID NO: 577, CDR-L3 comme décrite dans la SEQ ID NO: 578, CDR-H1 comme décrite dans la SEQ ID NO: 579, CDR-H2 comme décrite dans la SEQ ID NO: 580, et CDR-H3 comme décrite dans la SEQ ID NO: 581 ;
(f) CDR-L1 comme décrite dans la SEQ ID NO: 588, CDR-L2 comme décrite dans la SEQ ID NO: 589, CDR-L3 comme décrite dans la SEQ ID NO: 590, CDR-H1 comme décrite dans la SEQ ID NO: 591, CDR-H2 comme décrite dans la SEQ ID NO: 592, et CDR-H3 comme décrite dans la SEQ ID NO: 593 ;
(g) CDR-L1 comme décrite dans la SEQ ID NO: 599, CDR-L2 comme décrite dans la SEQ ID NO: 600, CDR-L3 comme décrite dans la SEQ ID NO: 601, CDR-H1 comme décrite dans la SEQ ID NO: 602, CDR-H2 comme décrite dans la SEQ ID NO: 603, et CDR-H3 comme décrite dans la SEQ ID NO: 604 ;
(h) CDR-L1 comme décrite dans la SEQ ID NO: 610, CDR-L2 comme décrite dans la SEQ ID NO: 611, CDR-L3 comme décrite dans la SEQ ID NO: 612, CDR-H1 comme décrite dans la SEQ ID NO: 613, CDR-H2 comme décrite dans la SEQ ID NO: 614, et CDR-H3 comme décrite dans la SEQ ID NO: 615 ;
(i) CDR-L1 comme décrite dans la SEQ ID NO: 621, CDR-L2 comme décrite dans la SEQ ID NO: 622, CDR-L3 comme décrite dans la SEQ ID NO: 623, CDR-H1 comme décrite dans la SEQ ID NO: 624, CDR-H2 comme décrite dans la SEQ ID NO: 625, et CDR-H3 comme décrite dans la SEQ ID NO: 626 ; et
(j) CDR-L1 comme décrite dans la SEQ ID NO: 633, CDR-L2 comme décrite dans la SEQ ID NO: 634, CDR-L3 comme décrite dans la SEQ ID NO: 635, CDR-H1 comme décrite dans la SEQ ID NO: 636, CDR-H2 comme décrite dans la SEQ ID NO: 637, et CDR-H3 comme décrite dans la SEQ ID NO: 638.

10. Construction d'anticorps pour une utilisation selon l'une quelconque des revendications précédentes,
(I) le deuxième domaine qui se lie à CD3 comprenant une région VL ayant une séquence d'acides aminés choisie dans le groupe constitué par celles décrites dans les SEQ ID NO: 550, SEQ ID NO: 551, SEQ ID NO: 584, SEQ ID NO: 585, SEQ ID NO: 629 et SEQ ID NO: 630 ; et/ou
(II) le deuxième domaine qui se lie à CD3 comprenant une région VH ayant une séquence d'acides aminés choisie dans le groupe constitué par celles décrites dans les SEQ ID NO: 537, SEQ ID NO: 538, SEQ ID NO: 548, SEQ ID NO: 549, SEQ ID NO: 560, SEQ ID NO: 561, SEQ ID NO: 571, SEQ ID NO: 572, SEQ ID NO: 582, SEQ ID NO: 583, SEQ ID NO: 594, SEQ ID NO: 595, SEQ ID NO: 605, SEQ ID NO: 606, SEQ ID NO: 616, SEQ ID NO: 617, SEQ ID NO: 627, SEQ ID NO: 628, SEQ ID NO: 639, SEQ ID NO: 640, et SEQ ID NO: 644.

11. Construction d'anticorps pour une utilisation selon l'une quelconque des revendications précédentes, le deuxième domaine qui se lie à CD3 comprenant une région VL et une région VH choisies dans le groupe constitué par :
(a) une région VL comme décrite dans la SEQ ID NO: 539 ou 521 et une région VH comme décrite dans la SEQ ID NO: 537 ou 538 ;
(b) une région VL comme décrite dans la SEQ ID NO: 550 ou 521 et une région VH comme décrite dans la SEQ ID NO: 548 ou 549 ;
(c) une région VL comme décrite dans la SEQ ID NO: 562 ou 521 et une région VH comme décrite dans la SEQ ID NO: 560 ou 561 ;
(d) une région VL comme décrite dans la SEQ ID NO: 573 ou 521 et une région VH comme décrite dans la SEQ ID NO: 571 ou 572 ;
(e) une région VL comme décrite dans la SEQ ID NO: 584 ou 585 et une région VH comme décrite dans la SEQ ID NO: 582 ou 583 ;
(f) une région VL comme décrite dans la SEQ ID NO: 596 ou 521 et une région VH comme décrite dans la SEQ ID NO: 594 ou 595 ;
(g) une région VL comme décrite dans la SEQ ID NO: 607 ou 585 et une région VH comme décrite dans la SEQ ID NO: 605 ou 606 ;
(h) une région VL comme décrite dans la SEQ ID NO: 618 ou 521 et une région VH comme décrite dans la SEQ ID NO: 616 ou 617 ;
(i) une région VL comme décrite dans la SEQ ID NO: 629 ou 630 et une région VH comme décrite dans la SEQ ID NO: 627 ou 628 ;
(j) une région VL comme décrite dans la SEQ ID NO: 641 ou 630 et une région VH comme décrite dans la SEQ ID NO: 639 ou 640 ; et
(k) une région VL comme décrite dans la SEQ ID NO: 645 et une région VH comme décrite dans la SEQ ID NO: 644.

12. Construction d'anticorps pour une utilisation selon l'une quelconque des revendications précédentes,
(I) le deuxième domaine qui se lie à CD3 comprenant ou étant constitué d'un polypeptide ayant une séquence d'acides aminés choisie dans le groupe constitué par celles décrites dans les SEQ ID NO: 540, 541, 552, 553, 563, 564, 574, 575, 586, 587, 597, 598, 608, 609, 619, 620, 631, 632, 642, 643, et 646 ; et/ou
(II) qui comprend un polypeptide ayant une séquence d'acides aminés choisie dans le groupe constitué par celles décrites dans les SEQ ID NO: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, et 530.

13. Construction d'anticorps pour une utilisation selon l'une quelconque des revendications précédentes, comprenant dans un ordre de la terminaison N à la terminaison C :
(a) le premier domaine ;
(b) un lieur peptidique ayant une séquence d'acides aminés choisie dans le groupe constitué par les SEQ ID NO: 687, 693 et 694 ;
(c) le deuxième domaine ;
(d) un lieur peptidique ayant une séquence d'acides aminés choisie dans le groupe constitué par les SEQ ID NO: 686, 687, 688, 689, 690, 691, 692, 693 ou 694 ;
(e) un premier monomère de polypeptide (comprenant une charnière, un domaine CH2 et un domaine CH3) ;
(f) un lieur peptidique ayant une séquence d'acides aminés choisie dans le groupe constitué par les SEQ ID NO: 695, 696, 697, 698 ou 699 ; et
(g) un deuxième monomère de polypeptide (comprenant une charnière, un domaine CH2 et un domaine CH3).

14. Construction d'anticorps pour une utilisation selon l'une quelconque des revendications précédentes, comprenant dans un ordre de la terminaison N à la terminaison C :
(a) le premier domaine ayant une séquence d'acides aminés choisie dans le groupe constitué par les SEQ ID NO: 9, 19, 29, 39, 49, 59, 69, 79, 89, 109, 129, 139, 149, 159, 169, 179, 189, 199, 209, 219, 229, 239, 249, 259, 269, 279, 289, 299, 309, 319, 329, 339, 349, 359, 369, 379, 389, 399, 409, 419, 429, 439, 449, 459, 469, 479, 489, 499, 519, et 529 ; le lieur peptidique comprise dans ces séquences et ayant la SEQ ID NO: 694 pouvant être remplacé par l'une quelconque parmi les SEQ ID NO: 686-693 et 695-699 ;
(b) un lieur peptidique ayant une séquence d'acides aminés choisie dans le groupe constitué par les SEQ ID NO: 687, 693 et 694 ;
(c) le deuxième domaine ayant une séquence d'acides aminés choisie dans le groupe constitué par les SEQ ID NO: 540, 541, 552, 553, 563, 564, 574, 575, 586, 587, 597, 598, 608, 609, 619, 620, 631, 632, 642, 643, et 646 ; le lieur peptidique comprise dans ces séquences et ayant la SEQ ID NO: 694 pouvant être remplacé par l'une quelconque parmi les SEQ ID NO: 686-693 et 695-699 ;
(d) un lieur peptidique ayant une séquence d'acides aminés choisie dans le groupe constitué par les SEQ ID NO: 686, 687, 688, 689, 690, 691, 692, 693, et 694 ; et
(e) le troisième domaine ayant une séquence d'acides aminés choisie dans le groupe constitué par les SEQ ID NO: 700-707.

15. Construction d'anticorps pour une utilisation selon l'une quelconque des revendications précédentes,
(I) comprenant dans un ordre de la terminaison N à la terminaison C :
(a) un polypeptide ayant une séquence d'acides aminés choisie dans le groupe constitué par celles décrites dans les SEQ ID NO: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, et 530 ;
(b) un lieur ayant une séquence d'acides aminés comme décrite dans la SEQ ID NO: 686 ; et
(c) un polypeptide ayant une séquence d'acides aminés comme décrite dans la SEQ ID NO: 700 ;
(II) qui possède un poids moléculaire d'environ 75 à environ 200 kDa, environ 80 à environ 175 kDa, environ 85 à environ 150 kDa, environ 90 à environ 130 kDa, environ 95 à environ 120 kDa et préférablement environ 100 à environ 115 kDa ou environ 105 à environ 110 kDa ; et/ou
(III) qui possède une demi-vie d'élimination (T_{1/2}) d'environ 3 à environ 14 jours, environ 4 à environ 12 jours, environ 3 ou 4 à environ 10 jours, environ 3 ou 4 à environ 8 jours, ou environ 5 à environ 7 jours.
